(19) Europäisches Patentamt
European Patent Office
Office européen des brevets

(11) EP 3 795 693 B1

(12) **EUROPEAN PATENT SPECIFICATION**

(45) Date of publication and mention
of the grant of the patent:
**15.04.2026 Bulletin 2026/16**

(21) Application number: **18918659.6**

(22) Date of filing: **18.05.2018**

(51) International Patent Classification (IPC):
**C12Q 1/68** (2018.01)

(52) Cooperative Patent Classification (CPC):
**C12Q 1/6886; C12Q 2600/112; C12Q 2600/158**

(86) International application number:
**PCT/CN2018/087470**

(87) International publication number:
**WO 2019/218338 (21.11.2019 Gazette 2019/47)**

(54) **METHOD FOR DIAGNOSING CANCER BY MEANS OF BIOPSY CELL SAMPLE**

VERFAHREN ZUR DIAGNOSE VON KREBS MITTELS BIOPSIEZELLENPROBE

PROCÉDÉ DE DIAGNOSTIC DU CANCER AU MOYEN D'UN ÉCHANTILLON DE CELLULE DE BIOPSIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.03.2021 Bulletin 2021/12**

(73) Proprietor: **Lisen Imprinting Diagnostics, Inc.**
**Dover, Delaware 19901 (US)**

(72) Inventors:
• **CHENG, Tong**
**Wuxi, Jiangsu 214000 (CN)**
• **ZHOU, Ning**
**Wuxi, Jiangsu 214000 (CN)**

(56) References cited:
EP-A1- 2 233 590    WO-A1-2016/154330
WO-A1-2017/181146    WO-A2-2006/137066
WO-A2-2009/105549    US-B2- 8 669 057

• KIM SE IK ET AL: "Genomic landscape of ovarian clear cell carcinoma via whole exome sequencing", GYNECOLOGIC ONCOLOGY., vol. 148, no. 2, 1 February 2018 (2018-02-01), GB, pages 375 - 382, XP055870159, ISSN: 0090-8258, DOI: 10.1016/j.ygyno.2017.12.005

• ALBRECHT BETTINA ET AL: "Array-based comparative genomic hybridization for the detection of DNA sequence copy number changes in Barrett's adenocarcinoma", THE JOURNAL OF PATHOLOGY, vol. 203, no. 3, 21 June 2004 (2004-06-21), pages 780 - 788, XP055870257, ISSN: 0022-3417, DOI: 10.1002/path.1576

• KIM, JOOMYEONG ET AL.: "Epigenetic Instability of Imprinted Genes in Human Cancers", NUCLEIC ACIDS RESEARCH, vol. 43, no. 22, 15 December 2015 (2015-12-15), pages 10689 - 10699, XP055653629, DOI: 10.1093/nar/gkv867

• LI, BO ET AL.: "Assisted Reproduction Causes Reduced Fetal Growth Associated with Downregulation of Paternally Expressed Imprinted Genes That Enhance Fetal Growth", BIOLOGY OF REPRODUCTION, vol. 94, no. 2, 45, 29 February 2016 (2016-02-29), pages 1 - 11, XP055653632, DOI: 10.1095/biolreprod.115.136051

EP 3 795 693 B1

## Description

### FIELD

The present invention relates to a method for cancer diagnosis by assaying imprinted genes in a tumor based on biopsy samples. The invention relates further to the use of the method in the detection of cancer.

### BACKGROUND

Cancer is a main disease that threatens human health. About 14 million cancer patients are newly diagnosed annually worldwide, and 8.2 million die annually from cancer, and the morbidity and mortality are still growing year by year. The development of cancer is gradual, so that the early-stage cancer is usually related to low malignancy, and a high five-year survival rate could be achieved in case of an immediate operative treatment. In contrast, the late-stage cancer is usually related to high malignancy and is more prone to metastasis, which hampers operative treatment and also lead to poor effectiveness in radiotherapy and chemotherapy. Therefore, the five-year survival rate is rather low in the late-stage cancer patients. Early diagnosis is a key step in cancer treatment, nevertheless, the early-stage cancer is often miss diagnosed due to its symptomless or atypical symptom. Imaging technologies such as ultrasound and CT are able to detect the early-stage cancer, while it is difficult to determine the benignity or malignancy without sampling and biopsy. The main methods currently used for biopsy sampling include fine needle aspiration, core needle aspiration biopsy, endoscope guided tissue biopsy, and the brush biopsy from bronchus, esophagus, oral cavity, and uterine cervix.

Fine needle aspiration (FNA) is a sampling method for detection of lump, which directly punctures into the larger lump palpable from the body surface through the hollow needle, or punctures for the deeper or smaller lump under the guidance of ultrasound or CT. FNA is widely used for the tumor sampling in thyroid, mammary gland, lymph node, parotid gland, pancreas, liver, lung, prostate, and ovary. In the traditional pathology, the benignity or malignancy of cells is determined by the cell size, shape, invasiveness, and their relationship with the surrounding tissue. Due to the small sample size obtained from FNA, the histological morphology is hard to be indicated, and in the meantime, the cell heteromorphism in the early-stage cancer is relatively low, which limits the accuracy of cytopathological diagnosis using FNA. In addition, FNA relies largely on the pathologist's experience, and a certain diagnose may not achieved in some medium- and late-stage cancers.

The sampling method for core needle aspiration is similar with that of FNA, except for the coarser needle and bigger tissue stripe which can provide more histological information. However, the sample size is still small when compared with the whole tumor tissue, and some histological features, especially the invasive relationship between the cancer cells and the surrounding tissue is still difficult to determine. Therefore, limitation still exists in core needle aspiration.

Endoscope-guided tissue biopsy is a sampling method for suspicious lesion under the guidance of gastroscopy, colonoscopy, cystoscopy, hysteroscopy, or nasopharyngolarygnoscopy, which is usually used for the diagnosis of esophagus cancer, gastric cancer, colorectal cancer, bladder cancer, endometrial cancer, and nasopharyngeal cancer. Larger sample size can be obtained in tissue biopsy, but a definitive diagnosis is still hard in some early-stage cancer patients because of the low tissue heteromorphism.

Brush biopsy is often used for getting samples from bronchus, oral cavity, esophagus, and uterine cervix, and the small sample size is also difficult to show the histological morphology and resulted in low diagnostic accuracy. The urine exfoliated cell examination is a standard approach used in the detection of urinary system cancer, but the diagnosis is mainly given according to the cell morphology, and the diagnostic accuracy is rather low. The cystoscopy and biopsy are still necessary for a definitive diagnosis, in which patients may suffer much.

In order to avoid the limitation of traditional pathology in the early diagnosis of cancer, many biomarkers are developed for cancer diagnosis at the cell and molecule level, such as the BRAF for papillary thyroid carcinoma, the BRAC for breast cancer, the CEA for lung cancer, and the PSA for prostate cancer. However, some deficiencies still exist in these biomarkers, for example, BRAF can only be used for the detection of papillary thyroid carcinoma but not for the follicular thyroid carcinoma; the sensitivity of PSA is very high, but its specificity is rather low, giving rise to high false positive rate. So far, some tumor antigen detection and in situ hybridization technologies are applied in the urine, but the ideal sensitivity and specificity have not been achieved.

In the development of cancer, the changes in molecule level (epigenetics and genetics) is fairly ahead of these in cellular morphology and organizational structure, hence the molecular biology is more sensitive in detecting early stage cancer. Genomic imprinting is a kind of epigenetic regulation on the genes. For genomic imprinting, the allele from a specific parent is methylated and then silenced, so only the other allele is expressed. These genes are named imprinted genes. Loss of imprinting refers to a kind of epigenetic change in which the imprinted gene is demethylated and the silenced allele is reactivated for expression. Plenty of studies have revealed that this phenomenon (loss of imprinting) is common in various cancers, and is earlier than the morphological change in cell and tissue morphology. For example, Kim et al., described in 2015 that the expression and DNA methylation levels of the majority of imprinted domains are often affected in various human cancers (Kim et al., 2015, Nucleic Acids Research, 43(22)).

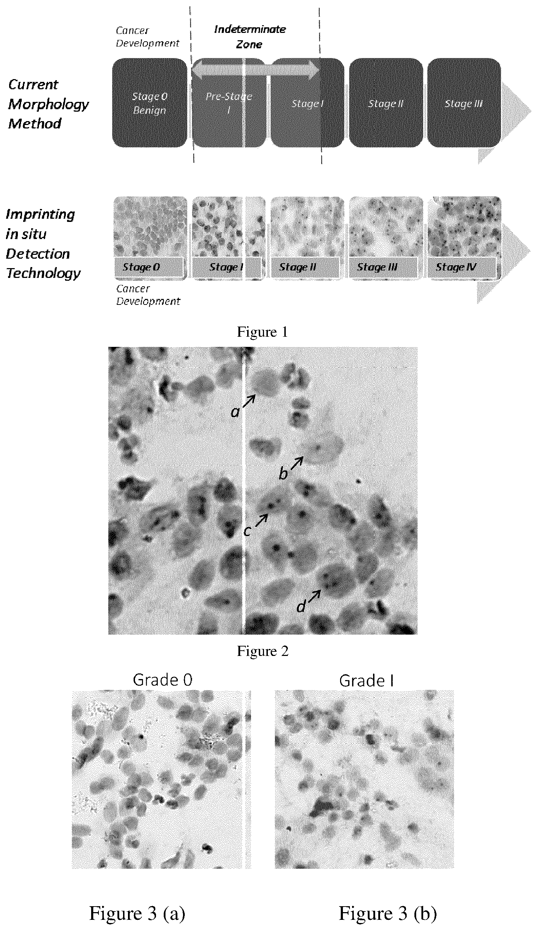

Meanwhile, in healthy cells, imprinting loss is very infrequent, which is in stark contrast with cancer cells. Therefore, the methylation status of the imprinted genes can be used as a pathological marker to analyze the abnormal condition of cells through certain molecular detecting technology. As shown in figure 1, the in situ detection for imprinted genes covers the shortages of the morphological pathology sensitively and effectively, wiping out the indeterminate zone of morphological pathology.

Based on these reasons, imprinted gene detection requires no histologic morphology and a small sample size, making it suitable for biopsy sample in cancer detection. The imprinted gene technology can be used for the detection of thyroid cancer, breast cancer, pancreatic cancer, lung cancer, liver cancer, colorectal cancer, bladder cancer, prostate cancer, gastric cancer, esophagus cancer, nasopharyngeal cancer, oral cancer, ovarian cancer, endometrial cancer, cervical cancer, central nervous system tumor, malignant parotid cancer, malignant lymphoma, or leukemia, and provide more accurate information for pre-diagnosis and final diagnosis, avoiding the problem of non-diagnosis due to insufficient cytological or histological heteromorphism. The imprinted gene detection can greatly increase the early diagnostic rate of cancer, and benefits for the patients by improving their life quality and prolonging their lifetime.

## SUMMARY

The invention is defined by the appended claims.

Considering the shortcomings of the present technologies, the present application provides a method for cancer diagnosis via biopsy samples. This method can observe the change of the imprinted genes at early stage cancer in a direct way, to determine the benignity or malignancy and the severity of cancer.

For the above propose, the present application provides a method for cancer diagnosis by assaying imprinted genes in a tumor based on biopsy samples,

the method comprising generating an expression profile of imprinted genes by calculating the expression level of imprinted genes showing loss of imprinting, the expression level of imprinted genes showing copy number variation, and the total expression level of imprinted genes in a tumor, to grade the expression of the imprinted genes; wherein the expression levels are calculated by the following formulas:

total expression level of an imprinted gene = (b+c+d)/(a+b+c+d)x100%;
the expression level of a normal imprinted gene = b/(b+c+d)x100%;
the expression level of an imprinted gene showing loss of imprinting = c/(b+c+d)x100%;
the expression level of an imprinted gene showing copy number variation = d/(b+c+d)x100%;
wherein, "a" represents cell nuclei with no mark inside after performing hematoxylin staining on a cell, which means the imprinted gene is not expressed in the cell nuclei;
"b" represents cell nuclei with a red/brown mark inside after performing hematoxylin staining on a cell, which means the imprinted gene exists in the cell nuclei;
"c" represents cell nuclei with two red/brown marks inside after performing hematoxylin staining on a cell, which means the imprinted gene loses imprinting in the cell nuclei; and
"d" represents cell nuclei with more than two red/brown marks inside after performing hematoxylin staining on a cell, which means the imprinted gene has a copy number variation in the cell nuclei;
wherein the imprinted gene is Z1 or Z16, Z1 is Gnas, and Z16 is Snrpn/Snurf;
the method further comprising classifying the expression profiles of imprinted genes into 5 grades;
the expression profile comprising the expression level of Z1 and Z16 showing loss of imprinting, the expression level of Z1 and Z16 showing copy number variation, and the total expression level of Z1 and Z16 is classified into 5 grades:

grade 0: any one or the combination of at least two of: the expression level of Z1 or Z16 showing loss of imprinting is less than 15%, the expression level of Z1 and Z16 showing copy number variation is less than 2%, or the total expression level of Z1 and Z16 is less than 25%;
grade I: any one or the combination of at least two of: the expression level of Z1 or Z16 showing loss of imprinting is 15-20%, the expression level of Z1 and Z16 showing copy number variation is 2-4%, or the total expression level of Z1 and Z16 is 25-30%;
grade II: any one or the combination of at least two of: the expression level of Z1 or Z16 showing loss of imprinting is 20-25%, the expression level of Z1 and Z16 showing copy number variation is 4-8%, or the total expression level of Z1 and Z16 is 30-40%;
grade III: any one or the combination of at least two of: the expression level of Z1 or Z16 showing loss of imprinting is 25-35%, the expression level of Z1 and Z16 showing copy number variation is 8-12%, or the total expression level of Z1 and Z16 is 40-50%;

grade IV: any one or the combination of at least two of: the expression level of Z1 or Z16 showing loss of imprinting is more than 35%, the expression level of Z1 and Z16 showing copy number variation is more than 12%, or the total expression level of Z1 and Z16 is more than 50%;

the method further comprising classifying the benignity or malignancy of the tumor to be determined as benign tumor, cancer potential, early-stage cancer, medium-stage cancer, and late-stage cancer;

preferably, the tumor is determined as a benign tumor, if the expression levels of both Z1 and Z16 showing loss of imprinting are lower than grade I; if the expression level of only one of Z1 and Z16 showing loss of imprinting is grade I; if the expression level of only one of Z1 and Z16 showing copy number variation is grade I;

preferably, the tumor is determined as cancer potential, if the expression levels of both Z1 and Z16 showing loss of imprinting are grade I; if the expression levels of both Z1 and Z16 showing copy number variation are grade I; if the expression level of only one of Z1 and Z16 showing loss of imprinting is grade I and the expression level of only one of Z1 and Z16 showing copy number variation is grade I; if the expression level of only one of Z1 and Z16 showing loss of imprinting is grade II; if the expression level of only one of Z1 and Z16 showing copy number variation is grade II;

preferably, the tumor is determined as an early-stage cancer, if the expression levels of both Z1 and Z16 showing loss of imprinting are grade II, and/or the expression levels of both Z1 and Z16 showing copy number variation are grade II; if the expression level of only one of Z1 and Z16 showing loss of imprinting is grade II and the expression level of only one of Z1 and Z16 showing copy number variation is grade II; if the expression level of only one of Z1 and Z16 showing loss of imprinting is grade III; if the expression level of only one of Z1 and Z16 showing copy number variation is grade III;

preferably, the tumor is determined as a medium-stage cancer, if the expression levels of both Z1 and Z16 showing loss of imprinting are grade III; if the expression levels of both Z1 and Z16 showing copy number variation are grade III; if the expression level of only one of Z1 and Z16 showing loss of imprinting is grade III and the expression level of only one of Z1 and Z16 showing copy number variation is grade III; if the expression level of only one of Z1 or Z16 showing loss of imprinting is grade IV; if the expression level of only one of Z1 or Z16 showing copy number variation is grade IV;

preferably, the tumor is determined as a late-stage cancer, if the expression levels of both Z1 and Z16 showing loss of imprinting are grade IV, and/or the expression levels of both Z1 and Z16 showing copy number variation are grade IV;

and wherein the tumor includes any one of thyroid tumor, breast tumor, pancreatic tumor, lung tumor, liver tumor, colorectal tumor, bladder tumor, prostate tumor, gastric tumor, esophagus tumor, nasopharyngeal tumor, oral tumor, ovarian tumor, endometrial tumor, cervical tumor, urinary system tumor, central nervous system tumor, parotid tumor, lymphoma, and leukemia.

The invention further provides for the use of the method described herein in the detection of cancer.

In the present application, the inventors find that by calculating the LOI, CNV, and TE of the imprinted gene Z1, the sensitivity reaches 99.2% for thyroid cancer diagnosis, 99.8% for breast cancer, 90.0% for pancreatic cancer, 99.8% for lung cancer, and 89.0% for urinary system cancer.

In addition, the inventors find that by calculating the LOI, CNV, and TE of the imprinted gene Z16, the sensitivity reaches 99.8% for thyroid cancer diagnosis, 91.7% for breast cancer, 90.0% for pancreatic cancer, 90.0% for lung cancer, and 89.0% for urinary system cancer.

According to the present application, the inventors find that the sensitivity can be further increased by calculating the expression of Z1 and Z16 with loss of imprinting and the expression of Z1 and Z16 with copy number variation. With combination of Z1 and Z16, the sensitivity for diagnosis of thyroid cancer, breast cancer, pancreatic cancer, lung cancer, and urinary system cancer can be higher than 99.9%.

In some embodiments of the present application, the imprinted genes also include any one of Z2, Z3, Z4, Z5, Z6, Z7, Z8, Z9, Z10, Z11, Z12, Z13, Z14, or Z15, or the combination of at least two; wherein, Z2 is Igf2, Z3 is Peg10, Z4 is Igf2r, Z5 is Mest, Z6 is Plag11, Z7 is Cdkn1c, Z8 is Dcn, Z9 is Dlk1, Z10 is Gatm, Z11 is Grb10, Z12 is Peg3, Z13 is Sgce, Z14 is Slc38a4, Z15 is Diras3.

In the present application, the inventors find that the addition of Z2, Z3, Z4, Z5, Z6, Z7, Z8, Z9, Z10, Z11, Z12, Z13, Z14, and Z15 on the basis of Z1 and Z16 helps to increase the accuracy of diagnosis. The inventors find that the sensitivities of imprinted genes Z2, Z3, Z4, Z5, Z6, Z7, Z8, Z9, Z10, Z11, Z12, Z13, Z14, and Z15 are different between cancers, which can be combined with other imprinted genes to determine the benignity and malignancy of cancer. Using the combination of the 16 imprinted genes Z1-Z16, the precise diagnosis of cancer can be achieved.

In the present application, the expression of an imprinted gene with loss of imprinting and the expression of an imprinted gene with copy number variation are calculated by the following formulas:

$$\text{Total expression} = (b+c+d)/(a+b+c+d)\times100\%;$$

$$\text{The expression of normal imprinted gene} = b/(b+c+d)\times100\%;$$

$$\text{The expression of an imprinted gene with loss of imprinting} = c/(b+c+d)\times100\%;$$

$$\text{The expression of an imprinted genes with copy number variation} = d/(b+c+d)\times100\%;$$

wherein, "a" represents cell nuclei with no mark inside after performing hematoxylin staining on cells, which means the imprinted gene has no expression in the cell nuclei; "b" represents cell nuclei with one red/brown mark inside after performing hematoxylin staining on cells, which means the imprinted gene exists in the cell nuclei; "c" represents cell nuclei with two red/brown marks inside after performing hematoxylin staining on cells, which means the imprinted gene loses imprinting in the cell nuclei; "d" represents cell nuclei with more than two red/brown marks inside after performing hematoxylin staining on cells, which means the imprinted gene has a copy number variation in the cell nuclei.

In the present application, for an imprinted gene with loss of imprinting, after performing hematoxylin staining on cells, there are two red/brown marks in the cell nuclei. For an imprinted gene with copy number variation, after performing hematoxylin staining on cells, there are more than two red/brown markers in the cell nuclei. The copy number variation is caused by the abnormal gene duplication in cancer cells, resulting in the expression of this gene as a triploid or even higher polyploid.

In the present application, the mark after hematoxylin staining is, but not limited to, red or brown, and the marks after other staining method with other colors can also be used for calculating the expression of an imprinted gene, the expression of an imprinted gene with loss of imprinting and the expression of an imprinted gene with copy number variation.

In the present application, imprinting gene and imprinted gene are the same concept, have the same meaning, and can be replaced by each other.

In some embodiments of the present application, the expression of an imprinted gene with loss of imprinting, the expression of an imprinted gene with copy number variation, and the total expression of an imprinted gene are classified into 5 grades.

In the present application, the expression of an imprinted gene with loss of imprinting, the expression of an imprinted gene with copy number variation, and the total expression of imprinted genes Z1 and Z16 are independent to each other.

In this application, the sensitivities of imprinted genes are different between cancers, and each index may fluctuate by 20% between cancers.

In some embodiments of the present invention, for thyroid tumor, the expression of Z1 with loss of imprinting, the expression of Z1 with copy number variation, and the total expression of Z1 are classified into 5 grades:

Grade 0: any one or the combination of at least two of: the expression of Z1 with loss of imprinting is less than 15%, the expression of Z1 with copy number variation is less than 1.5%, or the total expression of Z1 is less than 40%;
Grade I: any one or the combination of at least two of: the expression of Z1 with loss of imprinting is 15-20%, the expression of Z1 with copy number variation is 1.5-4%, or the total expression of Z1 is 40-45%;
Grade II: any one or the combination of at least two of: the expression of Z1 with loss of imprinting is 20-30%, the expression of Z1 with copy number variation is 4-8%, or the total expression of Z1 is 45-60%;
Grade III: any one or the combination of at least two of: the expression of Z1 with loss of imprinting is 30-40%, the expression of Z1 with copy number variation is 8-15%, or the total expression of Z1 is 60-65%;
Grade IV: any one or the combination of at least two of: the expression of Z1 with loss of imprinting is more than 40%, the expression of Z1 with copy number variation is more than 15%, or the total expression of Z1 is more than 65%.

For thyroid tumor, the expression of Z16 with loss of imprinting, the expression of Z16 with copy number variation, and the total expression of Z16 are classified into 5 grades:

Grade 0: any one or the combination of at least two of: the expression of Z16 with loss of imprinting is less than 15%, the expression of Z16 with copy number variation is less than 1.5%, or the total expression of Z16 is less than 30%;

Grade I: any one or the combination of at least two of: the expression of Z16 with loss of imprinting is 15-20%, the expression of Z16 with copy number variation is 1.5-4%, or the total expression of Z16 is 30-35%;
Grade II: any one or the combination of at least two of: the expression of Z16 with loss of imprinting is 20-30%, the expression of Z16 with copy number variation is 4-8%, or the total expression of Z16 is 35-50%;
Grade III: any one or the combination of at least two of: the expression of Z16 with loss of imprinting is 30-40%, the expression of Z16 with copy number variation is 8-15%, or the total expression of Z16 is 50-55%;
Grade IV: any one or the combination of at least two of: the expression of Z16 with loss of imprinting is more than 40%, the expression of Z16 with copy number variation is more than 15%, or the total expression of Z16 is more than 55%.

In some embodiments of the present application, the inventor found that combining Z3, Z11, Z13 with Z1 and Z16 can increase the diagnostic sensitivity for thyroid tumor. For example, when a combination of Z1 and Z3 were detected, the diagnostic sensitivity can reach 99.9% or more, when a combination of Z1 and Z11 were detected, the diagnostic sensitivity can reach 99.9% or more, when a combination of Z1 and Z13 were detected, the diagnostic sensitivity can reach 99.9% or more, when a combination of Z11 and Z16 were detected, the diagnostic sensitivity can reach 99.9% or more, when a combination of Z13 and Z16 were detected, the diagnostic sensitivity can reach 99.9% or more.

For breast tumor, the expression of Z1 and Z16 with loss of imprinting, the expression of Z1 and Z16 with copy number variation, and the total expression of Z1 and Z16 are classified into 5 grades:

Grade 0: any one or the combination of at least two of: the expression of Z1 and Z16 with loss of imprinting is less than 15%, the expression of Z1 and Z16 with copy number variation is less than 1%, or the total expression of Z1 and Z16 is less than 25%;
Grade I: any one or the combination of at least two of: the expression of Z1 and Z16 with loss of imprinting is 15-20%, the expression of Z1 and Z16 with copy number variation is 1-3%, or the total expression of Z1 and Z16 is 25-30%;
Grade II: any one or the combination of at least two of: the expression of Z1 and Z16 with loss of imprinting is 20-25%, the expression of Z1 and Z16 with copy number variation is 3-7%, or the total expression of Z1 and Z16 is 30-40%;
Grade III: any one or the combination of at least two of: the expression of Z1 and Z16 with loss of imprinting is 25-30%, the expression of Z1 and Z16 with copy number variation is 7-10%, or the total expression of Z1 and Z16 is 40-50%;
Grade IV: any one or the combination of at least two of: the expression of Z1 and Z16 with loss of imprinting is more than 30%, the expression of Z1 and Z16 with copy number variation is more than 10%, or the total expression of Z1 and Z16 is more than 50%.

In the present application, the expression of an imprinted gene with loss of imprinting, the expression of an imprinted gene with copy number variation, and the total expression of imprinted genes Z1 and Z16 are independent to each other.

In some embodiments of the present application, the inventor found that combining Z8, Z10, and Z13 with Z1 and Z16 can increase the diagnostic sensitivity for breast tumor. For example, when a combination of Z1 and Z8 were detected, the diagnostic sensitivity can reach 99.9% or more, when a combination of Z1 and Z10 were detected, the diagnostic sensitivity can reach 99.9% or more, when a combination of Z1 and Z11 were detected, the diagnostic sensitivity can reach 99.9% or more, when a combination of Z1 and Z13 were detected, the diagnostic sensitivity can reach 99.9% or more, when a combination of Z1 and Z16 were detected, the diagnostic sensitivity can reach 99.9% or more.

For pancreatic tumor, the expression of Z1 and Z16 with loss of imprinting, the expression of Z1 and Z16 with copy number variation, and the total expression of Z1 and Z16 are classified into 5 grades:

Grade 0: any one or the combination of at least two of: the expression of Z1 and Z16 with loss of imprinting is less than 15%, the expression of Z1 and Z16 with copy number variation is less than 2%, or the total expression of Z1 and Z16 is less than 20%;
Grade I: any one or the combination of at least two of: the expression of Z1 and Z16 with loss of imprinting is 15-20%, the expression of Z1 and Z16 with copy number variation is 2-4%, or the total expression of Z1 and Z16 is 20-30%;
Grade II: any one or the combination of at least two of: the expression of Z1 and Z16 with loss of imprinting is 20-25%, the expression of Z1 and Z16 with copy number variation is 4-8%, or the total expression of Z1 and Z16 is 30-40%;
Grade III: any one or the combination of at least two of: the expression of Z1 and Z16 with loss of imprinting is 25-30%, the expression of Z1 and Z16 with copy number variation is 8-12%, or the total expression of Z1 and Z16 is 40-50%;
Grade IV: any one or the combination of at least two of: the expression of Z1 and Z16 with loss of imprinting is more than 30%, the expression of Z1 and Z16 with copy number variation is more than 12%, or the total expression of Z1 and Z16 is more than 50%.

In the present application, the expression of an imprinted gene with loss of imprinting, the expression of an imprinted gene with copy number variation, and the total expression of Z1 and Z16 are independent to each other.

In some embodiments of the present application, the inventor found that combining Z5, Z10, Z11 with Z1 and Z16 can increase the diagnostic sensitivity for pancreatic tumor. For example, when a combination of Z1 and Z5 were detected, the diagnostic sensitivity can reach 99.9% or more, when a combination of Z1 and Z10 were detected, the diagnostic sensitivity can reach 99.9% or more, when a combination of Z1 and Z16 were detected, the diagnostic sensitivity can reach 99.9% or more, when a combination of Z10 and Z16 were detected, the diagnostic sensitivity can reach 99.9% or more, when a combination of Z11 and Z16 were detected, the diagnostic sensitivity can reach 99.9% or more.

For lung tumor, the expression of Z1 with loss of imprinting, the expression of Z1 with copy number variation, and the total expression of Z1 are classified into 5 grades:

   Grade 0: any one or the combination of at least two of: the expression of Z1 with loss of imprinting is less than 15%, the expression of Z1 with copy number variation is less than 2%, or the total expression of Z1 is less than 30%;
   Grade I: any one or the combination of at least two of: the expression of Z1 with loss of imprinting is 15-20%, the expression of Z1 with copy number variation is 2-4%, or the total expression of Z1 is 30-40%;
   Grade II: any one or the combination of at least two of: the expression of Z1 with loss of imprinting is 20-25%, the expression of Z1 with copy number variation is 4-8%, or the total expression of Z1 is 40-50%;
   Grade III: any one or the combination of at least two of: the expression of Z1 with loss of imprinting is 25-30%, the expression of Z1 with copy number variation is 8-12%, or the total expression of Z1 is 50-60%;
   Grade IV: any one or the combination of at least two of: the expression of Z1 with loss of imprinting is more than 30%, the expression of Z1 with copy number variation is more than 12%, or the total expression of Z1 is more than 60%.

For lung tumor, the expression of Z16 with loss of imprinting, the expression of Z16 with copy number variation, and the total expression of Z16 are classified into 5 grades:

   Grade 0: any one or the combination of at least two of: the expression of Z16 with loss of imprinting is less than 10%, the expression of Z16 with copy number variation is less than 1%, or the total expression of Z16 is less than 25%;
   Grade I: any one or the combination of at least two of: the expression of Z16 with loss of imprinting is 10-15%, the expression of Z16 with copy number variation is 1-2%, or the total expression of Z16 is 25-30%;
   Grade II: any one or the combination of at least two of: the expression of Z16 with loss of imprinting is 15-20%, the expression of Z16 with copy number variation is 2-5%, or the total expression of Z16 is 30-40%;
   Grade III: any one or the combination of at least two of: the expression of Z16 with loss of imprinting is 20-25%, the expression of Z16 with copy number variation is 5-8%, or the total expression of Z16 is 40-50%;
   Grade IV: any one or the combination of at least two of: the expression of Z16 with loss of imprinting is more than 25%, the expression of Z16 with copy number variation is more than 8%, or the total expression of Z16 is more than 50%.

In some embodiments of the present application, the inventor found that combining Z3, Z8, Z11 with Z1 and Z16 can increase the diagnostic sensitivity for lung tumor. For example, when a combination of Z1 and Z3 were detected, the diagnostic sensitivity can reach 99.9% or more, when a combination of Z1 and Z11 were detected, the diagnostic sensitivity can reach 99.9% or more, when a combination of Z1 and Z16 were detected, the diagnostic sensitivity can reach 99.9% or more, when a combination of Z10 and Z16 were detected, the diagnostic sensitivity can reach 99.9% or more, when a combination of Z3 and Z16 were detected, the diagnostic sensitivity can reach 99.9% or more.

For urinary system tumor, the expression of Z1 and Z16 with loss of imprinting, the expression of Z1 and Z16 with copy number variation, and the total expression of Z1 and Z16 are classified into 5 grades:

   Grade 0: any one or the combination of at least two of: the expression of Z1 and Z16 with loss of imprinting is less than 17%, the expression of Z1 and Z16 with copy number variation is less than 2%;
   Grade I: any one or the combination of at least two of: the expression of Z1 and Z16 with loss of imprinting is 17-20%, the expression of Z1 and Z16 with copy number variation is 2-3%;
   Grade II: any one or the combination of at least two of: the expression of Z1 and Z16 with loss of imprinting is 20-25%, the expression of Z1 and Z16 with copy number variation is 3-7%;
   Grade III: any one or the combination of at least two of: the expression of Z1 and Z16 with loss of imprinting is 25-30%, the expression of Z1 and Z16 with copy number variation is 7-12%;
   Grade IV: any one or the combination of at least two of: the expression of Z1 and Z16 with loss of imprinting is more than 30%, the expression of Z1 and Z16 with copy number variation is more than 12%.

In the present application, the expression of an imprinted gene with loss of imprinting, the expression of an imprinted gene with copy number variation, and the total expression of imprinted genes Z1 and Z16 are independent to each other.

In some embodiments of the present application, the inventor found that combining Z2, Z3, and Z4 with Z1 and Z16 can increase the diagnostic sensitivity for urinary system tumor. For example, when a combination of Z1 and Z5 were detected, the diagnostic sensitivity can reach 99.9% or more, when a combination of Z1 and Z10 were detected, the diagnostic sensitivity can reach 99.9% or more, when a combination of Z1 and Z16 were detected, the diagnostic sensitivity can reach 99.9% or more, when a combination of Z10 and Z16 were detected, the diagnostic sensitivity can reach 99.9% or more, when a combination of Z11 and Z16 were detected, the diagnostic sensitivity can reach 99.9% or more.

According to this invention, the following steps may be involved:

(1) obtaining a test sample;
(2) designing a probe specific for the sequence of the imprinted genes;
(3) performing in situ hybridization using the probe of step (2) to the test sample; and
(4) analyzing microscopic images and determining the expression status of the imprinted genes;

wherein, the analysis is performed by calculating the expressions of imprinted genes with loss of imprinting, the expressions of imprinted genes with copy number variation, and the total expressions of imprinted genes, and grading the expressions of imprinted genes with loss of imprinting, the expressions of imprinted genes with copy number variation, and the total expressions of imprinted genes to determine the benignity and malignancy of a tumor.

According to the present invention, the test sample of step (1) may be human tissues and/or cells.

In some embodiments of the present invention, the test sample includes any one or the combination of at least two of aspiration biopsy cells, biopsy cells, exfoliated cells, blood samples, or brush biopsy samples.

In the present application, the sampling procedure of biopsy samples includes acquiring cells from human tumor tissues by puncture, clamp and brushing, or acquiring exfoliated cells from urine, sputum, faeces, plural effusion and ascites, and then immediately fixing the sample in 10% neutral buffered formaldehyde solution or through other fixation method, followed by mounting to glass slides. Because the biopsy is less invasive and the acquisition of urine, sputum and faeces is non-invasive, the sampling process is easy, and the sample source can be located in contrast to the circulating blood, the biopsy has its special advantages as the experimental sample. The sampling procedure of blood includes acquiring the peripheral blood and lysing the red blood cell using a red blood cell lysis buffer, and then fixing the sample in 10% neutral buffered formaldehyde solution or through other fixation method, followed by mounting to glass slides.

In some embodiments of the present invention, the aspiration biopsy cells include fine or core needle aspiration biopsy samples, among which any one or the combination of at least two of the fine or core needle aspiration biopsy samples from thyroid, mammary gland, pancreas, lung, liver, prostate, ovary, lymph node, and parotid are preferable.

In some embodiments of the present invention, the biopsy cells include biopsy cells from any one or the combination of at least two of gastroscopy, colonoscopy, cystoscopy, hysteroscopy, or nasopharyngolarygnoscopy.

In some embodiments of the present invention, the exfoliated cells include exfoliated cells from any one or the combination of at least two of urine, sputum, faeces, plural effusion, or ascites.

In some embodiments of the present invention, the brush biopsy samples include the brushing samples from any one or the combination of at least two of bronchus, esophagus, oral cavity, or cervical.

In some embodiments of the present invention, in situ hybridization is performed using the RNAscope in situ hybridization method, the RNAscope in situ hybridization is performed by using singleplex or multiplex color assay kit or singleplex or multiplex fluorescence assay kit, among which, the singleplex red/brown color assay kit or multiplex fluorescence assay kit are preferable.

In the present application, the inventor calculates LOI, CNV, and TE in at least 100 cells with imprinted gene expression, to determine the benignity or malignancy of tumors through the grade of LOI, CNV, and TE. The benignity or malignancy of a tumor is classified into benign tumor, cancer potential, early-stage cancer, medium-stage cancer, and late-stage cancer.

The tumor is determined as a benign tumor, if the expression of both Z1 and Z16 with loss of imprinting are lower than grade I; if the expression of only one of Z1 and Z16 with loss of imprinting is grade I; if the expression of only one of Z1 and Z16 with copy number variation is grade I;

The tumor is determined as cancer potential, if the expression of both Z1 and Z16 with loss of imprinting are grade I; if the expression of both Z1 and Z16 with copy number variation are grade I; if the expression of only one of Z1 and Z16 with loss of imprinting is grade I and the expression of only one of Z1 and Z16 with copy number variation is grade I; if the expression of only one of Z1 and Z16 with loss of imprinting is grade II; if the expression of only one of Z1 and Z16 with copy number variation is grade II;

The tumor is determined as an early-stage cancer; if the expression of both Z1 and Z16 with loss of imprinting are grade II, and/or the expression of both Z1 and Z16 with copy number variation are grade II; if the expression of only one of Z1 and Z16 with loss of imprinting is grade II and the expression of only one of Z1 and Z16 with copy number variation is grade II; if the expression of only one of Z1 and Z16 with loss of imprinting is grade III; if the expression of only one of Z1 and Z16 with copy number variation is grade III;

The tumor is determined as a medium-stage cancer; if the expression of both Z1 and Z16 with loss of imprinting are grade III; if the expression of both Z1 and Z16 with copy number variation are grade III; if the expression of only one of Z1 and Z16 with loss of imprinting is grade III and the expression of only one of Z1 and Z16 with copy number variation is grade III; if the expression of only one of Z1 or Z16 with loss of imprinting is grade IV; if the expression of only one of Z1 or Z16 with copy number variation is grade IV;

The tumor is determined as a late-stage cancer if the expression of both Z1 and Z16 with loss of imprinting are grade IV, and/or the expression of both Z1 and Z16 with copy number variation are grade IV.

Second, the present application provides the use of the method mentioned in the first part for cancer detection and/or treatment.

Compared with the existing technologies, the present application has the following beneficial effects:

(1) The method described in the present application showed the phenomenon of loss of imprinting in the aspiration biopsy samples of thyroid, mammary gland, prostate, and lymph node, the bronchial brushing cell sample of lung, exfoliated cell samples from urine, sputum, faeces, and plural effusion, and biopsy cell samples from colonoscopy and cystoscopy. Through in situ labeling of imprinted genes, this method detects the change of the imprinted genes objectively, intuitively, early, and precisely. In addition, this method provides the quantitative model, and makes a significant contribution to the early diagnosis of thyroid cancer, breast cancer, pancreatic cancer, lung cancer, liver cancer, colorectal cancer, and urinary system cancer.

(2) The method described in the present application can provide quantitative detection results, to determine the type of tumor precisely, and grade the malignancy of tumor, which solves the problem that part of the cases cannot be clearly diagnosed by the traditional cytopathology and histopathology. Moreover, the method also breakthroughs the limitation of current tissue and cell morphological diagnosis, and provides assistance for the future targeted therapy.

(3) This method in the present application is able to distinguish the benignity and malignancy of the hurthle cell tumor (HCT) of thyroid at the molecule level, provides a solution to the difficulty in distinguishing the benignity and malignancy from tissue and cell morphology. It is also difficult for the morphological diagnosis to distinguish the follicular thyroid adenoma (FTA) from follicular thyroid cancer (FTC), which can be distinguished by this method on the cell level

(4) This method in the present application can be performed together with the immunohistochemical method, to diagnose the benignity or malignancy of pancreatic tumor accurately at early stage, and avoid the false negative and other side effects.

(5) For the tumors including breast cancer, lung cancer, and liver cancer that can metastasize through the lymphatic system, this method is not only able to determine the benignity or malignancy at early stage through the fine-needle aspiration biopsy, but also able to puncture the lymph nodes adjacent to the tumor to test the metastasis through lymphatic system.

(6) The method in the present application enables the clear distinction of pigmented nevus from skin melanoma and other skin diseases, especially for the differential diagnosis of early stage melanoma in situ, and resolving the difficulty in the morphological diagnosis.

(7) The method in the present application enables the early diagnosis of breast ductal adenocarcinoma in situ and distinguishing from the benign breast lesions.

(8) This method can accurately diagnose the early stage malignant potential and malignant of the polyp in colon, resolving the early diagnosis problem of colorectal cancer.

(9) This method can distinguish gastritis and gastric ulcer from early stage gastric cancer, resolving the early diagnosis problem of gastric cancer.

(10) This method is independent of the tissue morphology, therefore can also be used for the diagnosis of benignity or malignancy through the aspiration biopsy samples from prostate cancer, ovarian cancer, central nervous system tumor, malignant tumor in the parotid gland, and malignant lymphoma, the endoscopic biopsy samples from gastric cancer, esophagus cancer, nasopharynx cancer, and endometrial cancer, the brush biopsy samples from lung cancer, oral cancer, esophagus cancer, and cervical cancer, and the blood sample from leukemia.

(11) The method in the present application can diagnose the leukemia through a single drop of peripheral blood, which can avoid the large trauma caused by currently used bone marrow aspiration.

(12) The method proposed in the present application can screen the early urinary system cancer, lung cancer, and colorectal cancer through the exfoliated cells from urine, sputum, and faeces. The sample collection procedure is simple and with no need for professional operators, so the samples can be collected by the patient at home. This method is suitable for the large scale physical examination, post-operational and drug effect monitoring in urinary system cancer, especially the pre-operative and post-operative detection, and can provide solutions to the early detection of postoperative recurrence and immediate treatment.

(13) In the surgical operation, the benignity and malignancy of the paracancerous tissue is a key indicator to evaluate the patient's prognosis and survival. The method in the present application provides an importance guidance for it.

(14) The technologies, methods, and targeted drugs which can lead to the silence, knockout or rearrangement of the disease-related imprinted genes discovered by the method in the present application can be used to guide the later treatment and pharmacy.

## BRIEF DESCRIPTION OF DRAWINGS

Figure 1 shows the difference between the imprinted gene in situ detection method described in this application and the traditional morphology diagnostic method;

Figure 2 shows the aspiration cell smear of the thyroid cancer with nucleus stained by hematoxylin, wherein, "a" represents cell nuclei with no mark inside after performing hematoxylin staining on cells, which means the imprinted gene has no expression in the cell nuclei, "b" represents cell nuclei with one red/brown mark inside after performing hematoxylin staining on cells, which means the imprinted gene exists in the cell nuclei, "c" represents cell nuclei with two red/brown marks inside after performing hematoxylin staining on cells, which means the imprinted gene loses imprinting in the cell nuclei, "d" represents cell nuclei with more than two red/brown marks inside after performing hematoxylin staining on cells, which means the imprinted gene has a copy number variation in the cell nuclei;

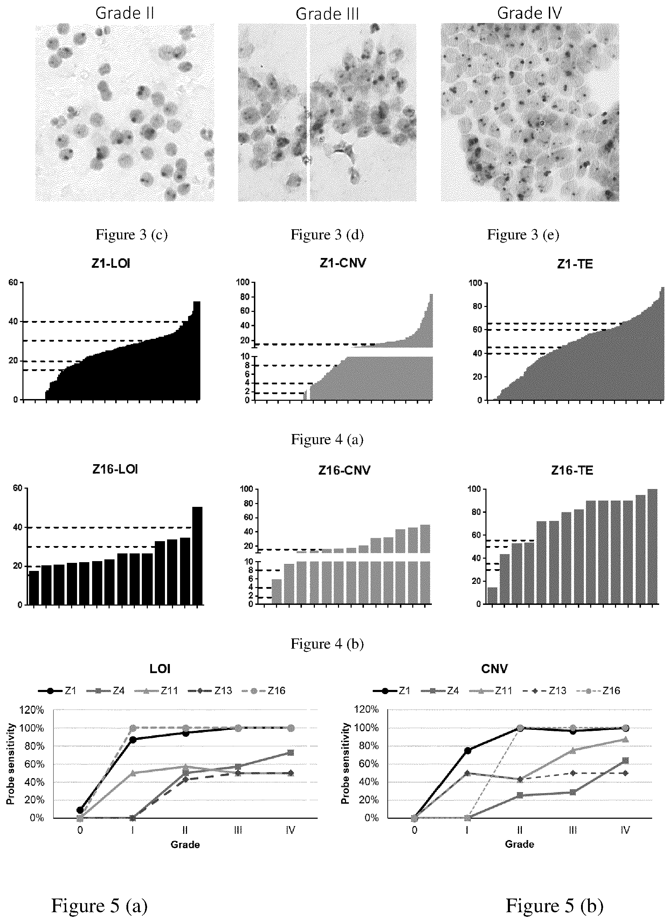

Figure 3 shows the expression status of 9 of the 16 imprinted genes mentioned in the present application in the aspiration cell smear of the thyroid cancer with different grades of malignancy, wherein, figure 3(a) indicates the expression status of the 9 genes in the aspiration cell smear of grade 0 thyroid tumor, figure 3(b) indicates the expression status of the 9 genes in the aspiration cell smear of grade I thyroid cancer, figure 3(c) indicates the expression status of the 9 genes in the aspiration cell smear of grade II thyroid cancer, figure 3(d) indicates the expression status of the 9 genes in the aspiration cell smear of grade III thyroid cancer, figure 3(e) indicates the expression status of the 9 genes in the aspiration cell smear of grade IV thyroid cancer;

Figure 4 demonstrates the distribution range and grading standards of the loss of imprinting, copy number variation and the total expression of imprinted genes Z1 and Z16 in aspiration cell samples of 177 thyroid tumors, wherein, figure 4(a) shows the distribution range and grading standard of loss of imprinting, copy number variation and total expression of Z1 in aspiration cell samples of 177 thyroid tumors, figure 4(b) shows the distribution range and grading standard of loss of imprinting, copy number variation and the total expression of Z16 in aspiration cell samples of 177 thyroid tumors;

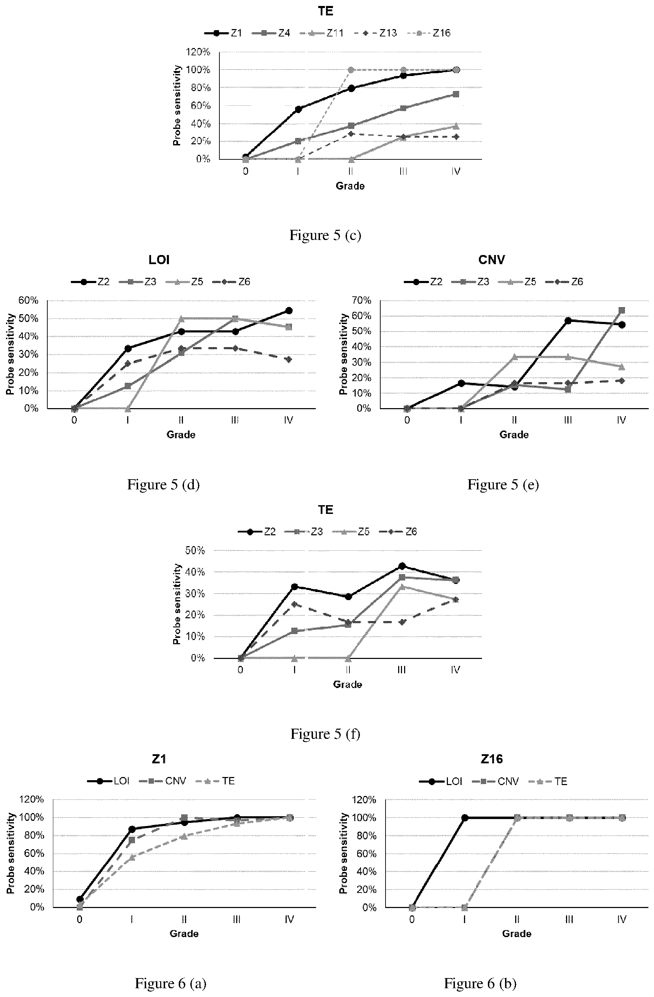

Figure 5(a) indicates the intensity of the loss of imprinting of Z1, Z4, Z11, Z13, and Z16 in thyroid cancer, figure 5(b) indicates the intensity of the copy number variation of Z1, Z4, Z11, Z13, and Z16 in thyroid cancer, figure 5(c) indicates the intensity of the total expression of Z1, Z4, Z11, Z13, and Z16 in thyroid cancer, figure 5(d) indicates the intensity of the loss of imprinting of Z2, Z3, Z5, and Z6 in thyroid cancer, figure 5(e) indicates the intensity of the copy number variation of Z2, Z3, Z5, and Z6 in thyroid cancer, figure 5(f) indicates the intensity of the total expression of Z2, Z3, Z5, and Z6 in thyroid cancer, wherein, LOI stands for the expression of an imprinted gene with loss of imprinting, CNV stands for the expression of an imprinted gene with copy number variation, and TE stands for the total expression of an imprinted gene;

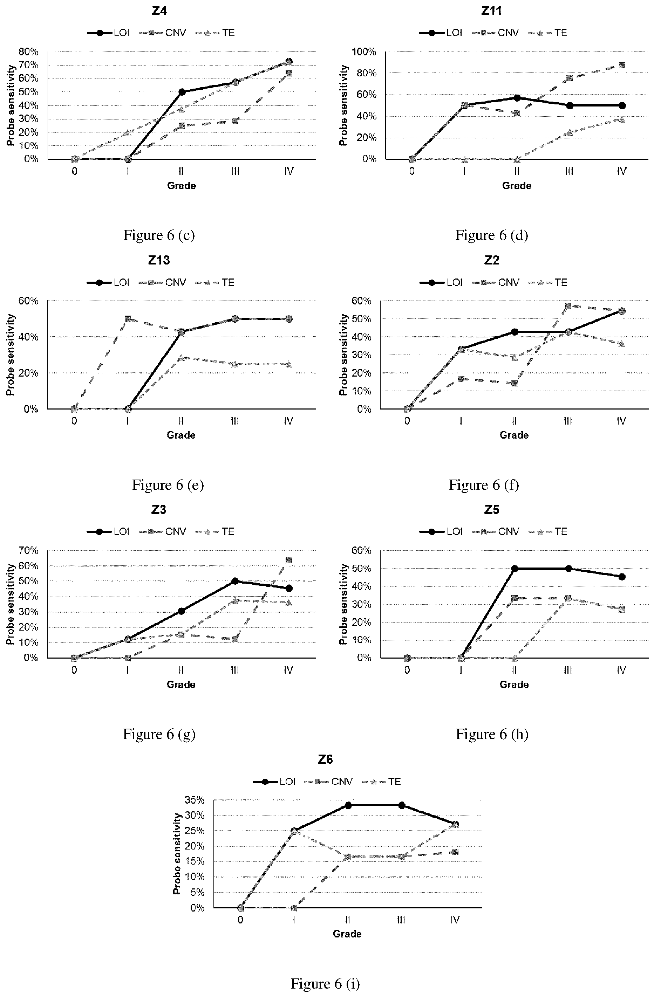

Figure 6(a) indicates the intensity of the loss of imprinting, copy number variation, and total expression of the imprinted gene Z1, figure 6(b) indicates the intensity of the loss of imprinting, copy number variation, and total expression of the imprinted gene Z16, figure 6(c) indicates the intensity of the loss of imprinting, copy number variation, and total expression of the imprinted gene Z4, figure 6(d) indicates the intensity of the loss of imprinting, copy number variation, and total expression of the imprinted gene Z11, figure 6(e) indicates the intensity of the loss of imprinting, copy number variation, and total expression of the imprinted gene Z13, figure 6(f) indicates the intensity of the loss of imprinting, copy number variation, and total expression of the imprinted gene Z2, figure 6(g) indicates the intensity of the loss of imprinting, copy number variation, and total expression of the imprinted gene Z3, figure 6(h) indicates the intensity of the loss of imprinting, copy number variation, and total expression of the imprinted gene Z5, figure 6(i) indicates the intensity of the loss of imprinting, copy number variation, and total expression of the imprinted gene Z6, wherein, LOI is the expression level of an imprinted gene with loss of imprinting, CNV is the expression of an imprinted gene with copy number variation, and TE is the total expression of an imprinted gene;

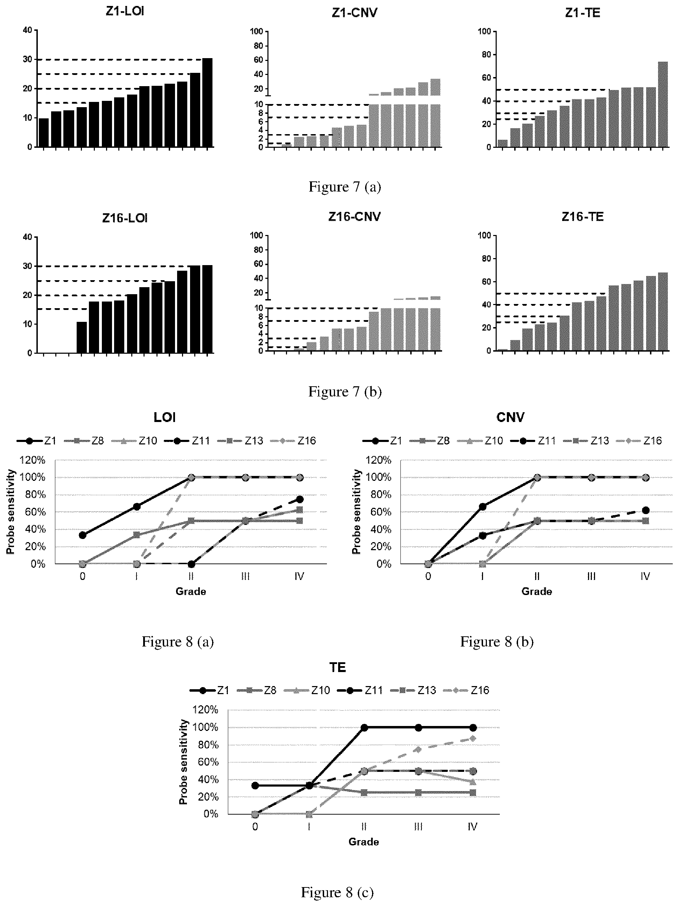

Figure 7 demonstrates the distribution range and grading standards of the loss of imprinting, copy number variation and the total expression of imprinted genes Z1 and Z16 in aspiration cell samples of 18 breast cancers, figure 7(a) shows the distribution range and grading standard of loss of imprinting, copy number variation and total expression of Z1 in aspiration cell samples of 18 breast cancers, figure 7(b) shows the distribution range and grading standard of loss of imprinting, copy number variation and total expression of Z16 in aspiration cell samples of 18 breast cancers;

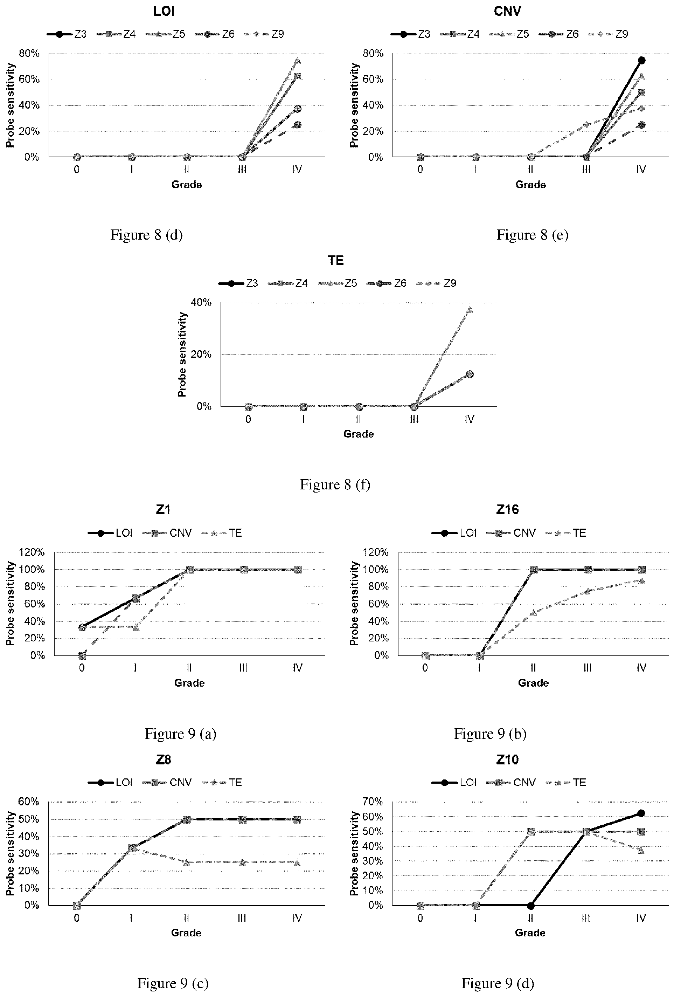

Figure 8(a) indicates the intensity of the loss of imprinting of Z1, Z8, Z10, Z11, Z13, and Z16 in breast cancer, figure 8(b) indicates the intensity of the copy number variation of Z1, Z8, Z10, Z11, Z13, and Z16 in breast cancers, figure 8(c) indicates the intensity of the total expression of Z1, Z8, Z10, Z11, Z13, and Z16 in breast cancers, figure 8(d) indicates the intensity of the loss of imprinting of Z3, Z4, Z5, Z6, and Z9 in breast cancer, figure 8(e) indicates the intensity of the copy number variation of Z3, Z4, Z5, Z6, and Z9 in breast cancer, figure 8(f) indicates the intensity of the total expression of Z3, Z4, Z5, Z6, and Z9 in breast cancer, Of which, LOI is the expression of an imprinted gene with loss of imprinting, CNV is the expression of an imprinted gene with copy number variation, and TE is the total expression of an imprinted gene;

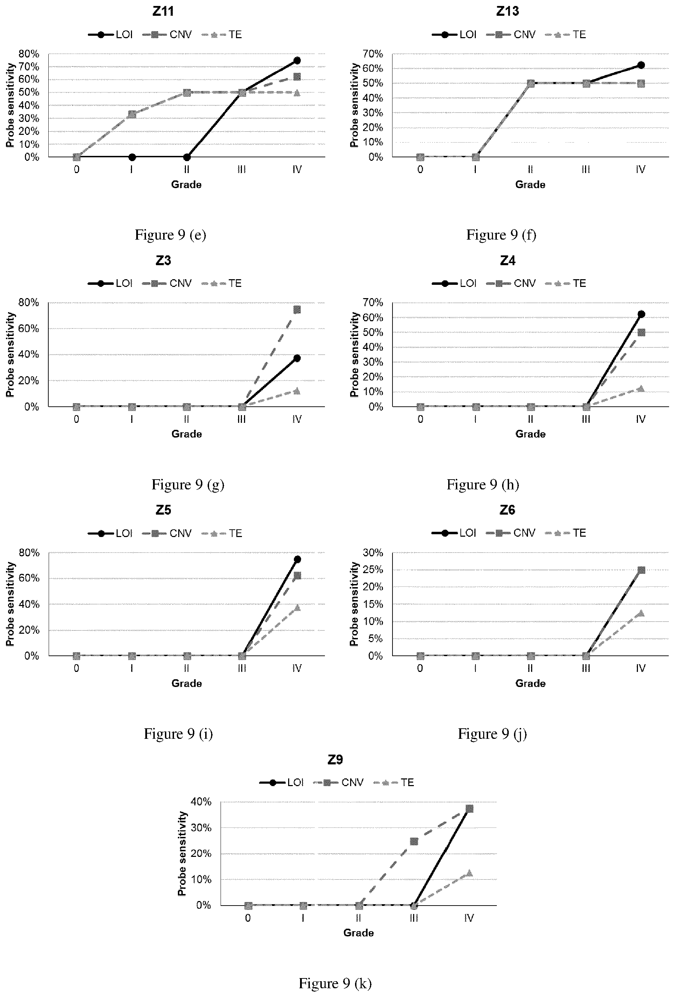

Figure 9(a) indicates the intensity of the loss of imprinting, copy number variation, and total expression of the imprinted gene Z1, figure 9(b) indicates the intensity of the loss of imprinting, copy number variation, and total expression of the imprinted gene Z16, figure 9(c) indicates the intensity of the loss of imprinting, copy number variation, and total expression of the imprinted gene Z8, figure 9(d) indicates the intensity of the loss of imprinting, copy number variation, and total expression of the imprinted gene Z10, figure 9(e) indicates the intensity of the loss of imprinting, copy number variation, and total expression of the imprinted gene Z11, figure 9(f) indicates the intensity of the loss of imprinting, copy number variation, and total expression of the imprinted gene Z13, figure 9(g) indicates the intensity of the loss of imprinting, copy number variation, and total expression of the imprinted gene Z3, figure 9(h) indicates the intensity of the loss of imprinting, copy number variation, and total expression of the imprinted gene Z4, figure 9(i) indicates the intensity of the loss of imprinting, copy number variation, and total expression of the imprinted gene Z5, figure 9(j) indicates the intensity of the loss of imprinting, copy number variation, and total expression of the imprinted gene Z6, figure 9(k) indicates the intensity of the loss of imprinting, copy number variation, and total expression of the imprinted gene Z9, wherein, LOI is the expression of an imprinted gene with loss of imprinting, CNV is the expression of an imprinted gene with copy number variation, and TE is the total expression of an imprinted gene;

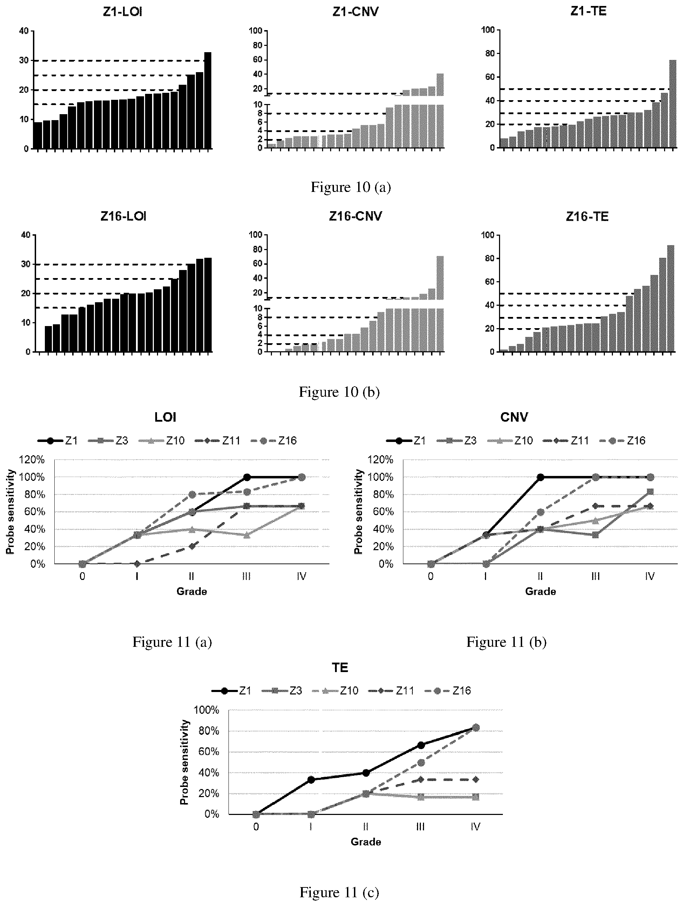

Figure 10 demonstrates the distribution range and grading standards of the loss of imprinting, copy number variation and the total expression of imprinted genes Z1 and Z16 in aspiration cell samples of 21 pancreatic cancers, figure 10(a) shows the distribution range and grading standards of loss of imprinting, copy number variation and total expression of Z1 in aspiration cell samples of 21 pancreatic cancers, figure 10(b) shows the distribution range and grading standards of loss of imprinting, copy number variation and total expression of Z16 in aspiration cell samples of 21 pancreatic cancers;

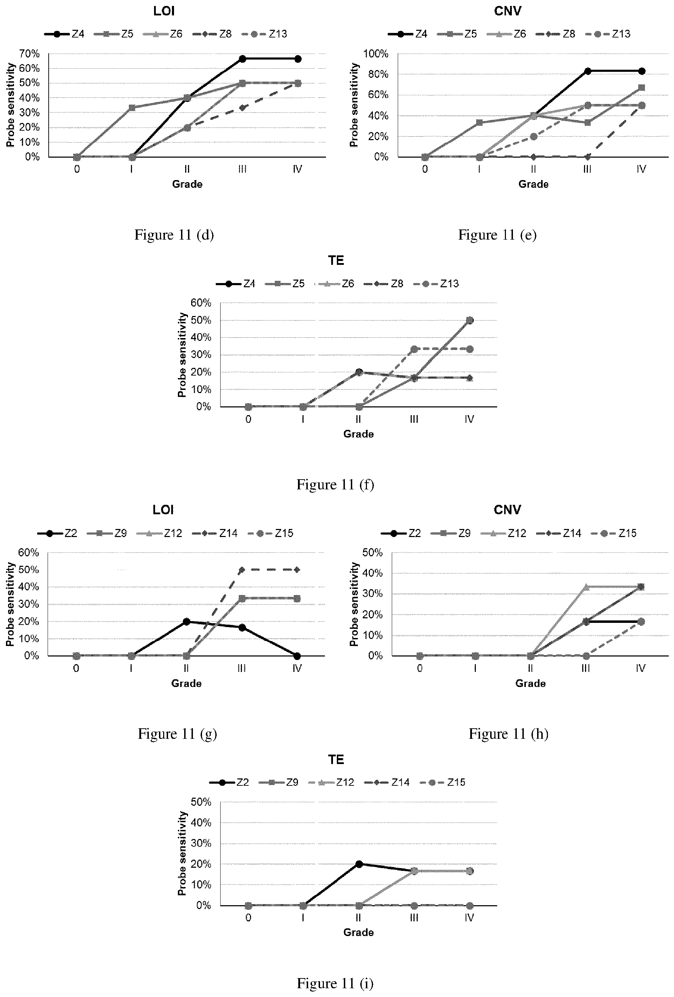

Figure 11(a) indicates the intensity of the loss of imprinting of Z1, Z3, Z10, Z11, and Z16 in pancreatic cancer, figure 11(b) indicates the intensity of the copy number variation of Z1, Z3, Z10, Z11, and Z16 in pancreatic cancer, figure 11(c) indicates the intensity of the total expression of Z1, Z3, Z10, Z11, and Z16 in pancreatic cancer, figure 11(d) indicates the intensity of the loss of imprinting of Z4, Z5, Z6, Z8, and Z13 in pancreatic cancer, figure 11(e) indicates the intensity of the copy number variation of Z4, Z5, Z6, Z8, and Z13 in pancreatic cancer, figure 11(f) indicates the intensity of the total expression of Z4, Z5, Z6, Z8, and Z13 in pancreatic cancer, figure 11(g) indicates the intensity of the loss of imprinting of Z2, Z9, Z12, Z14, and Z15 in pancreatic cancer, figure 11(h) indicates the intensity of the copy number variation of Z2, Z9, Z12, Z14, and Z15 in pancreatic cancer, figure 11(i) indicates the intensity of the total expression of Z2, Z9, Z12, Z14, and Z15 in pancreatic cancer, wherein, LOI means the expression level of genes with lost imprinting in the imprinted genes, CNV stands for the expression of gene with copy number variation in the imprinted genes, and TE stands for the total expression of imprinted genes;

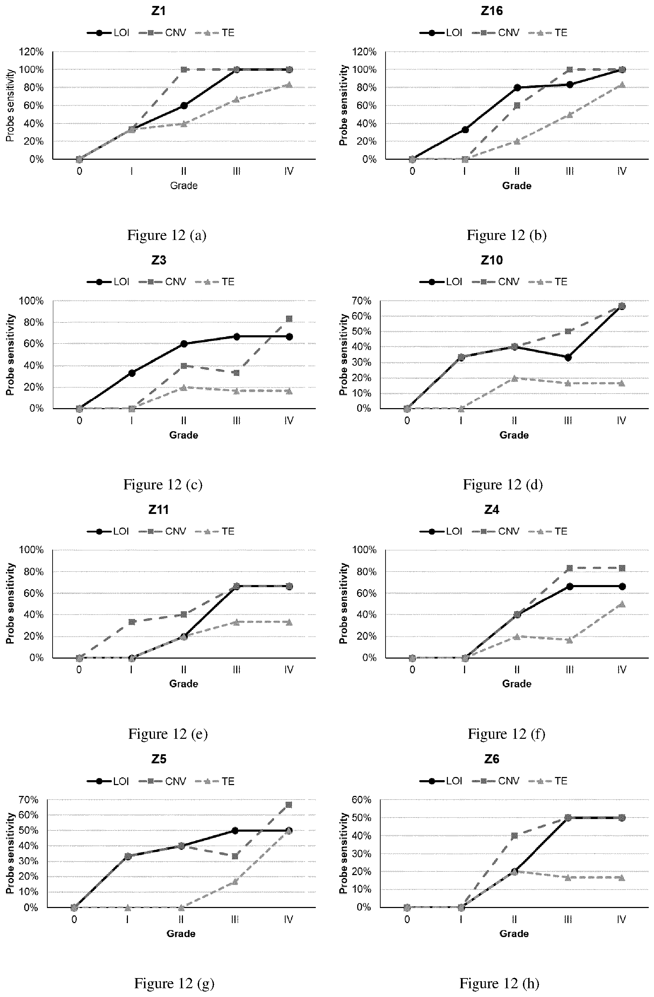

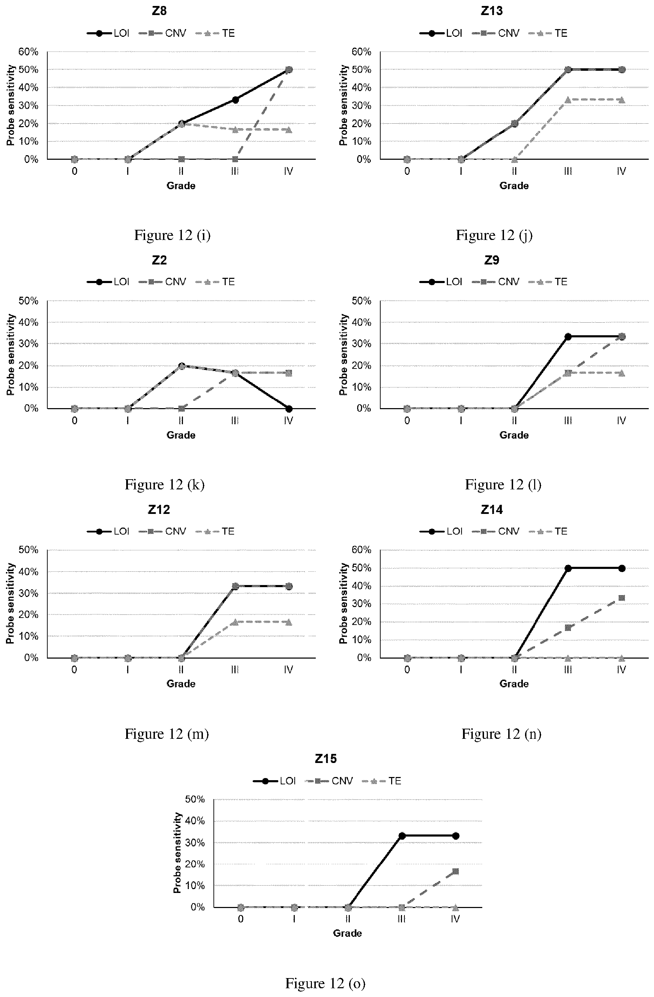

Figure 12(a) indicates the intensity of the loss of imprinting, copy number variation, and total expression of the imprinted gene Z1, figure 12(b) indicates the intensity of the loss of imprinting, copy number variation, and total expression of the imprinted gene Z16, figure 12(c) indicates the intensity of the loss of imprinting, copy number variation, and total expression of the imprinted gene Z3, figure 12(d) indicates the intensity of the loss of imprinting, copy number variation, and total expression of the imprinted gene Z10, figure 12(e) indicates the intensity of the loss of imprinting, copy number variation, and total expression of the imprinted gene Z11, figure 12(f) indicates the intensity of the loss of imprinting, copy number variation, and total expression of the imprinted gene Z4, figure 12(g) indicates the intensity of the loss of imprinting, copy number variation, and total expression of the imprinted gene Z5, figure 12(h) indicates the intensity of the loss of imprinting, copy number variation, and total expression of the imprinted gene Z6, figure 12(i) indicates the intensity of the loss of imprinting, copy number variation, and total expression of the imprinted gene Z8, figure 12(j) indicates the intensity of the loss of imprinting, copy number variation, and total expression of the imprinted gene Z13, figure 12(k) indicates the intensity of the loss of imprinting, copy number variation, and total expression of the imprinted gene Z2, figure 12(l) indicates the intensity of the loss of imprinting, copy number variation, and total expression of the imprinted gene Z9, figure 12(m) indicates the intensity of the loss of imprinting, copy number variation, and total expression of the imprinted gene Z12, figure 12(n) indicates the intensity of the loss of imprinting, copy number variation, and total expression of the imprinted gene Z14, figure 12(o) indicates the intensity of the loss of imprinting, copy number variation, and total expression of the imprinted gene Z15, wherein, LOI is the expression of an imprinted gene with loss of imprinting, CNV is the expression of an imprinted gene with copy number variation, and TE is the total expression of an imprinted gene;

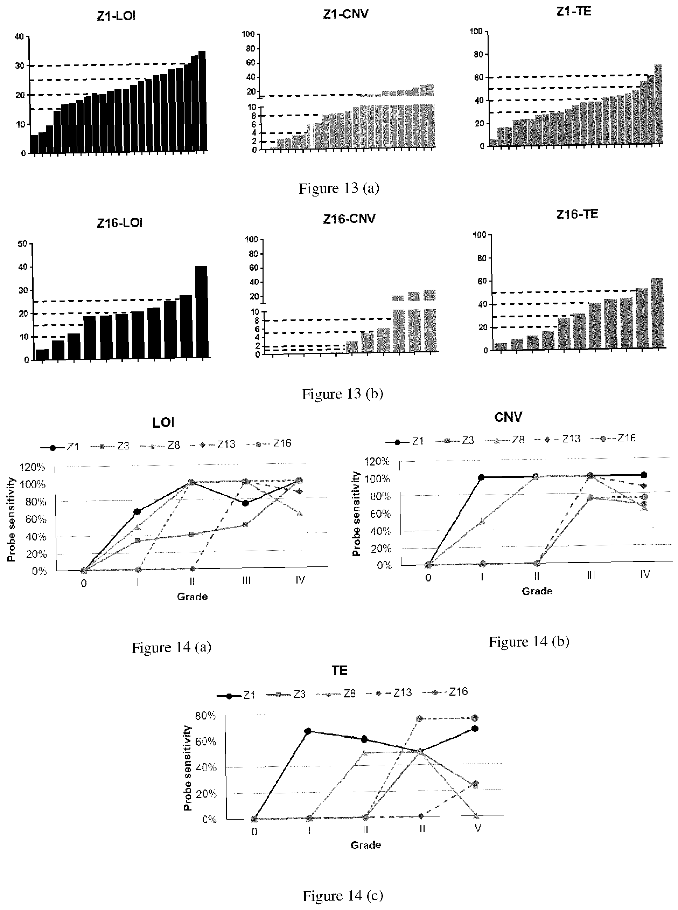

Figure 13 demonstrates the distribution range and grading standards of the loss of imprinting, the copy number variation and the total expression of Z1 and Z16 in 23 bronchial brush cell samples, figure 13(a) shows the distribution range and grading standards of the loss of imprinting, the copy number variation and the total expression of Z1 in 23 bronchial brush cell samples, figure 13(b) shows the distribution range and grading standards of the loss of imprinting, the copy number variation and the total expression of Z16 in 23 bronchial brush cell samples;

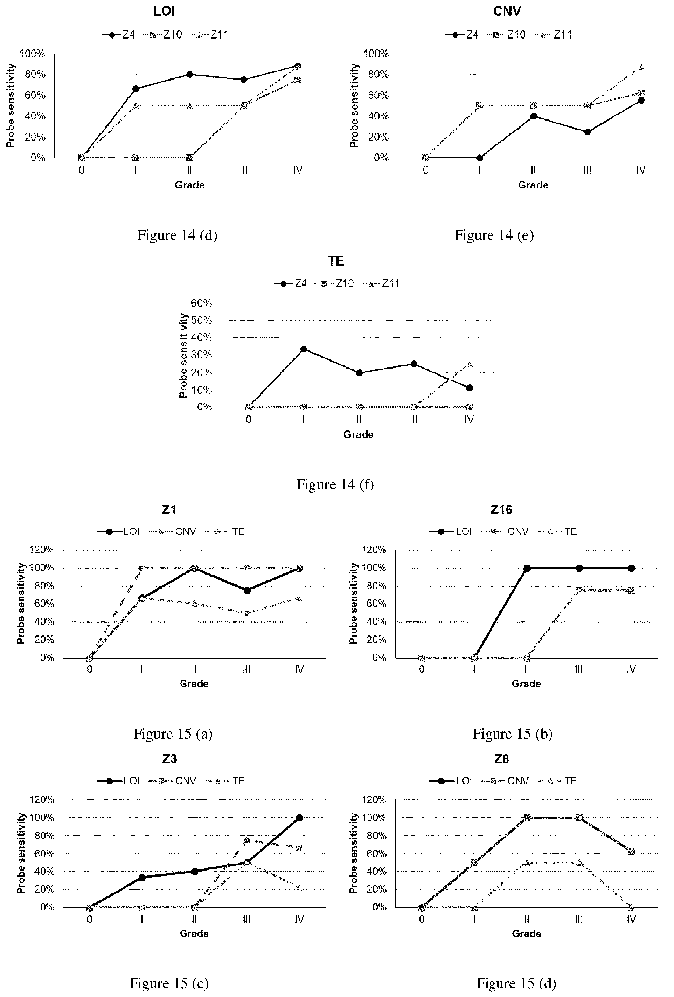

Figure 14(a) indicates the intensity of the loss of imprinting of Z1, Z3, Z8, Z13, and Z16 in lung cancer, figure 14(b) indicates the intensity of the copy number variation of Z1, Z3, Z8, Z13, and Z16 in lung cancer, figure 14(c) indicates the intensity of the total expression of Z1, Z3, Z8, Z13, and Z16 in lung cancer, figure 14(d) indicates the intensity of the loss of imprinting of Z4, Z10, and Z11 in lung cancer, figure 14(e) indicates the intensity of the copy number variation of Z4, Z10, and Z11 in lung cancer, figure 14(f) indicates the intensity of the total expression of Z4, Z10, and Z11 in lung cancer, Wherein, LOI is the expression of an imprinted gene with loss of imprinting, CNV is the expression of an imprinted gene with copy number variation, and TE stands for the total expression of an imprinted gene;

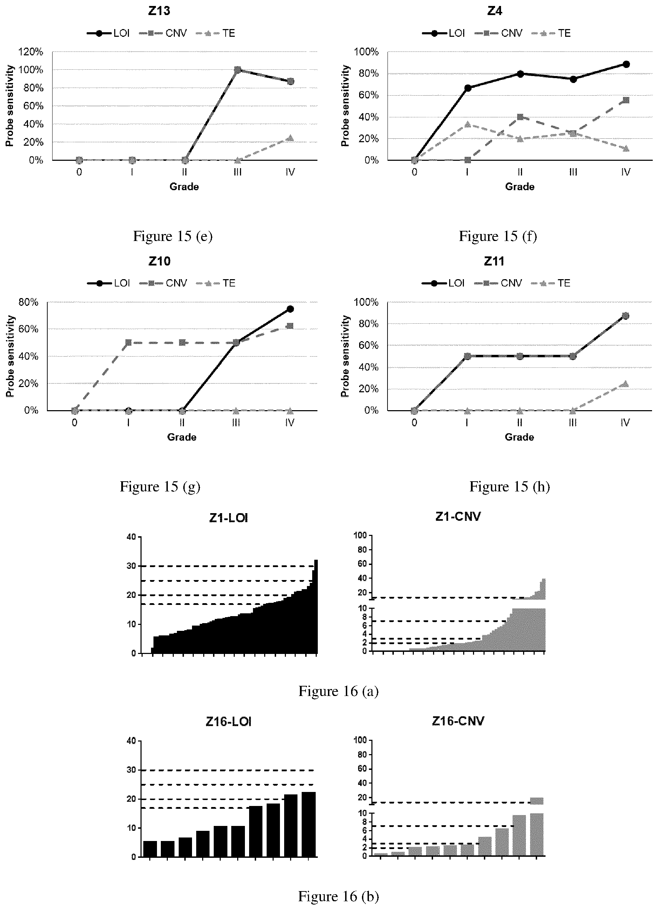

Figure 15(a) indicates the intensity of the loss of imprinting, copy number variation, and total expression of the imprinted gene Z1, figure 15(b) indicates the intensity of the loss of imprinting, copy number variation, and total expression of the imprinted gene Z16, figure 15(c) indicates the intensity of the loss of imprinting, copy number variation, and total expression of the imprinted gene Z3, figure 15(d) indicates the intensity of the loss of imprinting, copy number variation, and total expression of the imprinted gene Z8, figure 15(e) indicates the intensity of the loss of imprinting, copy number variation, and total expression of the imprinted gene Z13, figure 15(f) indicates the intensity of the loss of imprinting, copy number variation, and total expression of the imprinted gene Z4, figure 15(g) indicates the intensity of the loss of imprinting, copy number variation, and total expression of the imprinted gene Z10, figure 15(h) indicates the intensity of the loss of imprinting, copy number variation, and total expression of the imprinted gene Z11, wherein, LOI is the expression of an imprinted gene with loss of imprinting, CNV is the expression of an imprinted gene with copy number variation, and TE is the total expression of an imprinted gene;

Figure 16 demonstrates the distribution range and grading standards of the loss of imprinting, the copy number variation and the total expression of Z1 and Z16 used in 70 exfoliated cell samples from urine, figure 16(a) shows the distribution range and grading standards of the loss of imprinting, the copy number variation and the total expression of Z1 used in 70 exfoliated cell samples from urine, figure 16(b) shows the distribution range and grading standards of the loss of imprinting, and the copy number variation of Z16 used in 70 exfoliated cell samples from urine;

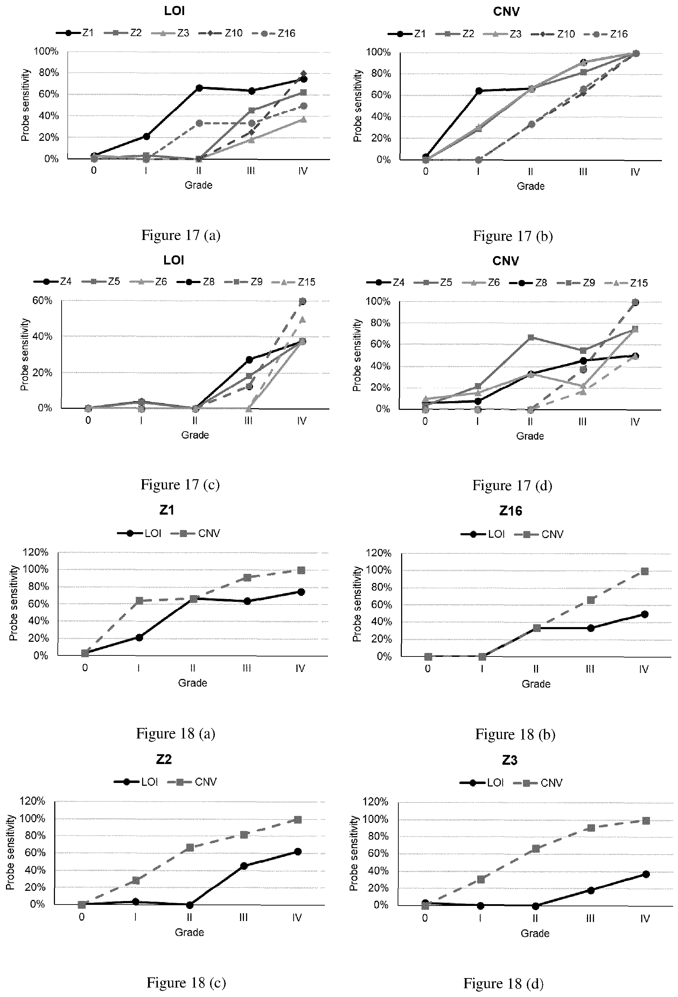

Figure 17(a) indicates the intensity of the loss of imprinting of Z1, Z2, Z3, Z10, and Z16 in urinary system cancer, figure 17(b) indicates the intensity of the copy number variation of Z1, Z2, Z3, Z10, and Z16 in urinary system cancer, figure 17(c) indicates the intensity of the loss of imprinting of Z4, Z5, Z6, Z8, Z9, and Z15 in urinary system cancer, figure 17(d) indicates the intensity of the copy number variation of Z4, Z5, Z6, Z8, Z9, and Z15 in urinary system cancer, wherein, LOI is the expression of an imprinted gene with loss of imprinting, and CNV is the expression of an imprinted gene with copy number variation;

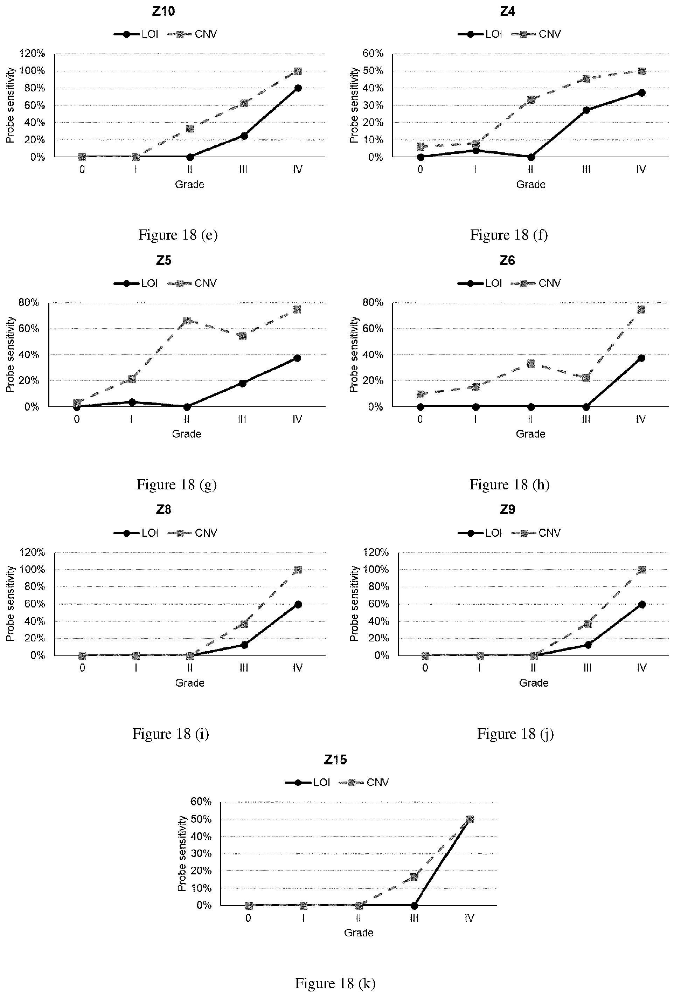

Figure 18(a) indicates the intensity of the loss of imprinting, copy number variation, and total expression of the imprinted gene Z1, figure 18(b) indicates the intensity of the loss of imprinting, copy number variation, and total expression of the imprinted gene Z16, figure 18(c) indicates the intensity of the loss of imprinting, copy number variation, and total expression of the imprinted gene Z2, figure 18(d) indicates the intensity of the loss of imprinting, copy number variation, and total expression of the imprinted gene Z3, figure 18(e) indicates the intensity of the loss of imprinting, copy number variation, and total expression of the imprinted gene Z10, figure 18(f) indicates the intensity of the loss of imprinting, copy number variation, and total expression of the imprinted gene Z4, figure 18(g) indicates the intensity of the loss of imprinting, copy number variation, and total expression of the imprinted gene Z5, figure 18(h) indicates the intensity of the loss of imprinting, copy number variation, and total expression of the imprinted gene Z6, figure 18(i) indicates the intensity of the loss of imprinting, copy number variation, and total expression of the imprinted gene Z8 figure 18(j) indicates the intensity of the loss of imprinting, copy number variation, and total expression of the imprinted gene Z9, figure 18(k) indicates the intensity of the loss of imprinting, copy number variation, and total expression of the imprinted gene Z15, wherein, LOI is the expression of an imprinted gene with loss of imprinting, and CNV is the expression of an imprinted gene with copy number variation;

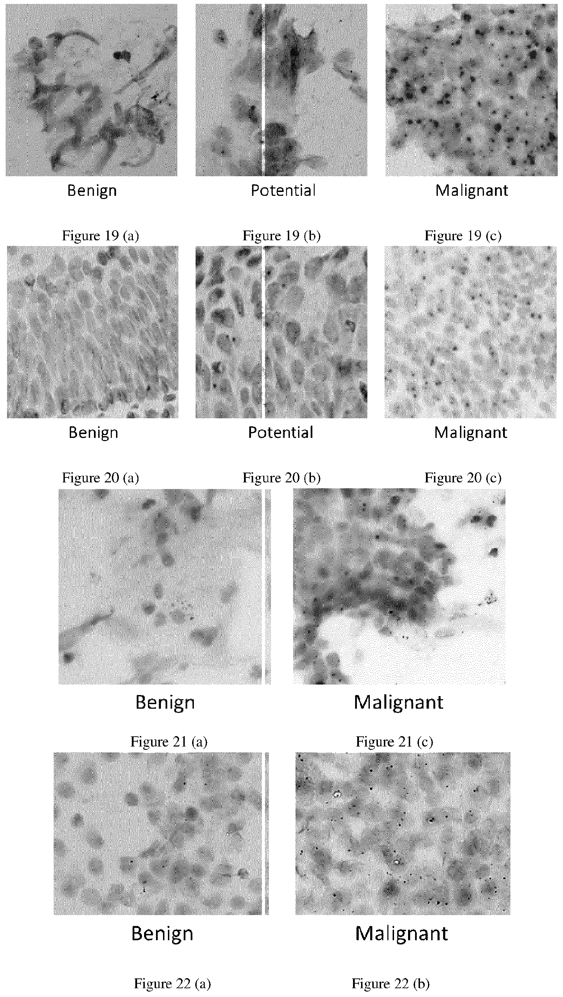

Figure 19(a) shows the expression status of the 16 imprinted genes in the colonoscopy biopsy samples from the benign colorectal tumor, figure 19(b) shows the expression status of the 16 imprinted genes in the colonoscopy biopsy samples from the malignant potential colorectal tumor, figure 19(c) shows the expression status of the 16 imprinted genes in the colonoscopy biopsy samples from the colorectal cancer;

Figure 20(a) shows the expression status of the 16 imprinted genes in the cystoscopy biopsy samples from the benign bladder tumor, figure 20(b) shows the expression status of the 16 imprinted genes in the cystoscopy biopsy samples from the malignant potential bladder tumor, figure 20(c) shows the expression status of the 16 imprinted genes in the cystoscopy biopsy samples from the bladder cancer;

Figure 21(a) shows the expression status of the 16 imprinted genes in the sputum cell samples from the benign lung tumor, figure 21(b) shows the expression status of the 16 imprinted genes in the sputum cell samples from the lung cancer;

Figure 22(a) shows the expression status of the 16 imprinted genes in the aspiration cell smear from the benign liver tumor, figure 22(b) shows the expression status of the 16 imprinted genes in the aspiration cell smear from the liver cancer;

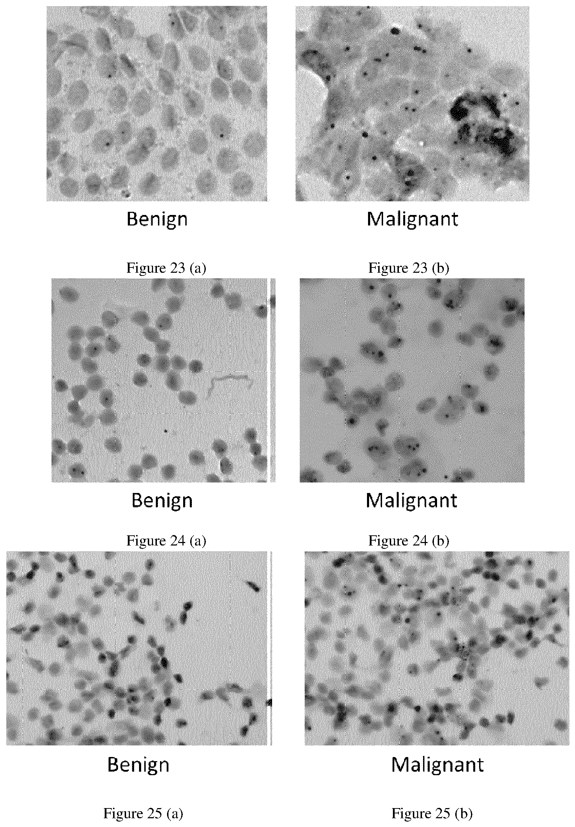

Figure 23(a) shows the expression status of the 16 imprinted genes in the aspiration cell smear from the benign prostate tumor, figure 23(b) shows the expression status of the 16 imprinted genes in the aspiration cell smear from the prostate cancer;

Figure 24(a) shows the expression status of the 16 imprinted genes in the aspiration cell smear from the benign breast tumor without metastasis, figure 24(b) shows the expression status of the 16 imprinted genes in the aspiration cell smear from the lymph node adjacent to the metastatic breast cancer;

Figure 25(a) shows the expression status of the 16 imprinted genes in the plural effusion cell samples from the benign lung tumor, figure 25(b) shows the expression status of the 16 imprinted genes in the plural effusion cell samples from the lung cancer;

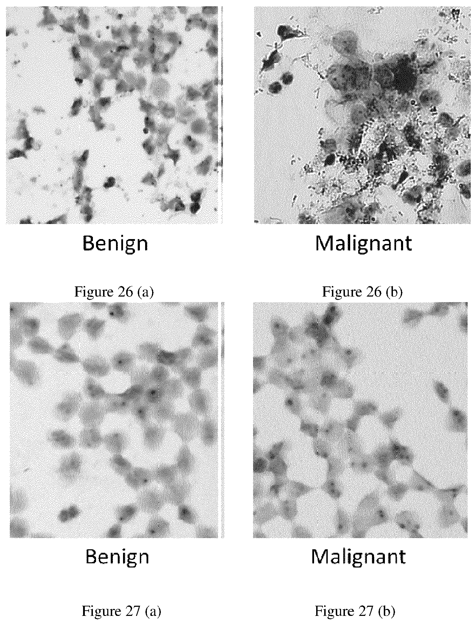

Figure 26(a) shows the expression status of the 16 imprinted genes in the faeces exfoliated cell samples from the benign colorectal tumor, figure 26(b) shows the expression status of the 16 imprinted genes in the faeces exfoliated cell samples from the colorectal cancer;

Figure 27(a) shows the expression status of the 16 imprinted genes in the blood samples from the healthy. figure 27(b) shows the expression status of the 16 imprinted genes in the blood samples from the leukemia patient.

## SPECIFIC IMPLEMENTATION MODALITIES

To further elaborate the technological methods taken in the present application and their effects, the technological scheme in the present application is further explained below with reference to the attached figures and embodiments, but the present application is not limited to the scope of the embodiments.

Example 1 Imprinted Gene Analysis in the Aspiration Cell Samples

The imprinted gene detection method includes the following steps:

(1) The aspiration cell sample was obtained using the biopsy needle, and then fixed in 10% neutral buffered formalin solution to protect the RNA from degradation. 24h later, the fixed sample was proceeded for the cell smear slide preparation.

(2) The sample was pretreated according to the RNAScope sample pretreatment protocol to block the endogenous peroxidase activity, increase the permeability and expose the RNA molecules.

(3) Probe design: specific primers were designed according to the sequence of the imprinted genes.

The probes were designed according to the imprinted genes Z1 (Gnas), Z2 (Igf2), Z3 (Peg10), Z4 (Igf2r), Z5 (Mest), Z6 (Plagl1), Z7 (Cdkn1c), Z8 (Dcn), Z9 (Dlk1), Z10 (Gatm), Z11 (Grb10), Z12 (Peg3), Z13 (Sgce), Z14 (Slc38a4), Z15 (Diras3), and Z16 (Snrpn/Snurf). Specifically, a sequence within an intron of each gene was selected for probe design. Specific probes were design by Advanced Cell Diagnostics.

(4) RNAScope in situ hybridization was performed on the samples using the probes of step (3) according to the protocol of the kit.

(5) After signal amplification and hematoxylin staining, the expression of the imprinted genes were analyzed through microscopic imaging.

In the model of the present application, the formulas for calculating the expression of an imprinted gene, the expression of an imprinted gene with loss of imprinting and the expression of an imprinted gene with copy number variation are as follows:

$$\text{Total expression of an imprinted gene} = (b+c+d)/(a+b+c+d) \times 100\%;$$

$$\text{Expression of a normal imprinted gene} = b/(b+c+d) \times 100\%;$$

$$\text{Expression of an imprinted gene with loss of imprinting} = c/(b+c+d) \times 100\%;$$

$$\text{Expression of an imprinted gene with copy number variation} = d/(b+c+d) \times 100\%;$$

wherein, a, b, c, and d are illustrated in the Figure 2, "a" represents cell nuclei with one red/brown mark inside after performing hematoxylin staining on cells, which means the imprinted gene has no expression in the cell nuclei; "b" represents cell nuclei with one red/brown mark inside after performing hematoxylin staining on cells, which means the imprinted gene exists in the cell nuclei; "c" represents cell nuclei with one red/brown mark inside after performing hematoxylin staining on cells, which means the imprinted gene loses imprinting in the cell nuclei; and "d" represents cell nuclei with one red/brown mark inside after performing hematoxylin staining on cells, which means the imprinted gene has copy number variation in the cell nuclei.

Take the thyroid cancer as an example, the detection results are shown in Figure 3 (a)-3(e). As shown in Figure 3 (a)-3(e), from grade 0 to grade IV, the proportion of the cells having imprinted gene with loss of imprinting (there are two marks in one nucleus) and cells having imprinted gene with copy number variation (there are three or more marks in one nucleus) increase with the stage of malignant tumor.

**Example 2 Imprinted Gene Analysis in 177 Aspiration Biopsy Cell Samples from Thyroid**

177 thyroid aspiration biopsy cell samples were obtained by needle aspiration. Other methods were the same as in Example 1. The results are shown in Figure 4 (a)-4 (b), Figure 5(a)-5(f), and Figure 6 (a)-6(i).

As shown in Figure 4 (a), for the imprinted gene Z1, grade 0 is defined as when the expression of the imprinted gene with loss of imprinting is less than 15%, and/or the expression of imprinted gene with copy number variation is less than 1.5%, and/or the total expression of imprinted gene is less than 40%. Grade I is defined as when the expression of the imprinted gene with loss of imprinting is between 15-20%, and/or the expression of imprinted gene with copy number variation is between 1.5-4%, and/or the total expression of imprinted gene is between 40-45%. Grade II is defined as when the expression of the imprinted gene with loss of imprinting is between 20-30%, and/or the expression of imprinted gene with copy number variation is between 4-8%, and/or the total expression of imprinted gene is between 45-60%. Grade III is defined as when the expression of the imprinted gene with loss of imprinting is between 30-40%, and/or the expression of imprinted gene with copy number variation is between 8-15%, and/or the total expression of imprinted gene is between 60-65%. Grade IV is defined as when the expression of the imprinted gene with loss of imprinting is more than 40%, and/or the expression of imprinted gene with copy number variation is more than 15%, and/or the total expression of imprinted gene is more than 65%.

As shown in Figure 4 (b), for the imprinted gene Z16, grade 0 is defined as when the expression of imprinted gene with loss of imprinting is less than 15%, and/or the expression of imprinted gene with copy number variation is less than 1.5%, and/or the total expression of imprinted gene is less than 40%. Grade I is defined as when the expression of imprinted gene with loss of imprinting is between 15-20%, and/or the expression of imprinted gene with copy number variation is between 1.5-4%, and/or the total expression of imprinted gene is between 40-45%. Grade II is defined as when the expression of imprinted gene with loss of imprinting is between 20-30%, and/or the expression of imprinted gene with copy number variation is between 4-8%, and/or the total expression of imprinted gene is between 45-60%. Grade III is defined as when the expression of imprinted gene with loss of imprinting is between 30-40%, and/or the expression of imprinted gene with copy number variation is between 8-15%, and/or the total expression of imprinted gene is between 60-65%. Grade IV is defined as when the expression of imprinted gene with loss of imprinting is more than 40%, and/or the expression of imprinted gene with copy number variation is more than 15%, and/or the total expression of imprinted gene is more than 65%.

From Figure 5(a)-5(f), it can be observed that the sensitivity for thyroid cancer and the intensity and status of loss of imprinting in thyroid cancer is different between imprinted genes Z1, Z2, Z3, Z4, Z5, Z6, Z7, Z8, Z9, Z10, Z11, Z12, Z13, Z14, Z15, and Z16. Among them, Z1, Z16, Z4, Z11, and Z13 show higher sensitivities.

From the comprehensive analysis of these 177 aspiration biopsy cell samples from thyroid, it can be concluded that:

The thyroid tumor is determined as a benign tumor, if the expression of both Z1 and Z16 with loss of imprinting are lower than grade I; if the expression of only one of Z1 and Z16 with loss of imprinting is grade I; if the expression of only one of Z1 and Z16 with copy number variation is grade I;

The thyroid tumor is determined as thyroid cancer potential, if the expression of both Z1 and Z16 with loss of imprinting are grade I; if the expression of both Z1 and Z16 with copy number variation are grade I; if the expression of only one of Z1 and Z16 with loss of imprinting is grade I and the expression of only one of Z1 and Z16 with copy number variation is grade I; if the expression of only one of Z1 and Z16 with loss of imprinting is grade II; if the expression of only one of Z1 and Z16 with copy number variation is grade II;

The thyroid tumor is determined as an early-stage thyroid cancer; if the expression of both Z1 and Z16 with loss of imprinting are grade II, and/or the expression of both Z1 and Z16 with copy number variation are grade II; if the expression of only one of Z1 and Z16 with loss of imprinting is grade II and the expression of only one of Z1 and Z16 with copy number variation is grade II; if the expression of only one of Z1 and Z16 with loss of imprinting is grade III; if the expression of only one of Z1 and Z16 with copy number variation is grade III;

The thyroid tumor is determined as a medium-stage thyroid cancer; if the expression of both Z1 and Z16 with loss of imprinting are grade III; if the expression of both Z1 and Z16 with copy number variation are grade III; if the expression of only one of Z1 and Z16 with loss of imprinting is grade III and the expression of only one of Z1 and Z16 with copy number variation is grade III; if the expression of only one of Z1 or Z16 with loss of imprinting is grade IV; if the expression of only one of Z1 or Z16 with copy number variation is grade IV;

The thyroid tumor is determined as a late-stage thyroid cancer if the expression of both Z1 and Z16 with loss of imprinting are grade IV, and/or the expression of both Z1 and Z16 with copy number variation are grade IV.

The sensitivity of each imprinted gene for thyroid cancer is shown in figure 6 (a)-figure6 (i):

The loss of imprinting, copy number variation and the total expression of imprinted gene Z1 increased rapidly at cancer potential stage, reached a high sensitivity in early-stage thyroid cancer, and maintained a high level in medium- and late-stage thyroid cancer; The loss of imprinting of imprinted gene Z16 increased rapidly at cancer potential stage, and maintained a high level during the progression of thyroid cancer; The copy number variation and total expression of imprinted gene Z16 increased rapidly in early-stage thyroid cancer, and maintained a high level in medium- and late-stage thyroid cancer.

The loss of imprinting and copy number variation of imprinted gene Z4 started increasing at early-stage thyroid cancer, and increased to a high level gradually at medium-stage and late-stage thyroid cancer; The increase of total expression of imprinted gene Z4 first appeared at cancer potential stage, increased with the progression of thyroid cancer, and reached a high level in late-stage thyroid cancer; The loss of imprinting of imprinted gene Z11 increased rapidly at cancer potential stage, but did not increase any more in the progression from early-stage to late-stage thyroid cancer; The copy number variation of imprinted gene Z11 increased rapidly at cancer potential stage, remained stable in early-stage thyroid cancer, and increased again in medium- and late-stage thyroid cancer; The increase of total expression of imprinted gene Z11 started in medium-stage thyroid cancer, and further increased in late-stage thyroid cancer. The copy number variation of imprinted gene Z13 increased rapidly at cancer potential stage, the loss of imprinting and total expression of Z13 started increasing in early-stage thyroid cancer, but remained stable in medium- and late-stage thyroid cancer.

The loss of imprinting and total expression of imprinted gene Z2 started increasing at cancer potential stage, and increased slowly during the progression of early-stage to late-stage thyroid cancer; The copy number variation of imprinted gene Z2 first appeared at cancer potential stage, stopped increasing in early-stage thyroid cancer, increased again in medium-stage thyroid cancer, and remained stable in late-stage thyroid cancer. The loss of imprinting and increase of total expression of imprinted gene Z3 first appeared at cancer potential stage, increased slowly during the progression of thyroid cancer, while the sensitivities were still not high in late-stage thyroid cancer; The copy number variation of imprinted gene Z3 was quite low before medium-stage thyroid cancer, while increased to a high level rapidly in late-stage thyroid cancer. The loss of imprinting and copy number variation of imprinted gene Z5 increased rapidly in early-stage thyroid cancer, and remained stable in medium- and late-stage thyroid cancer; The increase of total expression of imprinted gene Z5 first appeared in medium-stage thyroid cancer, and remained stable in late-stage thyroid cancer. The loss of imprinting and increase of total expression of imprinted gene Z6 first appeared at cancer potential stage, and maintained a low level during the progression of thyroid cancer; The copy number variation of imprinted gene Z6 first appeared in early-stage thyroid cancer, and maintained a low level in medium- and late-stage thyroid cancer.

Example 3 Imprinted Gene Analysis for 18 breast aspiration cell samples

The breast aspiration cell sample were obtained by needle aspiration. Other methods were the same as in Example 1. The results are shown in figure 7 (a)-7 (b), figure 8 (a)-8 (f), and figure 9 (a)-9 (k).

As shown in figure 7 (a), for the imprinted gene Z1, grade 0 is defined as when the expression of the imprinted gene with loss of imprinting is less than 15%, and/or the expression of imprinted gene with copy number variation is less than 1%, and/or the total expression of imprinted gene is less than 25%. Grade I is defined as when the expression of the imprinted gene with loss of imprinting is between 15-20%, and/or the expression of imprinted gene with copy number variation is between 1-3%, and/or the total expression of imprinted gene is between 25-30%. Grade II is defined as when the expression of the imprinted gene with loss of imprinting is between 20-25%, and/or the expression of imprinted gene with copy number variation is between 3-7%, and/or the total expression of imprinted gene is between 30-40%. Grade III is defined as when the expression of the imprinted gene with loss of imprinting is between 25-30%, and/or the expression of imprinted gene with copy number variation is between 7-10%, and/or the total expression of imprinted gene is between 40-50%. Grade IV is defined as when the expression of the imprinted gene with loss of imprinting is more than 30%, and/or the expression of imprinted gene with copy number variation is more than 10%, and/or the total expression of imprinted gene is more than 50%.

As shown in figure 7 (b), for the imprinted gene Z16, grade 0 is defined as when the expression of the imprinted gene with loss of imprinting is less than 15%, and/or the expression of imprinted gene with copy number variation is less than 1%, and/or the total expression of imprinted gene is less than 25%. Grade I is defined as when the expression of the imprinted gene with loss of imprinting is between 15-20%, and/or the expression of imprinted gene with copy number variation is between 1-3%, and/or the total expression of imprinted gene is between 25-30%. Grade II is defined as when the expression of the imprinted gene with loss of imprinting is between 20-25%, and/or the expression of imprinted gene with copy number variation is between 3-7%, and/or the total expression of imprinted gene is between 30-40%. Grade III is defined as when the expression of the imprinted gene with loss of imprinting is between 25-30%, and/or the expression of imprinted gene with copy number variation is between 7-10%, and/or the total expression of imprinted gene is between 40-50%. Grade IV is defined as when the expression of the imprinted gene with loss of imprinting is more than 30%, and/or the expression of imprinted gene with copy number variation is more than 10%, and/or the total expression of imprinted gene is more than 50%.

From the comprehensive analysis of these 18 breast aspiration cell samples, it can be concluded that:

The breast tumor is determined as a benign tumor, if the expression of both Z1 and Z16 with loss of imprinting are lower than grade I; if the expression of only one of Z1 and Z16 with loss of imprinting is grade I; if the expression of only one of Z1 and Z16 with copy number variation is grade I;

The breast tumor is determined as breast cancer potential, if the expression of both Z1 and Z16 with loss of imprinting are grade I; if the expression of both Z1 and Z16 with copy number variation are grade I; if the expression of only one of Z1 and Z16 with loss of imprinting is grade I and the expression of only one of Z1 and Z16 with copy number variation is grade I; if the expression of only one of Z1 and Z16 with loss of imprinting is grade II; if the expression of only one of Z1 and Z16 with copy number variation is grade II;

The breast tumor is determined as an early-stage breast cancer; if the expression of both Z1 and Z16 with loss of imprinting are grade II, and/or the expression of both Z1 and Z16 with copy number variation are grade II; if the expression of only one of Z1 and Z16 with loss of imprinting is grade II and the expression of only one of Z1 and Z16 with copy number variation is grade II; if the expression of only one of Z1 and Z16 with loss of imprinting is grade III; if the expression of only one of Z1 and Z16 with copy number variation is grade III;

The breast tumor is determined as a medium-stage breast cancer; if the expression of both Z1 and Z16 with loss of imprinting are grade III; if the expression of both Z1 and Z16 with copy number variation are grade III; if the expression of only one of Z1 and Z16 with loss of imprinting is grade III and the expression of only one of Z1 and Z16 with copy number variation is grade III; if the expression of only one of Z1 or Z16 with loss of imprinting is grade IV; if the expression of only one of Z1 or Z16 with copy number variation is grade IV;

The breast tumor is determined as a late-stage breast cancer if the expression of both Z1 and Z16 with loss of imprinting are grade IV, and/or the expression of both Z1 and Z16 with copy number variation are grade IV.

From figure 8 (a)-8 (f), it can be observed that the sensitivity for breast cancer and the intensity and status of loss of imprinting in breast cancer is different between imprinted genes Z1, Z2, Z3, Z4, Z5, Z6, Z7, Z8, Z9, Z10, Z11, Z12, Z13, Z14, Z15, and Z16. Among them, Z1, Z16, Z8, Z10, Z11, and Z13 show higher sensitivities.

The sensitivity of each imprinted gene for breast cancer is shown in figure 9 (a)-figure 9 (k): The loss of imprinting and copy number variation of imprinted gene Z1 increased rapidly at cancer potential stage, achieved a high sensitivity in early-stage breast cancer, and remained high in medium- and late-stage breast cancer; The total expression of imprinted gene Z1 was not high at cancer potential stage, but increased to a high level rapidly in early-stage breast cancer, and maintained a high level in medium- and late-stage breast cancer. The loss of imprinting and copy number variation of imprinted gene Z16 increased rapidly in early-stage breast cancer, and remained high in medium- and late-stage breast cancer; The increase of total expression of imprinted gene Z16 first appeared in early-stage breast cancer, and further increased to a high level in medium- and late-stage thyroid cancer.

The loss of imprinting and copy number variation of imprinted gene Z8 first appeared at cancer potential stage, further increased in early-stage breast cancer, and remained stable in medium- and late-stage breast cancer; The increase of total expression of imprinted gene Z8 first appeared at cancer potential stage, but showed no further increase in the progression of breast cancer. The copy number variation and total expression of imprinted gene Z10 increased rapidly in early-stage breast cancer, but showed no further increase in medium- and late-stage breast cancer; The loss of imprinting of imprinted gene Z10 first appeared in medium-stage breast cancer, and showed a slight increase in late-stage breast cancer. The copy number variation and increase of total expression of imprinted gene Z11 first appeared at cancer potential stage, while the increasing speed slowed down in early-stage breast cancer, and remained stable in medium- and late-stage breast cancer; The loss of imprinting of imprinted gene Z11 first appeared in medium-stage breast cancer, and increased to a high level in late-stage breast cancer. The loss of imprinting, copy number variation and total expression of imprinted gene Z13 increased rapidly in early-stage breast cancer, and remained stable in medium- and late-stage breast cancer. The loss of imprinting, copy number variation and increase of total expression of imprinted gene Z3 first appeared in late-stage breast cancer, wherein the level of copy number variation was relatively high, while the level of loss of imprinting and total expression were relatively low. The loss of imprinting, copy number variation and increase of total expression of imprinted genes Z4 and Z5 first appeared in late-stage breast cancer, wherein the level of loss of imprinting and copy number variation were relatively high, while the level of total expression was relatively low. The loss of imprinting, copy number variation and increase of total expression of imprinted gene Z6 first appeared in late-stage breast cancer, but their levels were not high. The copy number variation of imprinted gene Z9 first appeared in medium-stage breast cancer, and further increased in late-stage breast cancer; The loss of imprinting and increase of total expression of imprinted gene Z9 first appeared in late-stage breast cancer, but their levels were not high.

**Example 4 Imprinted Gene Analysis for 21 Pancreas Aspiration Cell Samples**

21 pancreatic aspiration cell samples were obtained by needle aspiration. Other methods were the same as in Example 1. The results are shown in figure 10 (a)-10 (b), figure 11 (a)-11 (i) and figure 12 (a)-12 (o).

As shown in figure 10 (a), for the imprinted gene Z1, grade 0 is defined as when the expression of the imprinted gene with loss of imprinting is less than 15%, and/or the expression of imprinted gene with copy number variation is less than 2%, and/or the total expression of imprinted gene is less than 20%. Grade I is defined as when the expression of the imprinted gene with loss of imprinting is between 15-20%, and/or the expression of imprinted gene with copy number variation is between 2-4%, and/or the total expression of imprinted gene is between 20-30%. Grade II is defined as when the expression of the imprinted gene with loss of imprinting is between 20-25%, and/or the expression of imprinted gene with copy number variation is between 4-8%, and/or the total expression of imprinted gene is between 30-40%. Grade III is defined as when the expression of the imprinted gene with loss of imprinting is between 25-30%, and/or the expression of imprinted gene with copy number variation is between 8-12%, and/or the total expression of imprinted gene is between 40-50%. Grade IV is defined as when the expression of the imprinted gene with loss of imprinting is more than 30%, and/or the expression of imprinted gene with copy number variation is more than 12%, and/or the total expression of imprinted gene is more than 50%.

As shown in figure 10 (b), for the imprinted gene Z16, grade 0 is defined as when the expression of the imprinted gene with loss of imprinting is less than 15%, and/or the expression of imprinted gene with copy number variation is less than 2%, and/or the total expression of imprinted gene is less than 20%. Grade I is defined as when the expression of the imprinted gene with loss of imprinting is between 15-20%, and/or the expression of imprinted gene with copy number variation is between 2-4%, and/or the total expression of imprinted gene is between 20-30%. Grade II is defined as when the expression of the imprinted gene with loss of imprinting is between 20-25%, and/or the expression of imprinted gene with copy number variation is between 4-8%, and/or the total expression of imprinted gene is between 30-40%. Grade III is defined as when the expression of the imprinted gene with loss of imprinting is between 25-30%, and/or the expression of imprinted gene with copy number variation is between 8-12%, and/or the total expression of imprinted gene is between 40-50%. Grade IV is defined as when the expression of the imprinted gene with loss of imprinting is more than 30%, and/or the expression of imprinted gene with copy number variation is more than 12%, and/or the total expression of imprinted gene is more than 50%.

From the comprehensive analysis of these 21 pancreatic aspiration cell samples, it can be concluded that:

The pancreatic tumor is determined as a benign tumor, if the expression of both Z1 and Z16 with loss of imprinting are lower than grade I; if the expression of only one of Z1 and Z16 with loss of imprinting is grade I; if the expression of only one of Z1 and Z16 with copy number variation is grade I;

The pancreatic tumor is determined as pancreatic cancer potential, if the expression of both Z1 and Z16 with loss of imprinting are grade I; if the expression of both Z1 and Z16 with copy number variation are grade I; if the expression of only one of Z1 and Z16 with loss of imprinting is grade I and the expression of only one of Z1 and Z16 with copy number variation is grade I; if the expression of only one of Z1 and Z16 with loss of imprinting is grade II; if the expression of only one of Z1 and Z16 with copy number variation is grade II;

The pancreatic tumor is determined as an early-stage pancreatic cancer; if the expression of both Z1 and Z16 with loss of imprinting are grade II, and/or the expression of both Z1 and Z16 with copy number variation are grade II; if the expression of only one of Z1 and Z16 with loss of imprinting is grade II and the expression of only one of Z1 and Z16 with copy number variation is grade II; if the expression of only one of Z1 and Z16 with loss of imprinting is grade III; if the expression of only one of Z1 and Z16 with copy number variation is grade III;

The pancreatic tumor is determined as a medium-stage pancreatic cancer; if the expression of both Z1 and Z16 with loss of imprinting are grade III; if the expression of both Z1 and Z16 with copy number variation are grade III; if the expression of only one of Z1 and Z16 with loss of imprinting is grade III and the expression of only one of Z1 and Z16 with copy number variation is grade III; if the expression of only one of Z1 or Z16 with loss of imprinting is grade IV; if the expression of only one of Z1 or Z16 with copy number variation is grade IV;

The pancreatic tumor is determined as a late-stage pancreatic cancer if the expression of both Z1 and Z16 with loss of imprinting are grade IV, and/or the expression of both Z1 and Z16 with copy number variation are grade IV.

From figure 11 (a)-11 (i), it can be observed that the sensitivity for pancreatic cancer and the intensity and status of loss of imprinting in pancreatic cancer is different between imprinted genes Z1, Z2, Z3, Z4, Z5, Z6, Z7, Z8, Z9, Z10, Z11, Z12, Z13, Z14, Z15, and Z16. Among them, Z1, Z16, Z3, Z10, Z11 show higher sensitivities.

The sensitivity of each imprinted gene for pancreatic cancer is shown in figure 12 (a)-12 (o): The loss of imprinting, copy number variation and increase of total expression of imprinted gene Z1 first appeared at cancer potential stage, among which the copy number variation increased in a fastest speed, reached a high level in early-stage pancreatic cancer and remained stable afterwards; The loss of imprinting also increased in a relatively fast speed, and reached a high level in medium-stage pancreatic cancer, The total expression increased slowly, and finally reached a high level in late-stage pancreatic cancer. The loss of imprinting of imprinted gene Z16 first appeared at cancer potential stage, increased gradually during the progression of pancreatic cancer, and reached a high level in late-stage pancreatic cancer; The copy number variation of imprinted gene Z16 first appeared in early-stage pancreatic cancer, increased rapidly in medium-stage pancreatic cancer, and maintained a high level in late-stage pancreatic cancer; The increase of total expression of imprinted gene Z16 first appeared in early-stage pancreatic cancer, kept increasing during the progression of pancreatic cancer, and reached a high level in late-stage pancreatic cancer.

The loss of imprinting of imprinted gene Z3 first appeared at cancer potential stage, kept increasing during the progression of pancreatic cancer, while the speed of increase slowed down in medium- and late-stage pancreatic cancer; The copy number variation of imprinted gene Z3 first appeared in early-stage pancreatic cancer, showed no significant increase in medium-stage pancreatic cancer, but increased again to a high level in late-stage pancreatic cancer; The increase of total expression of imprinted gene Z3 first appeared in early-stage pancreatic cancer, but maintained a low level in medium- and late-stage pancreatic cancer. The loss of imprinting and copy number variation of imprinted gene Z10 first appeared at cancer potential stage, showed no significant increase in early-stage and medium-stage pancreatic cancer, but increased again to a high level in late-stage pancreatic cancer; The increase of total expression of imprinted gene Z10 first appeared in early-stage pancreatic cancer, but maintained a low level in medium- and late-stage pancreatic cancer. The copy number variation of imprinted gene Z11 first appeared at cancer potential stage, increased gradually in early-stage and medium-stage pancreatic cancer, and maintained a high level in late-stage pancreatic cancer; The loss of imprinting and increase of total expression of imprinted gene Z11 first appeared in early-stage pancreatic cancer, among which the loss of imprinting increased at a relatively high speed, and maintained a high level in medium- and late-stage pancreatic cancer, while the total expression increased slowly, and its level was still not high in medium- and late-stage pancreatic cancer. The loss of imprinting and copy number variation of imprinted gene Z4 first appeared in early-stage pancreatic cancer, increased rapidly in medium-stage pancreatic cancer, and maintained a high level in late-stage pancreatic cancer; The increase of total expression of imprinted gene Z4 first appeared in early-stage pancreatic cancer, showed no significant increase in medium-stage pancreatic cancer, while increased again in late-stage pancreatic cancer. The loss of imprinting and copy number variation of imprinted gene Z5 first appeared at cancer potential stage, loss of imprinting showed no significant increase in the progression from early-stage to late-stage pancreatic cancer; copy number variation showed no significant increase in the progression from early-stage to medium-stage pancreatic cancer, but increased again to a high level in late-stage pancreatic cancer; The increase of total expression of imprinted gene Z5 first appeared in medium-stage pancreatic cancer, and continued increasing in late-stage pancreatic cancer but its level was still low. The loss of imprinting and copy number variation of imprinted gene Z6 first appeared in early-stage pancreatic cancer, increased during the progression of pancreatic cancer, and reached a high level in late-stage pancreatic cancer; The increase of total expression of imprinted gene Z6 first appeared in early-stage pancreatic cancer, but maintained a low level in medium- and late-stage pancreatic cancer. The loss of imprinting of imprinted gene Z8 first appeared in early-stage pancreatic cancer, increased slowly during the progression of pancreatic cancer, and reached a high level in late-stage pancreatic cancer; The copy number variation of imprinted gene Z8 appeared in late-stage pancreatic cancer and with a high level; The increase of total expression of imprinted gene Z8 first appeared in early-stage pancreatic cancer, but maintained a low level in medium- and late-stage pancreatic cancer. The loss of imprinting and copy number variation of imprinted gene Z13 first appeared in early-stage pancreatic cancer, increased rapidly in medium-stage pancreatic cancer, and remained stable in late-stage pancreatic cancer; The increase of total expression of imprinted gene Z13 first appeared in medium-stage pancreatic cancer, but stopped increasing in late-stage pancreatic cancer. The loss of imprinting and increase of total expression of imprinted gene Z2 first appeared in early-stage pancreatic cancer, The copy number variation of imprinted gene Z2 first appeared in medium-stage pancreatic cancer, but maintained a low level in medium- and late-stage pancreatic cancer. The loss of imprinting of imprinted gene Z9 first appeared in medium-stage pancreatic cancer, increased slowly during the progression of pancreatic cancer, and its level was still not high in late-stage pancreatic cancer; The copy number variation of imprinted gene Z9 first appeared in medium-stage pancreatic cancer, and kept increasing in late-stage pancreatic cancer, but its level was not high; The increase of total expression of imprinted gene Z9 first appeared in medium-stage pancreatic cancer, but stopped increasing in late-stage pancreatic cancer. The loss of imprinting, copy number variation and increase of total expression of imprinted gene Z12 first appeared in medium-stage pancreatic cancer, but stopped increasing in late-stage pancreatic cancer. The loss of imprinting of imprinted gene Z14 increased rapidly in medium-stage pancreatic cancer, and maintained a high level in late-stage pancreatic cancer; The copy number variation of imprinted gene Z14 first appeared in medium-stage pancreatic cancer, and continued increasing in late-stage pancreatic cancer, but its level was not high; Imprinted gene Z14 did not show significant increase of the total expression during the progression of pancreatic cancer. The loss of imprinting of imprinted gene Z15 first appeared in medium-stage pancreatic cancer, but stopped increasing in late-stage pancreatic cancer; The copy number variation of imprinted gene Z15 first appeared in late-stage pancreatic cancer, but its level was not high; Imprinted gene Z15 did not show significant increase of total expression in the progression of pancreatic cancer.

Example 5 Imprinted Gene Analysis for 23 Bronchial Brush Cell Samples

The lung tumor cell samples were obtained through bronchial brushing. Other methods are the same as in Example 1. The results are shown in figure 13 (a)-13 (b), figure 14 (a)-14 (f) and figure 15 (a)-15 (h).

As shown in figure 13 (a), for the imprinted gene Z1, grade 0 is defined as when the expression of the imprinted gene with loss of imprinting is less than 15%, and/or the expression of imprinted gene with copy number variation is less than 2%, and/or the total expression of imprinted gene is less than 30%. Grade I is defined as when the expression of the imprinted gene with loss of imprinting is between 15-20%, and/or the expression of imprinted gene with copy number variation is between 2-4%, and/or the total expression of imprinted gene is between 30-40%. Grade II is defined as when the expression of the imprinted gene with loss of imprinting is between 20-25%, and/or the expression of imprinted gene with copy number variation is between 4-8%, and/or the total expression of imprinted gene is between 40-50%. Grade III is defined as when the expression of the imprinted gene with loss of imprinting is between 25-30%, and/or the expression of imprinted gene with copy number variation is between 8-12%, and/or the total expression of imprinted gene is between 50-60%. Grade IV is defined as when the expression of the imprinted gene with loss of imprinting is more than 30%, and/or the expression of imprinted gene with copy number variation is more than 12%, and/or the total expression of imprinted gene is more than 60%.

As shown in figure 13 (b), for the imprinted gene Z16, grade 0 is defined as when the expression of the imprinted gene with loss of imprinting is less than 10%, and/or the expression of imprinted gene with copy number variation is less than 1%, and/or the total expression of imprinted gene is less than 25%. Grade I is defined as when the expression of the imprinted gene with loss of imprinting is between 10-15%, and/or the expression of imprinted gene with copy number variation is between 1-2%, and/or the total expression of imprinted gene is between 25-30%. Grade II is defined as when the expression of the imprinted gene with loss of imprinting is between 15-20%, and/or the expression of imprinted gene with copy number variation is between 2-5%, and/or the total expression of imprinted gene is between 30-40%. Grade III is defined as when the expression of the imprinted gene with loss of imprinting is between 20-25%, and/or the expression of imprinted gene with copy number variation is between 5-8%, and/or the total expression of imprinted gene is between 40-50%. Grade IV is defined as when the expression of the imprinted gene with loss of imprinting is more than 25%, and/or the expression of imprinted gene with copy number variation is more than 8%, and/or the total expression of imprinted gene is more than 50%.

From the comprehensive analysis of these 23 bronchial brushing cell samples, it can be concluded that:

The lung tumor is determined as a benign tumor, if the expression of both Z1 and Z16 with loss of imprinting are lower than grade I; if the expression of only one of Z1 and Z16 with loss of imprinting is grade I; if the expression of only one of Z1 and Z16 with copy number variation is grade I;

The lung tumor is determined as lung cancer potential, if the expression of both Z1 and Z16 with loss of imprinting are grade I; if the expression of both Z1 and Z16 with copy number variation are grade I; if the expression of only one of Z1 and Z16 with loss of imprinting is grade I and the expression of only one of Z1 and Z16 with copy number variation is grade I; if the expression of only one of Z1 and Z16 with loss of imprinting is grade II; if the expression of only one of Z1 and Z16 with copy number variation is grade II;

The lung tumor is determined as an early-stage lung cancer; if the expression of both Z1 and Z16 with loss of imprinting are grade II, and/or the expression of both Z1 and Z16 with copy number variation are grade II; if the expression of only one of Z1 and Z16 with loss of imprinting is grade II and the expression of only one of Z1 and Z16 with copy number variation is grade II; if the expression of only one of Z1 and Z16 with loss of imprinting is grade III; if the expression of only one of Z1 and Z16 with copy number variation is grade III;

The lung tumor is determined as a medium-stage lung cancer; if the expression of both Z1 and Z16 with loss of imprinting are grade III; if the expression of both Z1 and Z16 with copy number variation are grade III; if the expression of only one of Z1 and Z16 with loss of imprinting is grade III and the expression of only one of Z1 and Z16 with copy number variation is grade III; if the expression of only one of Z1 or Z16 with loss of imprinting is grade IV; if the expression of only one of Z1 or Z16 with copy number variation is grade IV;

The lung tumor is determined as a late-stage lung cancer if the expression of both Z1 and Z16 with loss of imprinting are grade IV, and/or the expression of both Z1 and Z16 with copy number variation are grade IV.

From 14 (a)-14 (f), it can be observed that the sensitivity for lung cancer and the intensity and status of loss of imprinting in lung cancer is different between imprinted genes Z1, Z2, Z3, Z4, Z5, Z6, Z7, Z8, Z9, Z10, Z11, Z12, Z13, Z14, Z15, and Z16. Among them, Z1, Z16, Z3, Z8, Z13 show higher sensitivities.

The sensitivity of each imprinted gene for lung cancer is shown in figure 15 (a)-figure 15 (h): The loss of imprinting and copy number variation of imprinted gene Z1 increased rapidly at cancer potential stage, and maintained a high level during the progression of lung cancer; The total expression of imprinted gene Z1 increased rapidly at cancer potential stage, while stopped increasing in the progression from early-stage to late-stage lung cancer. The loss of imprinting of imprinted gene Z16 increased rapidly in early-stage lung cancer, and maintained a high level in medium- and late-stage lung cancer; The copy number variation and total expression of imprinted gene Z16 increased rapidly in medium-stage lung cancer, and maintained a high level in late-stage lung cancer.

The loss of imprinting of imprinted gene Z3 first appeared at cancer potential stage, showed no significant increase in early-stage and medium-stage lung cancer, while increased to a high level rapidly in late-stage lung cancer; The copy number variation of imprinted gene Z3 increased to a high level rapidly in medium-stage lung cancer, and remained stable in late-stage lung cancer; The increase of total expression of imprinted gene Z3 first appeared in medium-stage lung cancer, but the increase was not significant in late-stage lung cancer. The loss of imprinting and copy number variation of imprinted gene Z8 first appeared at cancer potential stage, further increase in early-stage lung cancer, and maintained a high level in medium-stage lung cancer, but decreased in late-stage lung cancer; The increase of total expression of imprinted gene Z8 appeared in early-stage and medium-stage lung cancer, but returned to a low level in late-stage lung cancer. The loss of imprinting and copy number variation of imprinted gene Z13 increased rapidly in medium-stage lung cancer, and maintained a high level in late-stage lung cancer; The total expression of imprinted gene Z13 slightly increased in late-stage lung cancer. The loss of imprinting of imprinted gene Z4 increased rapidly at cancer potential stage, and maintained a high level from early-stage to late-stage lung cancer; The copy number variation of imprinted gene Z4 first appeared in early-stage lung cancer, showed no significant increase in medium-stage lung cancer, while increased again to a high level in late-stage lung cancer; The increase of total expression of imprinted gene Z4 first appeared at cancer potential stage, but stopped increasing in the progression of lung cancer. The copy number variation of imprinted gene Z10 increased rapidly at cancer potential stage, and maintained a high level from early-stage to late-stage lung cancer; The loss of imprinting of imprinted gene Z10 increased rapidly in medium-stage lung cancer, and reached a high level in late-stage lung cancer; During the progression of lung cancer, imprinted gene Z10 showed no significant increase of total expression. The loss of imprinting and copy number variation of imprinted gene Z11 increased rapidly at cancer potential stage, and showed no significant increase in early-stage and medium-stage lung cancer, but increased again to a high level in late-stage lung cancer; The total expression of imprinted gene Z11 showed a slight increase only in late-stage lung cancer.

Example 6 Imprinted Gene Analysis for 70 Urine Exfoliated Cell Samples

The cell samples were obtained from the urine of urinary system tumor patients. Other methods were the same as in Example 1. The results are shown in figure 16 (a)-16 (b), figure17 (a)-17 (d) and figure 18 (a)-18 (k).

As shown in figure 16 (a), for the imprinted gene Z1, grade 0 is defined as when the expression of the imprinted gene with loss of imprinting is less than 17%, and/or the expression of imprinted gene with copy number variation is less than 2%. Grade I is defined as when the expression of the imprinted gene with loss of imprinting is between 17-20%, and/or the expression of imprinted gene with copy number variation is between 2-3%. Grade II is defined as when the expression of the imprinted gene with loss of imprinting is between 20-25%, and/or the expression of imprinted gene with copy number variation is between 3-7%. Grade III is defined as when the expression of the imprinted gene with loss of imprinting is between 25-30%, and/or the expression of imprinted gene with copy number variation is between 7-12%. Grade IV is defined as when the expression of the imprinted gene with loss of imprinting is more than 30%, and/or the expression of imprinted gene with copy number variation is more than 12%.

As shown in figure 16 (b), for the imprinted gene Z16, grade 0 is defined as when the expression of the imprinted gene with loss of imprinting is less than 17%, and/or the expression of imprinted gene with copy number variation is less than 2%. Grade I is defined as when the expression of the imprinted gene with loss of imprinting is between 17-20%, and/or the expression of imprinted gene with copy number variation is between 2-3%. Grade II is defined as when the expression of the imprinted gene with loss of imprinting is between 20-25%, and/or the expression of imprinted gene with copy number variation is between 3-7%. Grade III is defined as when the expression of the imprinted gene with loss of imprinting is between 25-30%, and/or the expression of imprinted gene with copy number variation is between 7-12%. Grade IV is defined as when the expression of the imprinted gene with loss of imprinting is more than 30%, and/or the expression of imprinted gene with copy number variation is more than 12%.

From the comprehensive analysis of these 70 urine exfoliated cell samples, it can be concluded that:

The urinary system tumor is determined as a benign tumor, if the expression of both Z1 and Z16 with loss of imprinting are lower than grade I; if the expression of only one of Z1 and Z16 with loss of imprinting is grade I; if the expression of only one of Z1 and Z16 with copy number variation is grade I;

The urinary system tumor is determined as urinary system cancer potential, if the expression of both Z1 and Z16 with loss of imprinting are grade I; if the expression of both Z1 and Z16 with copy number variation are grade I; if the expression of only one of Z1 and Z16 with loss of imprinting is grade I and the expression of only one of Z1 and Z16 with copy number variation is grade I; if the expression of only one of Z1 and Z16 with loss of imprinting is grade II; if the expression of only one of Z1 and Z16 with copy number variation is grade II;

The urinary system tumor is determined as an early-stage urinary system cancer; if the expression of both Z1 and Z16 with loss of imprinting are grade II, and/or the expression of both Z1 and Z16 with copy number variation are grade II; if the expression of only one of Z1 and Z16 with loss of imprinting is grade II and the expression of only one of Z1 and Z16 with copy number variation is grade II; if the expression of only one of Z1 and Z16 with loss of imprinting is grade III; if the expression of only one of Z1 and Z16 with copy number variation is grade III;

The urinary system tumor is determined as a medium-stage urinary system cancer; if the expression of both Z1 and Z16 with loss of imprinting are grade III; if the expression of both Z1 and Z16 with copy number variation are grade III; if the expression of only one of Z1 and Z16 with loss of imprinting is grade III and the expression of only one of Z1 and Z16 with copy number variation is grade III; if the expression of only one of Z1 or Z16 with loss of imprinting is grade IV; if the expression of only one of Z1 or Z16 with copy number variation is grade IV;

The urinary system tumor is determined as a late-stage urinary system cancer if the expression of both Z1 and Z16 with loss of imprinting are grade IV, and/or the expression of both Z1 and Z16 with copy number variation are grade IV.

From figure 17 (a)-7 (d), it can be observed that the sensitivity for urinary system cancer and the intensity and status of loss of imprinting in urinary system cancer is different between imprinted genes Z1, Z2, Z3, Z4, Z5, Z6, Z7, Z8, Z9, Z10, Z11, Z12, Z13, Z14, Z15, and Z16. Among them, Z1, Z16, Z2, Z3, and Z10 show higher sensitivities.

The sensitivity of each imprinted gene for urinary system cancer is shown in figure 18 (a)-figure18 (k): The loss of imprinting of imprinted gene Z1 first appeared at cancer potential stage, further increased in early-stage urinary system cancer, and maintained a high level in medium- and late-stage urinary system cancer; The copy number variation of imprinted gene Z1 increased rapidly at cancer potential stage, and further increased to a high level in the progression from medium-stage to late-stage urinary system cancer. The loss of imprinting of imprinted gene Z16 first appeared in early-stage urinary system cancer, but was not increasing in medium- and late-stage urinary system cancer; The copy number variation of imprinted gene Z16 first appears in early-stage urinary system cancer, and gradually increased to a high level during the progression of urinary system cancer.

The loss of imprinting of imprinted gene Z2 first appeared in medium-stage urinary system cancer, and reached a high level in late-stage urinary system cancer; The copy number variation of imprinted gene Z2 first appeared at cancer potential stage, and gradually increased to a high level during the progression of urinary system cancer. The loss of imprinting of imprinted gene Z3 first appeared in medium-stage urinary system cancer, and kept increasing in late-stage urinary system cancer, but its level was not high; The copy number variation of imprinted gene Z3 first appeared at cancer potential stage, and gradually increased to a high level during the progression of urinary system cancer. The loss of imprinting of imprinted gene Z10 first appeared in medium-stage urinary system cancer, and increased to a high level rapidly in late-stage urinary system cancer; The copy number variation of imprinted gene Z10 first appeared in early-stage urinary system cancer, and gradually increased to a high level during the progression of urinary system cancer. The loss of imprinting of imprinted gene Z4 first appeared in medium-stage urinary system cancer, and kept increasing in late-stage urinary system cancer, but its level was not high; The copy number variation of imprinted gene Z4 first appeared at cancer potential stage, and gradually increased to a high level during the progression of cancer. The loss of imprinting of imprinted gene Z5 first appeared in medium-stage urinary system cancer, and kept increasing in late-stage cancer, but its level was not high; The copy number variation of imprinted gene Z5 first appeared at cancer potential stage, increased rapidly in early-stage cancer, and maintained a high level in medium- and late-stage urinary system cancer. The copy number variation of imprinted gene Z6 first appeared at cancer potential stage, but did not increase to a high level rapidly until late-stage cancer; The loss of imprinting of imprinted gene Z6 slightly increased in late-stage urinary system cancer. The loss of imprinting and copy number variation of imprinted gene Z8 and Z9 first appeared in medium-stage urinary system cancer, and increased to a high level rapidly in late-stage urinary system cancer. The copy number variation of imprinted gene Z15 first appeared in medium-stage urinary system cancer, and increased to a high level rapidly in late-stage cancer; The loss of imprinting of imprinted gene Z15 showed a large increase in late-stage urinary system cancer.

**Example 7 Imprinted Gene Analysis for Colonoscopy Biopsy Cells**

The biopsy samples were obtained under colonoscopy. Other methods were the same as in Example 1. The results are shown in Figure 19 (a)-Figure 19 (c).

As shown in Figure 19 (a)-Figure 19 (c), Figure 19 (a) shows a benign colorectal polyp, Figure 19 (b) shows a malignant potential colorectal tumor, and Figure 19 (c) shows a colorectal cancer. The proportion of cells carrying imprinted genes with loss of imprinting and copy number variation increased gradually with the malignancy of cancer.

**Example 8 Imprinted Gene Analysis for Cystoscopy Biopsy Cells**

The biopsy samples were obtained under cystoscopy. Other methods were the same as in Example 1. The results are shown in Figure 20 (a)-Figure 20 (c).

As shown in Figure 20 (a)-Figure 20 (c), Figure 20 (a) shows a benign bladder tumor, Figure 20 (b) shows a malignant potential bladder tumor, and Figure 20 (c) shows a bladder cancer. The proportion of cells carrying imprinted genes with loss of imprinting and copy number variation increased gradually with the malignancy of cancer.

**Example 9 Imprinted Gene Analysis for Sputum Exfoliated Cells**

The cell samples were obtained from the sputum of lung tumor patients. Other methods were the same as in Example 1. The results are shown in Figure 21 (a)-Figure 21 (b).

As shown in Figure 21 (a)-Figure 21 (b), Figure 21 (a) shows a benign lung tumor, Figure 21 (b) shows a lung cancer. The proportion of cells carrying imprinted genes with loss of imprinting and copy number variation increased gradually with the malignancy of cancer.

**Example 10 Imprinted Gene Analysis for Liver Aspiration Biopsy Cells**

The aspiration biopsy cell samples were obtained by liver puncture. Other methods were the same as in Example 1. The results are shown in Figure 22 (a)-Figure 22 (b).

As shown in Figure 22 (a)-Figure 22 (b), Figure 22 (a) shows a benign liver tumor, Figure 22 (b) shows a liver cancer. The proportion of cells carrying imprinted genes with loss of imprinting and copy number variation increased gradually with the malignancy of cancer.

**Example 11 Imprinted Gene Analysis for Prostate Aspiration Biopsy Cells**

The aspiration biopsy cell samples were obtained by prostate puncture. Other methods were the same as in Example 1. The results are shown in Figure 23 (a)-Figure 23 (b).

As shown in Figure 23 (a)-Figure 23 (b), Figure 23 (a) shows a benign prostate tumor, Figure 23 (b) shows a prostate cancer. The proportion of cells carrying imprinted genes with loss of imprinting and copy number variation increased gradually with the malignancy of cancer.

**Example 12 Imprinted Gene Analysis for Lymph Node Aspiration Biopsy Cells**

The aspiration biopsy cell samples were obtained from lymph nodes adjacent to breast tumor. Other methods were the same as in Example 1. The results are shown in Figure 24 (a)-Figure 24 (b).

As shown in Figure 24 (a)-Figure 24 (b), Figure 24 (a) shows aspiration cells from a lymph node adjacent to a benign non-metastatic breast tumor, Figure 24 (b) shows aspiration cells from a lymph node adjacent to a metastatic breast cancer. Only a few cells in the lymph node adjacent to benign breast tumor carried imprinted gene with loss of imprinting, and no cells were found to have imprinted gene with copy number variation, while a large number of cells in the lymph node adjacent to metastatic breast cancer carried imprinted gene with loss of imprinting and imprinted gene with copy number variation.

**Example 13 Imprinted Gene Analysis for Plural Effusion Cells**

The plural effusion cell samples were obtained by puncture. Other methods were the same as in Example 1. The results are shown in Figure 25 (a)-Figure 25 (b).

As shown in Figure 25 (a)-Figure 25 (b), Figure 25 (a) shows the plural effusion cell from a benign lung tumor patient, Figure 25 (b) shows the plural effusion cell from a lung cancer patient. Only a few cells in the plural effusion of benign lung tumor patient carried imprinted gene with loss of imprinting, and no cells were found to have imprinted gene with copy number variation, while a large number of cells in the plural effusion of lung cancer patient carried imprinted gene with loss of imprinting and imprinted gene with copy number variation.

**Example 14 Imprinted Gene Analysis for Fecal Exfoliated Cells**

The cell samples were obtained from the faeces of colorectal tumor patients. Other methods were the same as in Example 1. The results are shown in Figure 26 (a)-Figure 26 (b).

As shown in Figure 26 (a)-Figure 26 (b), Figure 26 (a) shows a benign colorectal tumor, Figure 26 (b) shows a colorectal cancer. The proportion of cells carrying imprinted genes with loss of imprinting and copy number variation increased gradually with the malignancy of cancer.

**Example 15 Imprinted Gene Analysis for Blood Cell**

The cell samples were obtained from peripheral blood of healthy person and patient with leukemia, and the red blood cells were removed using red blood cell lysis solution. Other methods were the same as in Example 1. The results are shown in Figure 27 (a)-Figure 27 (b).

As shown in Figure 27 (a)-Figure 27 (b), Figure 27 (a) shows the blood cell of healthy person, Figure 27 (b) shows the blood cell of patient with leukemia. Only a few cells in the blood sample of healthy person carried imprinted gene with loss of imprinting, and no cells were found to have imprinted gene with copy number variation, while a large number of cells in the blood sample of patient with leukemia carried imprinted gene with loss of imprinting and imprinted gene with copy number variation.

In summary, the method of the present application presents the characteristic of the loss of imprinting on the thyroid sample, breast sample, pancreatic sample, prostate sample, lymph node aspiration cell sample, bronchial brushing cell sample of the lung, urine, sputum, faeces, plural effusion cell sample, colonoscopy biopsy sample, and cystoscopy biopsy cell sample in an intuitive manner. By *in situ* labeling of imprinted genes, objective, intuitive, early, and accurate detection of changes in imprinted genes is achieved, and quantitative model is also provided, making a significant contribution to the early diagnosis of thyroid cancer, breast cancer, pancreatic cancer, lung cancer, liver cancer, colorectal cancer and urinary system cancer.

The Applicant declares that the present application illustrates the detailed methods of the present invention by the above examples, but not limited to the above detailed examples, that is, it does not mean that the present invention must rely on the detailed methods described above to be implemented. It should be apparent to those skilled in the art that any modifications of the present application, the equivalent replacement of each raw material of the products of the present application, the addition of an auxiliary component, the selection of a specific manner, and the like, are all within the scope of protection and disclosure of the present application.

**Claims**

1. A method for cancer diagnosis by assaying imprinted genes in a tumor based on biopsy samples,

    the method comprising generating an expression profile of imprinted genes by calculating the expression level of imprinted genes showing loss of imprinting, the expression level of imprinted genes showing copy number variation, and the total expression level of imprinted genes in a tumor, to grade the expression of the imprinted genes;

    wherein the expression levels are calculated by the following formulas:

$$\text{total expression level of an imprinted gene} = (b+c+d)/(a+b+c+d)\times100\%;$$

$$\text{the expression level of a normal imprinted gene} = b/(b+c+d)\times100\%;$$

$$\text{the expression level of an imprinted gene showing loss of imprinting} = c/(b+c+d)\times100\%;$$

$$\text{the expression level of an imprinted gene showing copy number variation} = d/(b+c+d)\times100\%;$$

    wherein, "a" represents cell nuclei with no mark inside after performing hematoxylin staining on a cell, which means the imprinted gene is not expressed in the cell nuclei;

"b" represents cell nuclei with a red/brown mark inside after performing hematoxylin staining on a cell, which means the imprinted gene exists in the cell nuclei;

"c" represents cell nuclei with two red/brown marks inside after performing hematoxylin staining on a cell, which means the imprinted gene loses imprinting in the cell nuclei; and

"d" represents cell nuclei with more than two red/brown marks inside after performing hematoxylin staining on a cell, which means the imprinted gene has a copy number variation in the cell nuclei;

wherein the imprinted gene is Z1 or Z16, Z1 is Gnas, and Z16 is Snrpn/Snurf;

the method further comprising classifying the expression profiles of imprinted genes into 5 grades;

the expression profile comprising the expression level of Z1 and Z16 showing loss of imprinting, the expression level of Z1 and Z16 showing copy number variation, and the total expression level of Z1 and Z16 is classified into 5 grades:

grade 0: any one or the combination of at least two of: the expression level of Z1 or Z16 showing loss of imprinting is less than 15%, the expression level of Z1 and Z16 showing copy number variation is less than 2%, or the total expression level of Z1 and Z16 is less than 25%;

grade I: any one or the combination of at least two of: the expression level of Z1 or Z16 showing loss of imprinting is 15-20%, the expression level of Z1 and Z16 showing copy number variation is 2-4%, or the total expression level of Z1 and Z16 is 25-30%;

grade II: any one or the combination of at least two of: the expression level of Z1 or Z16 showing loss of imprinting is 20-25%, the expression level of Z1 and Z16 showing copy number variation is 4-8%, or the total expression level of Z1 and Z16 is 30-40%;

grade III: any one or the combination of at least two of: the expression level of Z1 or Z16 showing loss of imprinting is 25-35%, the expression level of Z1 and Z16 showing copy number variation is 8-12%, or the total expression level of Z1 and Z16 is 40-50%;

grade IV: any one or the combination of at least two of: the expression level of Z1 or Z16 showing loss of imprinting is more than 35%, the expression level of Z1 and Z16 showing copy number variation is more than 12%, or the total expression level of Z1 and Z16 is more than 50%;

the method further comprising classifying the benignity or malignancy of the tumor to be determined as benign tumor, cancer potential, early-stage cancer, medium-stage cancer, and late-stage cancer;

preferably, the tumor is determined as a benign tumor, if the expression levels of both Z1 and Z16 showing loss of imprinting are lower than grade I; if the expression level of only one of Z1 and Z16 showing loss of imprinting is grade I; if the expression level of only one of Z1 and Z16 showing copy number variation is grade I;

preferably, the tumor is determined as cancer potential, if the expression levels of both Z1 and Z16 showing loss of imprinting are grade I; if the expression levels of both Z1 and Z16 showing copy number variation are grade I; if the expression level of only one of Z1 and Z16 showing loss of imprinting is grade I and the expression level of only one of Z1 and Z16 showing copy number variation is grade I; if the expression level of only one of Z1 and Z16 showing loss of imprinting is grade II; if the expression level of only one of Z1 and Z16 showing copy number variation is grade II;

preferably, the tumor is determined as an early-stage cancer, if the expression levels of both Z1 and Z16 showing loss of imprinting are grade II, and/or the expression levels of both Z1 and Z16 showing copy number variation are grade II; if the expression level of only one of Z1 and Z16 showing loss of imprinting is grade II and the expression level of only one of Z1 and Z16 showing copy number variation is grade II; if the expression level of only one of Z1 and Z16 showing loss of imprinting is grade III; if the expression level of only one of Z1 and Z16 showing copy number variation is grade III;

preferably, the tumor is determined as a medium-stage cancer, if the expression levels of both Z1 and Z16 showing loss of imprinting are grade III; if the expression levels of both Z1 and Z16 showing copy number variation are grade III; if the expression level of only one of Z1 and Z16 showing loss of imprinting is grade III and the expression level of only one of Z1 and Z16 showing copy number variation is grade III; if the expression level of only one of Z1 or Z16 showing loss of imprinting is grade IV; if the expression level of only one of Z1 or Z16 showing copy number variation is grade IV;

preferably, the tumor is determined as a late-stage cancer, if the expression levels of both Z1 and Z16 showing loss of imprinting are grade IV, and/or the expression levels of both Z1 and Z16 showing copy number variation are grade IV;

and wherein the tumor includes any one of thyroid tumor, breast tumor, pancreatic tumor, lung tumor, liver tumor, colorectal tumor, bladder tumor, prostate tumor, gastric tumor, esophagus tumor, nasopharyngeal tumor, oral tumor, ovarian tumor, endometrial tumor, cervical tumor, urinary system tumor, central nervous system tumor, parotid tumor, lymphoma, and leukemia.

2. The method according to claim 1, wherein the imprinted genes also include any one or a combination of at least two of Z2, Z3, Z4, Z5, Z6, Z7, Z8, Z9, Z10, Z11, Z12, Z13, Z14 and Z15; wherein the imprinted gene Z2 is Igf2, the imprinted gene Z3 is Peg10, the imprinted gene Z4 is Igf2r, the imprinted gene Z5 is Mest, the imprinted gene Z6 is Plagl1, the imprinted gene Z7 is Cdkn1c, the imprinted gene Z8 is Dcn, the imprinted gene Z9 is Dlk1, the imprinted gene Z10 is Gatm, the imprinted gene Z11 is Grb10, the imprinted gene Z12 is Peg3, the imprinted gene Z13 is Sgce, the imprinted gene Z14 is Slc38a4, the imprinted gene Z15 is Diras3.

3. The method according to any one of claims 1 to 2,
wherein preferably, for thyroid tumor, the expression profile of Z1 is classified into 5 grades:

grade 0: any one or the combination of at least two of: the expression level of Z1 showing loss of imprinting is less than 15%, the expression level of Z1 showing copy number variation is less than 1.5%, or the total expression level of Z1 is less than 40%;
grade I: any one or the combination of at least two of: the expression level of Z1 showing loss of imprinting is 15-20%, the expression level of Z1 showing copy number variation is 1.5-4%, or the total expression level of Z1 is 40-45%;
grade II: any one or the combination of at least two of: the expression level of Z1 showing loss of imprinting is 20-30%, the expression level of Z1 showing copy number variation is 4-8%, or the total expression level of Z1 is 45-60%;
grade III: any one or the combination of at least two of: the expression level of Z1 showing loss of imprinting is 30-40%, the expression level of Z1 showing copy number variation is 8-15%, or the total expression level of Z1 is 60-65%;
grade IV: any one or the combination of at least two of: the expression level of Z1 showing loss of imprinting is more than 40%, the expression level of Z1 showing copy number variation is more than 15%, or the total expression level of Z1 is more than 65%;
for thyroid tumor, the expression profile of Z16 is classified into 5 grades:

grade 0: any one or the combination of at least two of: the expression level of Z16 showing loss of imprinting is less than 15%, the expression level of Z16 showing copy number variation is less than 1.5%, or the total expression level of Z16 is less than 30%;
grade I: any one or the combination of at least two of: the expression level of Z16 showing loss of imprinting is 15-20%, the expression level of Z16 showing copy number variation is 1.5-4%, or the total expression level of Z16 is 30-35%;
grade II: any one or the combination of at least two of: the expression level of Z16 showing loss of imprinting is 20-30%, the expression level of Z16 showing copy number variation is 4-8%, or the total expression level of Z16 is 35-50%;
grade III: any one or the combination of at least two of: the expression level of Z16 showing loss of imprinting is 30-40%, the expression level of Z16 showing copy number variation is 8-15%, or the total expression level of Z16 is 50-55%;
grade IV: any one or the combination of at least two of: the expression level of Z16 showing loss of imprinting is more than 40%, the expression level of Z16 showing copy number variation is more than 15%, or the total expression level of Z16 is more than 55%;
preferably, for breast tumor, the expression profile of Z1 and Z16 is classified into 5 grades:

grade 0: any one or the combination of at least two of: the expression level of Z1 and Z16 showing loss of imprinting is less than 15%, the expression level of Z1 and Z16 showing copy number variation is less than 1%, or the total expression level of Z1 and Z16 is less than 25%;
grade I: any one or the combination of at least two of: the expression level of Z1 and Z16 showing loss of imprinting is 15-20%, the expression level of Z1 and Z16 showing copy number variation is 1-3%, or the total expression level of Z1 and Z16 is 25-30%;
grade II: any one or the combination of at least two of: the expression level of Z1 and Z16 showing loss of imprinting is 20-25%, the expression level of Z1 and Z16 showing copy number variation is 3-7%, or the total expression level of Z1 and Z16 is 30-40%;

grade III: any one or the combination of at least two of: the expression level of Z1 and Z16 showing loss of imprinting is 25-30%, the expression level of Z1 and Z16 showing copy number variation is 7-10%, or the total expression level of Z1 and Z16 is 40-50%;

grade IV: any one or the combination of at least two of: the expression level of Z1 and Z16 showing loss of imprinting is more than 30%, the expression level of Z1 and Z16 showing copy number variation is more than 10%, or the total expression level of Z1 and Z16 is more than 50%;

preferably, for pancreatic tumor, the expression profile of Z1 and Z16 is classified into 5 grades:

grade 0: any one or the combination of at least two of: the expression level of Z1 and Z16 showing loss of imprinting is less than 15%, the expression level of Z1 and Z16 showing copy number variation is less than 2%, or the total expression level of Z1 and Z16 is less than 20%;

grade I: any one or the combination of at least two of: the expression level of Z1 and Z16 showing loss of imprinting is 15-20%, the expression level of Z1 and Z16 showing copy number variation is 2-4%, or the total expression level of Z1 and Z16 is 20-30%;

grade II: any one or the combination of at least two of: the expression level of Z1 and Z16 showing loss of imprinting is 20-25%, the expression level of Z1 and Z16 showing copy number variation is 4-8%, or the total expression level of Z1 and Z16 is 30-40%;

grade III: any one or the combination of at least two of: the expression level of Z1 and Z16 showing loss of imprinting is 25-30%, the expression level of Z1 and Z16 showing copy number variation is 8-12%, or the total expression level of Z1 and Z16 is 40-50%;

grade IV: any one or the combination of at least two of: the expression level of Z1 and Z16 showing loss of imprinting is more than 30%, the expression level of Z1 and Z16 showing copy number variation is more than 12%, or the total expression level of Z1 and Z16 is more than 50%.

4. The method according to any one of claims 1 to 3, wherein for lung tumor, the expression profile of Z1 is classified into 5 grades:

grade 0: any one or the combination of at least two of: the expression level of Z1 showing loss of imprinting is less than 15%, the expression level of Z1 showing copy number variation is less than 2%, or the total expression level of Z1 is less than 30%;

grade I: any one or the combination of at least two of: the expression level of Z1 showing loss of imprinting is 15-20%, the expression level of Z1 showing copy number variation is 2-4%, or the total expression level of Z1 is 30-40%;

grade II: any one or the combination of at least two of: the expression level of Z1 showing loss of imprinting is 20-25%, the expression level of Z1 showing copy number variation is 4-8%, or the total expression level of Z1 is 40-50%;

grade III: any one or the combination of at least two of: the expression level of Z1 showing loss of imprinting is 25-30%, the expression level of Z1 showing copy number variation is 8-12%, or the total expression level of Z1 is 50-60%;

grade IV: any one or the combination of at least two of: the expression level of Z1 showing loss of imprinting is more than 30%, the expression level of Z1 showing copy number variation is more than 12%, or the total expression level of Z1 is more than 60%;

for lung tumor, the expression profile of Z16 is classified into 5 grades:

grade 0: any one or the combination of at least two of: the expression level of Z16 showing loss of imprinting is less than 10%, the expression level of Z16 showing copy number variation is less than 1%, or the total expression level of Z16 is less than 25%;

grade I: any one or the combination of at least two of: the expression level of Z16 showing loss of imprinting is 10-15%, the expression level of Z16 showing copy number variation is 1-2%, or the total expression level of Z16 is 25-30%;

grade II: any one or the combination of at least two of: the expression level of Z16 showing loss of imprinting is 15-20%, the expression level of Z16 showing copy number variation is 2-5%, or the total expression level of Z16 is 30-40%;

grade III: any one or the combination of at least two of: the expression level of Z16 showing loss of imprinting is 20-25%, the expression level of Z16 showing copy number variation is 5-8%, or the total expression level of Z16 is 40-50%;

grade IV: any one or the combination of at least two of: the expression level of Z16 showing loss of imprinting is more than 25%, the expression level of Z16 showing copy number variation is more than 8%, or the total expression level of Z16 is more than 50%;
preferably, for urinary system tumor, the expression profile of Z1 and Z16is classified into 5 grades:

grade 0: any one or the combination of at least two of: the expression level of Z1 and Z16 showing loss of imprinting is less than 17%, the expression level of Z1 and Z16 showing copy number variation is less than 2%;
grade I: any one or the combination of at least two of: the expression level of Z1 and Z16 showing loss of imprinting is 17-20%, the expression level of Z1 and Z16 showing copy number variation is 2-3%;
grade II: any one or the combination of at least two of: the expression level of Z1 and Z16 showing loss of imprinting is 20-25%, the expression level of Z1 and Z16 showing copy number variation is 3-7%;
grade III: any one or the combination of at least two of: the expression level of Z1 and Z16 showing loss of imprinting is 25-30%, the expression level of Z1 and Z16 showing copy number variation is 7-12%;
grade IV: any one or the combination of at least two of: the expression level of Z1 and Z16 showing loss of imprinting is more than 30%, the expression level of Z1 and Z16 showing copy number variation is more than 12%.

5. The method according to any one of claims 1 to 4, further comprising:

(1) obtaining a test sample;
(2) designing a probe specific for the sequence of the imprinted genes;
(3) performing *in situ* hybridization using the probe of step (2) to the test sample; and
(4) analyzing microscopic images and determining the expression status of the imprinted genes;

wherein, the analysis is performed by generating an expression profile of imprinted genes by calculating the expression levels of imprinted genes showing loss of imprinting, the expression levels of imprinted genes showing copy number variation, and the total expression levels of imprinted genes, and grading the expression profiles of imprinted genes to determine the benignity and malignancy of a tumor.

6. The method according to any one of claims 1 to 5, wherein the test sample of step (1) is human tissues and/or cells;

preferably, the test sample includes any one or the combination of at least two of aspiration biopsy cells, biopsy cells, exfoliated cells, blood sample, or brush biopsy sample;
preferably, the aspiration biopsy cells include fine or core needle aspiration biopsy samples, among which anyone or the combination of at least two of the fine or core needle aspiration biopsy samples from thyroid, mammary gland, pancreas, lung, liver, prostate, ovary, lymph node, and parotid are preferable;
preferably, the biopsy cells include biopsy cells from anyone or the combination of at least two of gastroscopy, colonoscopy, cystoscopy, hysteroscopy, or nasopharyngolarygnoscopy;
preferably, the exfoliated cells include exfoliated cells from anyone or the combination of at least two of urine, sputum, faeces, plural effusion, or ascites; and
preferably, the brush biopsy samples include the brushing samples from anyone or the combination of at least two of bronchus, esophagus, oral cavity, or cervical.

7. The method according to any one of claims 1 to 6, wherein the *in situ* hybridization is performed using the RNAscope *in situ* hybridization method; and
preferably, the RNAscope *in situ* hybridization is performed by using singleplex or multiplex color assay kit or singleplex or multiplex fluorescence assay kit, among which, the singleplex red/brown color assay kit or multiplex fluorescence assay kit are preferable.

8. Use of the method according to any one of the claims 1 to 7 in the detection of cancer.

**Patentansprüche**

1. Verfahren zur Krebsdiagnose durch Untersuchen von geprägten Genen in einem Tumor basierend auf Biopsieproben, wobei das Verfahren umfasst:

Erstellen eines Expressionsprofils geprägter Gene durch Berechnen des Expressionsniveaus geprägter Gene, die Verlust der Prägung zeigen, des Expressionsniveaus geprägter Gene, die Kopienzahlvariation zeigen, und des Gesamtexpressionsniveaus geprägter Gene in einem Tumor, um die Expression der geprägten Gene zu bewerten;

wobei die Expressionsniveaus durch die folgenden Formeln berechnet werden:

Gesamtexpressionsniveau eines geprägten Gens = (b+c+d)/(a+b+c+d)x100 %;

Expressionsniveau eines normalen geprägten Gens = b/(b+c+d)x100 %;

Expressionsniveau eines geprägten Gens, das einen Verlust der Prägung zeigt = c/(b+c+d) x 100 % Expressionsniveau eines geprägten Gens, das eine Kopienzahlvariation zeigt = d/(b+c+d) x 100 %;

wobei "a" Zellkerne ohne Markierung im Inneren nach Durchführung einer Hämatoxylin-Färbung an einer Zelle darstellt, was bedeutet, dass das geprägte Gen in den Zellkernen nicht exprimiert wird;

"b" Zellkerne mit einer roten/braunen Markierung im Inneren nach Durchführung einer Hämatoxylin-Färbung an einer Zelle darstellt, was bedeutet, dass das geprägte Gen in den Zellkernen existiert;

"c" Zellkerne mit zwei roten/braunen Markierungen im Inneren nach Durchführung einer Hämatoxylin-Färbung an einer Zelle darstellt, was bedeutet, dass das geprägte Gen die Prägung in den Zellkernen verliert; und

"d" Zellkerne mit mehr als zwei roten/braunen Markierungen im Inneren nach Durchführung einer Hämatoxylin-Färbung an einer Zelle darstellt, was bedeutet, dass das geprägte Gen Kopienzahlvariation in den Zellkernen hat;

wobei das geprägte Gen Z1 oder Z16 ist, Z1 Gnas ist, und Z16 Snrpn/Snurf ist;

wobei das Verfahren ferner Klassifizieren der Expressionsprofile der geprägten Gene in 5 Grade umfasst;

wobei das Expressionsprofil, umfassend das Expressionsniveau von Z1 und Z16, das Verlust der Prägung zeigt, das Expressionsniveau von Z1 und Z16, das Kopienzahlvariation zeigt, und das Gesamtexpressionsniveau von Z1 und Z16, in 5 Grade klassifiziert wird:

Grad 0: irgendeines oder die Kombination von mindestens zwei von:

wobei das Expressionsniveau von Z1 oder Z16, das Verlust der Prägung zeigt, weniger als 15 % ist, das Expressionsniveau von Z1 und Z16, das Kopienzahlvariation zeigt, weniger als 2 % ist oder das Gesamtexpressionsniveau von Z1 und Z16 weniger als 25 % ist;

Grad I: irgendeines oder die Kombination aus mindestens zwei von:

wobei das Expressionsniveau von Z1 oder Z16, das Verlust der Prägung zeigt, 15-20 % ist, das Expressionsniveau von Z1 und Z16, das Kopienzahlvariation zeigt, 2-4 % ist oder das Gesamtexpressionsniveau von Z1 und Z16 25-30 % ist;

Grad II: irgendeines oder die Kombination von mindestens zwei von:

wobei das Expressionsniveau von Z1 oder Z16, das einen Verlust der Prägung zeigt, 20-25 % ist, das Expressionsniveau von Z1 und Z16, das eine Kopienzahlvariation zeigt, 4-8 % ist oder das Gesamtexpressionsniveau von Z1 und Z16 30-40 % ist;

Grad III: irgendeines oder die Kombination von mindestens zwei von:

wobei das Expressionsniveau von Z1 oder Z16, das Verlust der Prägung zeigt, 25-35 % ist, das Expressionsniveau von Z1 und Z16, das Kopienzahlvariation zeigt, 8-12 % ist oder das Gesamtexpressionsniveau von Z1 und Z16 40-50 % ist;

Grad IV: irgendeines oder die Kombination aus mindestens zwei von:

wobei das Expressionsniveau von Z1 oder Z16, das Verlust der Prägung zeigt, mehr als 35 % ist, das Expressionsniveau von Z1 und Z16, das Kopienzahlvariation zeigt, mehr als 12 % ist oder das Gesamtexpressionsniveau von Z1 und Z16 mehr als 50 % ist;

wobei das Verfahren ferner das Klassifizieren der Gutartigkeit oder Bösartigkeit des Tumors umfasst, der als gutartiger Tumor, potenzieller Krebs, Krebs im Frühstadium, Krebs im mittleren Stadium und Krebs im Spätstadium zu bestimmen ist;

wobei vorzugsweise der Tumor als gutartiger Tumor bestimmt wird, wenn die Expressionsniveaus von beiden, Z1 und Z16, die Verlust der Prägung zeigen, niedriger als Grad I sind;

wenn das Expressionsniveau von nur einem von Z1 und Z16, die Verlust der Prägung zeigen, Grad I ist;

wenn das Expressionsniveau von nur einem von Z1 und Z16, die Kopienzahlvariation zeigen, Grad I ist;

wobei vorzugsweise der Tumor als potentieller Krebs bestimmt wird, wenn die Expressionsniveaus von beiden, Z1 und Z16, die Verlust der Prägung zeigen, Grad I sind;
wenn die Expressionsniveaus von beiden, Z1 und Z16, die Kopienzahlvariation zeigen, Grad I sind;
wenn das Expressionsniveau von nur einem von Z1 und Z16,
die Verlust der Prägung zeigen, Grad I ist und das Expressionsniveau von nur einem von Z1 und Z16, die Kopienzahlvariation zeigen, Grad I ist;
wenn das Expressionsniveau von nur einem von Z1 und Z16, das Verlust der Prägung zeigt, Grad II ist;
wenn das Expressionsniveau von nur einem von Z1 und Z16, das Kopienzahlvariation zeigt, Grad II ist;
wobei vorzugsweise der Tumor als Krebs im Frühstadium bestimmt wird, wenn die Expressionsniveaus von beiden, Z1 und Z16, die Verlust der Prägung zeigen, Grad II sind, und/oder die Expressionsniveaus von beiden, Z1 und Z16, die Kopienzahlvariation zeigen, Grad II sind;
wenn das Expressionsniveau von nur einem von Z1 und Z16, die Verlust der Prägung zeigen, Grad II ist und das Expressionsniveau von nur einem von Z1 und Z16, die eKopienzahlvariation zeigen, Grad II ist;
wenn das Expressionsniveau von nur einem von Z1 und Z16, die Verlust der Prägung zeigen, Grad III ist;
wenn das Expressionsniveau von nur einem von Z1 und Z16, die Kopienzahlvariation zeigen, Grad III ist;
wobei vorzugsweise der Tumor als Krebs im mittleren Stadium bestimmt wird, wenn die Expressionsniveaus von beiden, Z1 und Z16, die Verlust der Prägung zeigen, Grad III sind;
wenn die Expressionsniveaus von beiden, Z1 und Z16, die Kopienzahlvariation zeigen, Grad III sind;
wenn das Expressionsniveau von nur einem von Z1 und Z16, die Verlust der Prägung zeigen, Grad III ist und das Expressionsniveau von nur einem von Z1 und Z16, die Kopienzahlvariation zeigen, Grad II ist;
wenn das Expressionsniveau von nur einem von Z1 oder Z16, die Verlust der Prägung zeigen, Grad IV ist;
wenn das Expressionsniveau von nur einem von Z1 oder Z16, die Kopienzahlvariation zeigen, Grad IV ist;
wobei vorzugsweise der Tumor als Krebs im Spätstadium bestimmt wird, wenn die Expressionsniveaus von beiden, Z1 und Z16, die Verlust der Prägung zeigen, Grad IV sind, und/oder die Expressionsniveaus von beiden, Z1 und Z16, die Kopienzahlvariation zeigen, Grad IV sind;
und wobei der Tumor irgendeinen einschließt von: Schilddrüsentumor, Brusttumor, Pankreastumor, Lungentumor, Lebertumor, kolorektaler Tumor, Blasentumor, Prostatatumor, Magentumor, Ösophagustumor, Nasopharynx-Tumor, Mundtumor, Ovarialtumor, Endometriumtumor, Zervikaltumor, Tumor des Harnsystems, Tumor des Zentralnervensystems, Parotistumor, Lymphom und Leukämie.

2. Verfahren nach Anspruch 1, wobei die geprägten Gene auch irgendeines oder die Kombination von mindestens zwei von Z2, Z3, Z4, Z5, Z6, Z7, Z8, Z9, Z10, Z11, Z12, Z13, Z14 und Z15 einschließen;
wobei das geprägte Gen Z2 Igf2 ist, das geprägte Gen Z3 Peg10 ist, das geprägte Gen Z4 Igf2r ist, das geprägte Gen Z5 Mest ist, das geprägte Gen Z6 Plagl1 ist, das geprägte Gen Z7 Cdkn1c ist, das geprägte Gen Z8 Den ist, das geprägte Gen Z9 Dlk1 ist, das geprägte Gen Z10 Gatm ist, das geprägte Gen Z11 Grb10 ist, das geprägte Gen Z12 Peg3 ist, das geprägte Gen Z13 Sgce ist, das geprägte Gen Z14 Slc38a4 ist, das geprägte Gen Z15 Diras3 ist.

3. Verfahren nach einem der Ansprüche 1 bis 2, wobei vorzugsweise für Schilddrüsentumoren das Expressionsprofil von Z1 in 5 Grade klassifiziert wird:

Grad 0: irgendeines oder die Kombination von mindestens zwei von:
wobei das Expressionsniveau von Z1, das einen Verlust der Prägung zeigt, weniger als 15 % ist, das Expressionsniveau von Z1, das eine Kopienzahlvariation zeigt, weniger als 1,5 % ist, oder des Gesamtexpressionsniveau von Z1 weniger als 40 % ist;
Grad I: irgendeines oder die Kombination von mindestens zwei von:
wobei das Expressionsniveau von Z1, das Verlust der Prägung zeigt, 15-20 % ist, das Expressionsniveau von Z1, das Kopienzahlvariation zeigt, 1,54 % ist, oder das Gesamtexpressionsniveau von Z1 40-45 % ist;
Grad II: irgendeines oder die Kombination von mindestens zwei von:
wobei das Expressionsniveau von Z1, das Verlust der Prägung zeigt, 20-30 % ist, das Expressionsniveau von Z1, das Kopienzahlvariation zeigt, 48 % ist, oder das Gesamtexpressionsniveau von Z1 45-60 % ist;
Grad III: irgendeines oder die Kombination aus mindestens zwei von:
wobei das Expressionsniveau von Z1, das Verlust der Prägung zeigt, 30-40 % ist, das Expressionsniveau von Z1, das Kopienzahlvariation zeigt, 8-15 % ist, oder das Gesamtexpressionsniveau von Z1 60-65 % ist;
Grad IV: irgendeines oder die Kombination von mindestens zwei von:

wobei das Expressionsniveau von Z1, das Verlust der Prägung zeigt, mehr als 40 % ist, das Expressionsniveau von Z1, das Kopienzahlvariation zeigt, mehr als 15 % ist, oder das Gesamtexpressionsniveau von Z1 mehr als 65 % ist;

wobei für Schilddrüsentumor das Expressionsprofil von Z16 in 5 Grade klassifiziert wird:

Grad 0: irgendeines oder die Kombination von mindestens zwei von:
wobei das Expressionsniveau von Z16, das Verlust der Prägung zeigt, weniger als 15 % ist, das Expressionsniveau von Z16, das Kopienzahlvariation zeigt, weniger als 1,5 % ist, oder das Gesamtexpressionsniveau von Z16 weniger als 30 % ist;

Grad I: irgendeines oder die Kombinationvon mindestens zwei von:
wobei das Expressionsniveau von Z16, das Verlust der Prägung zeigt, 15-20 % ist, das Expressionsniveau von Z16, das Kopienzahlvariation zeigt, 1,5-4 % ist, oder das Gesamtexpressionsniveau von Z16 30-35 % ist;

Grad II: irgendeines oder die Kombination von mindestens zwei von:
wobei das Expressionsniveau von Z16, das Verlust der Prägung zeigt, 20-30 % ist, das Expressionsniveau von Z16, das Kopienzahlvariation zeigt, 4-8 % ist, oder das Gesamtexpressionsniveau von Z16 35-50 % ist;

Grad III: irgendeines oder die Kombination von mindestens zwei von:
wobei das Expressionsniveau von Z16, das Verlust der Prägung zeigt, 30-40 % ist, das Expressionsniveau von Z16, das Kopienzahlvariation zeigt, 8-15 % ist, oder das Gesamtexpressionsniveau von Z16 50-55 % ist;

Grad IV: irgendeines oder die Kombination von mindestens zwei von:

wobei das Expressionsniveau von Z16, das Verlust der Prägung zeigt, mehr als 40 % ist, das Expressionsniveau von Z16, das Kopienzahlvariation zeigt, mehr als 15 % ist, oder das Gesamtexpressionsniveau von Z16 mehr als 55 % ist;

wobei vorzugsweise für Brusttumor das Expressionsprofil von Z1 und Z16 in 5 Grade klassifiziert wird:

Grad 0: irgendeines oder die Kombination von mindestens zwei von:
wobei das Expressionsniveau von Z1 und Z16, das Verlust der Prägung zeigt, weniger als 15 % ist, das Expressionsniveau von Z1 und Z16, das Kopienzahlvariation zeigt, weniger als 1 % ist, oder das Gesamtexpressionsniveau von Z1 und Z16 weniger als 25 % ist;

Grad I: irgendeines oder die Kombination von mindestens zwei von:
wobei das Expressionsniveau von Z1 und Z16, das Verlust der Prägung zeigt, 15-20 % ist, das Expressionsniveau von Z1 und Z16, das Kopienzahlvariation zeigt, 1-3 % ist, oder das Gesamtexpressionsniveau von Z1 und Z16 25-30 % ist;

Grad II: irgendeines oder die Kombination von mindestens zwei von:
wobei das Expressionsniveau von Z1 und Z16, das Verlust der Prägung zeigt, 20-25 % ist, das Expressionsniveau von Z1 und Z16, das Kopienzahlvariation zeigt, 3-7 % ist, oder das Gesamtexpressionsniveau von Z1 und Z16 30-40 % ist;

Grad III: irgendeines oder die Kombination von mindestens zwei von:
wobei das Expressionsniveau von Z1 und Z16, das Verlust der Prägung zeigt, 25-30 % ist, das Expressionsniveau von Z1 und Z16, das Kopienzahlvariation zeigt, 7-10 % ist, oder das Gesamtexpressionsniveau von Z1 und Z16 40-50 % ist;

Grad IV: irgendeines oder die Kombination von mindestens zwei von:

wobei das Expressionsniveau von Z1 und Z16, das Verlust der Prägung zeigt, mehr als 30 % ist, das Expressionsniveau von Z1 und Z16, das Kopienzahlvariation zeigt, mehr als 10 % ist, oder das Gesamtexpressionsniveau von Z1 und Z16 mehr als 50 % ist;

wobei vorzugsweise für Pankreastumor das Expressionsprofil von Z1 und Z16 in 5 Grade klassifiziert wird:

Grad 0: irgendeines oder die Kombination von mindestens zwei von:
wobei das Expressionsniveau von Z1 und Z16, das Verlust der Prägung zeigt, weniger als 15 % ist, das Expressionsniveau von Z1 und Z16, das Kopienzahlvariation zeigt, weniger als 2 % ist, oder das Gesamtexpressionsniveau von Z1 und Z16 weniger als 20 % ist;

Grad I: irgendeines oder die Kombination von mindestens zwei von:
wobei das Expressionsniveau von Z1 und Z16, das Verlust der Prägung zeigt, 15-20 % ist, das Expressionsniveau von Z1 und Z16, das Kopienzahlvariation zeigt, 2-4 % ist, oder das Gesamtexpressionsniveau von Z1 und Z16 20-30 % ist;
Grad II: irgendeines oder die Kombination von mindestens zwei von:
wobei das Expressionsniveau von Z1 und Z16, das Verlust der Prägung zeigt, 20-25 % ist, das Expressionsniveau von Z1 und Z16, das Kopienzahlvariation zeigt, 4-8 % ist, oder das Gesamtexpressionsniveau von Z1 und Z16 30-40 % ist;
Grad III: irgendeines oder die Kombination von mindestens zwei von:
wobei das Expressionsniveau von Z1 und Z16, das Verlust der Prägung zeigt, 25-30 % ist, das Expressionsniveau von Z1 und Z16, das Kopienzahlvariation zeigt, 8-12 % ist, oder das Gesamtexpressionsniveau von Z1 und Z16 40-50 % ist;
Grad IV: irgendeines oder die Kombination von mindestens zwei von:
wobei das Expressionsniveau von Z1 und Z16, das Verlust der Prägung zeigt, mehr als 30 % ist, das Expressionsniveau von Z1 und Z16, das Kopienzahlvariation zeigt, mehr als 12 % ist, oder das Gesamtexpressionsniveau von Z1 und Z16 mehr als 50 % ist.

4. Verfahren nach einem der Ansprüche 1 bis 3, wobei für Lungentumor das Expressionsprofil von Z1 in 5 Grade klassifiziert wird:

Grad 0: irgendeines oder die Kombination von mindestens zwei von:
wobei das Expressionsniveau von Z1, das Verlust der Prägung zeigt, weniger als 15 % ist, das Expressionsniveau von Z1, das Kopienzahlvariation zeigt, weniger als 2 % ist, oder das Gesamtexpressionsniveau von Z1 weniger als 30 % ist;
Grad I: irgendeines oder die Kombination von mindestens zwei von:
wobei das Expressionsniveau von Z1, das Verlust der Prägung zeigt, 15-20 % ist, das Expressionsniveau von Z1, das Kopienzahlvariation zeigt, 2-4 % ist, oder das Gesamtexpressionsniveau von Z1 30-40 % ist;
Grad II: irgendeines oder die Kombination von mindestens zwei von:
wobei das Expressionsniveau von Z1, das Verlust der Prägung zeigt, 20-25 % ist, das Expressionsniveau von Z1, das Kopienzahlvariation zeigt, 4-8 % ist, oder das Gesamtexpressionsniveau von Z1 40-50 % ist;
Grad III: irgendeines oder die Kombination von mindestens zwei von:
wobei das Expressionsniveau von Z1, das Verlust der Prägung zeigt, 25-30 % ist, das Expressionsniveau von Z1, das Kopienzahlvariation zeigt, 8-12 % ist, oder das Gesamtexpressionsniveau von Z1 50-60 % ist;
Grad IV: irgendeines oder die Kombination von mindestens zwei von:

wobei das Expressionsniveau von Z1, das Verlust der Prägung zeigt, mehr als 30 % ist, das Expressionsniveau von Z1, das Kopienzahlvariation zeigt, mehr als 12 % ist, oder das Gesamtexpressionsniveau von Z1 mehr als 60 % ist;
wobei für Lungenturmor das Expressionsprofil von Z16 in 5 Grade klassifiziert wird:

Grad 0: irgendeines oder die Kombination von mindestens zwei von:
wobei das Expressionsniveau von Z16, das Verlust der Prägung zeigt, weniger als 10 % ist, das Expressionsniveau von Z16, das Kopienzahlvariation zeigt, weniger als 1 % ist, oder das Gesamtexpressionsniveau von Z16 weniger als 25 % ist;
Grad I: irgendeines oder die Kombination von mindestens zwei von:
wobei das Expressionsniveau von Z16, das Verlust der Prägung zeigt, 10-15 % ist, das Expressionsniveau von Z16, das Kopienzahlvariation zeigt, 1-2 % ist, oder das Gesamtexpressionsniveau von Z16 25-30 % ist;
Grad II: irgendeines oder die Kombination von mindestens zwei von:
wobei das Expressionsniveau von Z16, das Verlust der Prägung zeigt, 15-20 % ist, das Expressionsniveau von Z16, das Kopienzahlvariation zeigt, 2-5 % ist, oder das Gesamtexpressionsniveau von Z16 30-40 % ist;
Grad III: irgendeines oder die Kombination von mindestens zwei von
wobei das Expressionsniveau von Z16, das Verlust der Prägung zeigt, 20-25 % ist, das Expressionsniveau von Z16, das Kopienzahlvariation zeigt, 5-8 % ist, oder das Gesamtexpressionsniveau von Z16 40-50 % ist;
Grad IV: irgendeines oder die Kombination von mindestens zwei von:

wobei das Expressionsniveau von Z16, das Verlust der Prägung zeigt, mehr als 25 % ist, das Expressionsniveau von Z16, das Kopienzahlvariation zeigt, mehr als 8 % ist, oder das Gesamtexpressionsniveau von Z16 mehr als 50 % ist;
wobei vorzugsweise für Tumor des Harnsystems das Expressionsprofil von Z1 und Z16 in 5 Grade klassifiziert wird:

Grad 0: irgendeines oder die Kombination von mindestens zwei von:
wobei das Expressionsniveau von Z1 und Z16, das Verlust der Prägung zeigt, weniger als 17 % ist, das Expressionsniveau von Z1 und Z16, das Kopienzahlvariation zeigt, weniger als 2 % ist;
Grad I: irgendeines oder die Kombination von mindestens zwei von:
wobei das Expressionsniveau von Z1 und Z16, das Verlust der Prägung zeigt, 17-20 % ist, das Expressionsniveau von Z1 und Z16, das Kopienzahlvariation zeigt, 2-3 % ist;
Grad II: irgendeines oder die Kombination von mindestens zwei von:
wobei das Expressionsniveau von Z1 und Z16, das Verlust der Prägung zeigt, 20-25 % ist, das Expressionsniveau von Z1 und Z16, das Kopienzahlvariation zeigt, 3-7 % ist;
Grad III: irgendeines oder die Kombination von mindestens zwei von:
wobei das Expressionsniveau von Z1 und Z16, das Verlust der Prägung zeigt, 25-30 % ist, das Expressionsniveau von Z1 und Z16, das Kopienzahlvariation zeigt, 7-12 % ist;
Grad IV: irgendeines oder die Kombination von mindestens zwei von:
wobei das Expressionsniveau von Z1 und Z16, das Verlust der Prägung zeigt, mehr als 30 % ist, das Expressionsniveau von Z1 und Z16, das eine Kopienzahlvariation zeigt, mehr als 12 % ist.

5. Verfahren nach einem der Ansprüche 1 bis 4, ferner umfassend:

(1) Erhalten einer Testprobe;
(2) Entwerfen einer Sonde, spezifisch für die Sequenz der geprägten Gene;
(3) Durchführen einer In-situ-Hybridisierung unter Verwendung der Sonde aus Schritt (2) an der Testprobe; und
(4) Analysieren von Mikroskopbildern und Bestimmen des Expressionsstatus der geprägten Gene;

wobei die Analyse durchgeführt wird, durch Erstellen eines Expressionsprofils von geprägten Genen, durch Berechnen der Expressionsniveaus von geprägten Genen, die Verlust der Prägung zeigen, der Expressionsniveaus von geprägten Genen, die Kopienzahlvariation zeigen, und der Gesamtexpressionsniveaus von geprägten Genen, und Einstufen der Expressionsprofile der geprägten Gene, um die Gutartigkeit und Bösartigkeit eines Tumors zu bestimmen.

6. Verfahren nach einem der Ansprüche 1 bis 5, wobei die Testprobe in Schritt (1) menschliche Gewebe und/oder Zellen sind;

wobei vorzugsweise die Testprobe irgendeine oder die Kombination von mindestens zwei von Aspirationsbiopsiezellen, Biopsiezellen, exfolierte Zellen, Blutproben oder Bürstenbiopsieproben einschließt;
wobei vorzugsweise die Aspirationsbiopsiezellen Fein- oder Kernnadelaspirationsbiopsieproben einschließen, von denen irgendeine oder die Kombination von mindestens zwei der Fein- oder Kernnadelaspirationsbiopsieproben aus Schilddrüse, Brustdrüse, Pankreas, Lunge, Leber, Prostata, Eierstock, Lymphknoten und Ohrspeicheldrüse bevorzugt sind;
wobei vorzugsweise die Biopsiezellen Biopsiezellen von irgendeiner oder der Kombination von mindestens zwei von Gastroskopie, Koloskopie, Zystoskopie, Hysteroskopie oder Nasopharyngolaryngoskopie einschließen;
wobei vorzugsweise die exfolierten Zellen exfolierte Zellen von irgendeiner oder der Kombination von mindestens zwei von Urin, Sputum, Fäkalien, Pleuraerguss oder Aszites einschließen; und
wobei vorzugsweise die Bürstenbiopsieproben die Bürstenproben von irgendeiner oder der Kombination von mindestens zwei von Bronchus, Speiseröhre, Mundhöhle oder Gebärmutterhals einschließen.

7. Verfahren nach einem der Ansprüche 1 bis 6, wobei die In-situ-Hybridisierung unter Verwendung des RNAscope-In-situ-Hybridisierungsverfahrens durchgeführt wird; und
wobei vorzugsweise die RNAscope-in-situ-Hybridisierung unter Verwendung eines Singleplex- oder Multiplex-Farbtestkits oder Singleplexoder Multiplex-Fluoreszenztestkits durchgeführt wird, von denen das Singleplex-Rot/-Braun-Farbtestkit oder das Multiplex-Fluoreszenztestkit bevorzugt sind.

8. Verwendung des Verfahrens nach einem der Ansprüche 1 bis 7 zum Nachweis von Krebs.

**Revendications**

1. Méthode pour diagnostiquer un cancer par dosage de gènes soumis à empreinte dans une tumeur sur la base d'échantillons de biopsie, la méthode comprenant la génération d'un profil d'expression de gènes soumis à empreinte par calcul du niveau d'expression de gènes soumis à empreinte présentant une perte d'empreinte, du niveau d'expression de gènes soumis à empreinte présentant une variation du nombre de copies, et du niveau d'expression total de gènes soumis à empreinte dans une tumeur, pour un classement de l'expression des gènes soumis à empreinte ;

   dans laquelle les niveaux d'expression sont calculés par les formules suivantes :

   niveau d'expression total d'un gène soumis à empreinte = (b+c+d)/(a+b+c+d) × 100 % ;

   niveau d'expression d'un gène soumis à empreinte normal = b/(b+c+d) × 100 % ;

   niveau d'expression d'un gène soumis à empreinte présentant une perte d'empreinte = c/(b+c+d) × 100 % ;

   niveau d'expression d'un gène imprimé présentant une variation du nombre de copies = d/(b+c+d) × 100 % ;

   dans laquelle "a" représente les noyaux cellulaires sans marquage interne après qu'une coloration à l'hématoxyline a été effectuée sur une cellule, ce qui signifie que le gène soumis à empreinte n'est pas exprimé dans les noyaux cellulaires ;
   "b" représente les noyaux cellulaires avec un marquage rouge/marron interne après qu'une coloration à l'hématoxyline a été effectuée sur une cellule, ce qui signifie que le gène soumis à empreinte existe dans les noyaux cellulaires ;
   "c" représente les noyaux cellulaires avec deux marquages rouge/marron internes après qu'une coloration à l'hématoxyline a été effectuée sur une cellule, ce qui signifie que le gène soumis à empreinte présente une perte d'empreinte dans les noyaux cellulaires ; et
   "d" représente les noyaux cellulaires avec plus de deux marquages rouge/marron internes après qu'une coloration à l'hématoxyline a été effectuée sur une cellule, ce qui signifie que le gène soumis à empreinte présente une variation du nombre de copies dans les noyaux cellulaires ;
   dans laquelle le gène imprimé est Z1 ou Z16, Z1 est Gnas, et Z16 est Snrpn/Snurf ;
   la méthode comprenant en outre la classification des profils d'expression de gènes soumis à empreinte en 5 grades ;
   le profil d'expression comprenant le niveau d'expression de Z1 et Z16 présentant une perte d'empreinte, le niveau d'expression de Z1 et Z16 présentant une variation du nombre de copies, et le niveau d'expression total de Z1 et Z16 sont classés en 5 grades :

   grade 0 : l'un quelconque ou la combinaison d'au moins deux parmi : le niveau d'expression de Z1 ou Z16 présentant une perte d'empreinte est inférieur à 15 %, le niveau d'expression de Z1 et Z16 présentant une variation du nombre de copies est inférieur à 2 %, et le niveau d'expression total de Z1 et Z16 est inférieur à 25 % ;
   grade I : l'un quelconque ou la combinaison d'au moins deux parmi : le niveau d'expression de Z1 ou Z16 présentant une perte d'empreinte est de 15 à 20 %, le niveau d'expression de Z1 et Z16 présentant une variation du nombre de copies est de 2 à 4 %, et le niveau d'expression total de Z1 et Z16 est de 25 à 30 % ;
   grade II : l'un quelconque ou la combinaison d'au moins deux parmi : le niveau d'expression de Z1 ou Z16 présentant une perte d'empreinte est de 20 à 25 %, le niveau d'expression de Z1 et Z16 présentant une variation du nombre de copies est de 4 à 8 %, et le niveau d'expression total de Z1 et Z16 est de 30 à 40 % ;
   grade III : l'un quelconque ou la combinaison d'au moins deux parmi : le niveau d'expression de Z1 ou Z16 présentant une perte d'empreinte est de 25 à 35 %, le niveau d'expression de Z1 et Z16 présentant une variation du nombre de copies est de 8 à 12 %, et le niveau d'expression total de Z1 et Z16 est de 40 à 50 % ;
   grade IV : l'un quelconque ou la combinaison d'au moins deux parmi : le niveau d'expression de Z1 ou Z16 présentant une perte d'empreinte est supérieur à 35 %, le niveau d'expression de Z1 et Z16 présentant une variation du nombre de copies est supérieur à 12 %, et le niveau d'expression total de Z1 et Z16 est supérieur à 50 % ;
   la méthode comprenant en outre la classification de la bénignité ou de la malignité de la tumeur qui doit être déterminée comme étant une tumeur bénigne, un potentiel cancéreux, un cancer à un stade précoce, un cancer à un stade intermédiaire, ou un cancer à un stade avancé ;

de préférence, la tumeur est déterminée comme étant une tumeur bénigne si les niveaux d'expression tant de Z1 que de Z16 présentant une perte d'empreinte sont inférieurs au grade I ; si le niveau d'expression d'un seul parmi Z1 et Z16 présentant une perte d'empreinte est de grade I ; si le niveau d'expression d'un seul parmi Z1 et Z16 présentant une variation du nombre de copies est de grade I ;

de préférence, la tumeur est déterminée comme étant un potentiel cancéreux si les niveaux d'expression tant de Z1 que de Z16 présentant une perte d'empreinte sont de grade I ; si les niveaux d'expression tant de Z1 que de Z16 présentant une variation du nombre de copies sont de grade I ; si le niveau d'expression d'un seul parmi Z1 et Z16 présentant une perte d'empreinte est de grade I et le niveau d'expression d'un seul parmi Z1 et Z16 présentant une variation du nombre de copies est de grade I ; si le niveau d'expression d'un seul parmi Z1 et Z16 présentant une perte d'empreinte est de grade II ; si le niveau d'expression d'un seul parmi Z1 et Z16 présentant une variation du nombre de copies est de grade II ;

de préférence, la tumeur est déterminée comme étant un cancer à un stade précoce si les niveaux d'expression tant de Z1 que de Z16 présentant une perte d'empreinte sont de grade II, et/ou les niveaux d'expression tant de Z1 que de Z16 présentant une variation du nombre de copies sont de grade II ; si le niveau d'expression d'un seul parmi Z1 et Z16 présentant une perte d'empreinte est de grade II et le niveau d'expression d'un seul parmi Z1 et Z16 présentant une variation du nombre de copies est de grade II ; si l'expression d'un seul parmi Z1 et Z16 présentant une perte d'empreinte est de grade III ; si le niveau d'expression d'un seul parmi Z1 et Z16 présentant une variation du nombre de copies est de grade III ;

de préférence, la tumeur est déterminée comme étant un cancer à un stade intermédiaire si les niveaux d'expression tant de Z1 que de Z16 présentant une perte d'empreinte sont de grade III ; si les niveaux d'expression tant de Z1 que de Z16 présentant une variation du nombre de copies sont de grade III ; si le niveau d'expression d'un seul parmi Z1 et Z16 présentant une perte d'empreinte est de grade III et le niveau d'expression d'un seul parmi Z1 et Z16 présentant une variation du nombre de copies est de grade III ; si le niveau d'expression d'un seul parmi Z1 et Z16 présentant une perte d'empreinte est de grade IV ; si le niveau d'expression d'un seul parmi Z1 et Z16 présentant une variation du nombre de copies est de grade IV ;

de préférence, la tumeur est déterminée comme étant un cancer à un stade avancé si les niveaux d'expression tant de Z1 que de Z16 présentant une perte d'empreinte sont de grade IV, et/ou les niveaux d'expression tant de Z1 que de Z16 présentant une variation du nombre de copies sont de grade IV ;

et dans laquelle la tumeur englobe l'une quelconque parmi une tumeur thyroïdienne, une tumeur mammaire, une tumeur pancréatique, une tumeur pulmonaire, une tumeur hépatique, une tumeur colorectale, une tumeur de la vessie, une tumeur prostatique, une tumeur gastrique, une tumeur œsophagienne, une tumeur nasopharyngée, une tumeur orale, une tumeur ovarienne, une tumeur endométriale, une tumeur du col, une tumeur du système urinaire, une tumeur du système nerveux central, une tumeur parotidienne, un lymphome, et une leucémie.

2. Méthode selon la revendication 1, dans laquelle les gènes soumis à empreinte englobent aussi l'un quelconque ou une combinaison d'au moins deux parmi Z2, Z3, Z4, Z5, Z6, Z7, Z8, Z9, Z10, Z11, Z12, Z13, Z14 et Z15 ; dans laquelle le gène soumis à empreinte Z2 est Igf2, le gène soumis à empreinte Z3 est Peg10, le gène soumis à empreinte Z4 est Igf2r, le gène soumis à empreinte Z5 est Mest, le gène soumis à empreinte Z6 est Plagl1, le gène soumis à empreinte Z7 est Cdkn1c, le gène soumis à empreinte Z8 est Dcn, le gène soumis à empreinte Z9 est Dlk1, le gène soumis à empreinte Z10 est Gatm, le gène soumis à empreinte Z11 est Grb10, le gène soumis à empreinte Z12 est Peg3, le gène soumis à empreinte Z13 est Sgce, le gène soumis à empreinte Z14 est Slc38a4, le gène soumis à empreinte Z15 est Diras3.

3. Méthode selon l'une quelconque des revendications 1 et 2, dans laquelle, de préférence, pour une tumeur thyroïdienne, le profil d'expression de Z1 est classé en 5 grades :

grade 0 : l'un quelconque ou la combinaison d'au moins deux parmi : le niveau d'expression de Z1 présentant une perte d'empreinte est inférieur à 15 %, le niveau d'expression de Z1 présentant une variation du nombre de copies est inférieur à 1,5 %, et le niveau d'expression total de Z1 est inférieur à 40 % ;

grade I : l'un quelconque ou la combinaison d'au moins deux parmi : le niveau d'expression de Z1 présentant une perte d'empreinte est de 15 à 20 %, le niveau d'expression de Z1 présentant une variation du nombre de copies est de 1,5 à 4 %, et le niveau d'expression total de Z1 est de 40 à 45 % ;

grade II : l'un quelconque ou la combinaison d'au moins deux parmi : le niveau d'expression de Z1 présentant une perte d'empreinte est de 20 à 30 %, le niveau d'expression de Z1 présentant une variation du nombre de copies est de 4 à 8 %, et le niveau d'expression total de Z1 est de 45 à 60 % ;

grade III : l'un quelconque ou la combinaison d'au moins deux parmi : le niveau d'expression de Z1 présentant une perte d'empreinte est de 30 à 40 %, le niveau d'expression de Z1 présentant une variation du nombre de copies est de 8 à 15 %, et le niveau d'expression total de Z1 est de 60 à 65 % ;

grade IV : l'un quelconque ou la combinaison d'au moins deux parmi : le niveau d'expression de Z1 présentant une perte d'empreinte est supérieur à 40 %, le niveau d'expression de Z1 présentant une variation du nombre de copies est supérieur à 15 %, et le niveau d'expression total de Z1 est supérieur à 65 % ;

pour une tumeur thyroïdienne, le profil d'expression de Z16 est classé en 5 grades :

grade 0 : l'un quelconque ou la combinaison d'au moins deux parmi : le niveau d'expression de Z16 présentant une perte d'empreinte est inférieur à 15 %, le niveau d'expression de Z16 présentant une variation du nombre de copies est inférieur à 1,5 %, et le niveau d'expression total de Z16 est inférieur à 30 % ;

grade I : l'un quelconque ou la combinaison d'au moins deux parmi : le niveau d'expression de Z16 présentant une perte d'empreinte est de 15 à 20 %, le niveau d'expression de Z16 présentant une variation du nombre de copies est de 1,5 à 4 %, et le niveau d'expression total de Z16 est de 30 à 35 % ;

grade II : l'un quelconque ou la combinaison d'au moins deux parmi : le niveau d'expression de Z16 présentant une perte d'empreinte est de 20 à 30 %, le niveau d'expression de Z16 présentant une variation du nombre de copies est de 4 à 8 %, et le niveau d'expression total de Z16 est de 35 à 50 % ;

grade III : l'un quelconque ou la combinaison d'au moins deux parmi : le niveau d'expression de Z16 présentant une perte d'empreinte est de 30 à 40 %, le niveau d'expression de Z16 présentant une variation du nombre de copies est de 8 à 15 %, et le niveau d'expression total de Z16 est de 50 à 55 % ;

grade IV : l'un quelconque ou la combinaison d'au moins deux parmi : le niveau d'expression de Z16 présentant une perte d'empreinte est supérieur à 40 %, le niveau d'expression de Z16 présentant une variation du nombre de copies est supérieur à 15 %, et le niveau d'expression total de Z16 est supérieur à 55 % ;

de préférence, pour une tumeur mammaire , le profil d'expression de Z1 et Z16 est classé en 5 grades :

grade 0 : l'un quelconque ou la combinaison d'au moins deux parmi : le niveau d'expression de Z1 et Z16 présentant une perte d'empreinte est inférieur à 15 %, le niveau d'expression de Z1 et Z16 présentant une variation du nombre de copies est inférieur à 1 %, et le niveau d'expression total de Z1 et Z16 est inférieur à 25 % ;

grade I : l'un quelconque ou la combinaison d'au moins deux parmi : le niveau d'expression de Z1 et Z16 présentant une perte d'empreinte est de 15 à 20 %, le niveau d'expression de Z1 et Z16 présentant une variation du nombre de copies est de 1 à 3 %, et le niveau d'expression total de Z1 et Z16 est de 25 à 30 % ;

grade II : l'un quelconque ou la combinaison d'au moins deux parmi : le niveau d'expression de Z1 et Z16 présentant une perte d'empreinte est de 20 à 25 %, le niveau d'expression de Z1 et Z16 présentant une variation du nombre de copies est de 3 à 7 %, et le niveau d'expression total de Z1 et Z16 est de 30 à 40 % ;

grade III : l'un quelconque ou la combinaison d'au moins deux parmi : le niveau d'expression de Z1 et Z16 présentant une perte d'empreinte est de 25 à 30 %, le niveau d'expression de Z1 et Z16 présentant une variation du nombre de copies est de 7 à 10 %, et le niveau d'expression total de Z1 et Z16 est de 40 à 50 % ;

grade IV : l'un quelconque ou la combinaison d'au moins deux parmi : le niveau d'expression de Z1 et Z16 présentant une perte d'empreinte est supérieur à 30 %, le niveau d'expression de Z1 et Z16 présentant une variation du nombre de copies est supérieur à 10 %, et le niveau d'expression total de Z1 et Z16 est supérieur à 50 % ;

de préférence, pour une tumeur pancréatique, le profil d'expression de Z1 et Z16 est classé en 5 grades :

grade 0 : l'un quelconque ou la combinaison d'au moins deux parmi : le niveau d'expression de Z1 et Z16 présentant une perte d'empreinte est inférieur à 15 %, le niveau d'expression de Z1 et Z16 présentant une variation du nombre de copies est inférieur à 2 %, et le niveau d'expression total de Z1 et Z16 est inférieur à 20 % ;

grade I : l'un quelconque ou la combinaison d'au moins deux parmi : le niveau d'expression de Z1 et Z16 présentant une perte d'empreinte est de 15 à 20 %, le niveau d'expression de Z1 et Z16 présentant une variation du nombre de copies est de 2 à 4 %, et le niveau d'expression total de Z1 et Z16 est de 20 à 30 % ;

grade II : l'un quelconque ou la combinaison d'au moins deux parmi : le niveau d'expression de Z1 et Z16 présentant une perte d'empreinte est de 20 à 25 %, le niveau d'expression de Z1 et Z16 présentant une variation du nombre de copies est de 4 à 8 %, et le niveau d'expression total de Z1 et Z16 est de 30 à 40 % ;

grade III : l'un quelconque ou la combinaison d'au moins deux parmi : le niveau d'expression de Z1 et Z16 présentant une perte d'empreinte est de 25 à 30 %, le niveau d'expression de Z1 et Z16 présentant une variation du nombre de copies est de 8 à 12 %, et le niveau d'expression total de Z1 et Z16 est de 40 à 50 % ;

grade IV : l'un quelconque ou la combinaison d'au moins deux parmi : le niveau d'expression de Z1 et Z16 présentant une perte d'empreinte est supérieur à 30 %, le niveau d'expression de Z1 et Z16 présentant une variation du nombre de copies est supérieur à 12 %, et le niveau d'expression total de Z1 et Z16 est supérieur à 50 %.

4. Méthode selon l'une quelconque des revendications 1 à 3, dans laquelle, pour une tumeur pulmonaire, le profil d'expression de Z1 est classé en 5 grades :

grade 0 : l'un quelconque ou la combinaison d'au moins deux parmi : le niveau d'expression de Z1 présentant une perte d'empreinte est inférieur à 15 %, le niveau d'expression de Z1 présentant une variation du nombre de copies est inférieur à 2 %, et le niveau d'expression total de Z1 est inférieur à 30 % ;

grade I : l'un quelconque ou la combinaison d'au moins deux parmi : le niveau d'expression de Z1 présentant une perte d'empreinte est de 15 à 20 %, le niveau d'expression de Z1 présentant une variation du nombre de copies est de 2 à 4 %, et le niveau d'expression total de Z1 est de 30 à 40 % ;

grade II : l'un quelconque ou la combinaison d'au moins deux parmi : le niveau d'expression de Z1 présentant une perte d'empreinte est de 20 à 25 %, le niveau d'expression de Z1 présentant une variation du nombre de copies est de 4 à 8 %, et le niveau d'expression total de Z1 est de 40 à 50 % ;

grade III : l'un quelconque ou la combinaison d'au moins deux parmi : le niveau d'expression de Z1 présentant une perte d'empreinte est de 25 à 30 %, le niveau d'expression de Z1 présentant une variation du nombre de copies est de 8 à 12 %, et le niveau d'expression total de Z1 est de 50 à 60 % ;

grade IV : l'un quelconque ou la combinaison d'au moins deux parmi : le niveau d'expression de Z1 présentant une perte d'empreinte est supérieur à 30 %, le niveau d'expression de Z1 présentant une variation du nombre de copies est supérieur à 12 %, et le niveau d'expression total de Z1 est supérieur à 60 % ;

pour une tumeur pulmonaire, le profil d'expression de Z16 est classé en 5 grades :

grade 0 : l'un quelconque ou la combinaison d'au moins deux parmi : le niveau d'expression de Z16 présentant une perte d'empreinte est inférieur à 10 %, le niveau d'expression de Z16 présentant une variation du nombre de copies est inférieur à 1 %, et le niveau d'expression total de Z16 est inférieur à 25 % ;

grade I : l'un quelconque ou la combinaison d'au moins deux parmi : le niveau d'expression de Z16 présentant une perte d'empreinte est de 10 à 15 %, le niveau d'expression de Z16 présentant une variation du nombre de copies est de 1 à 2 %, et le niveau d'expression total de Z16 est de 25 à 30 % ;

grade II : l'un quelconque ou la combinaison d'au moins deux parmi : le niveau d'expression de Z16 présentant une perte d'empreinte est de 15 à 20 %, le niveau d'expression de Z16 présentant une variation du nombre de copies est de 2 à 5 %, et le niveau d'expression total de Z1 est de 30 à 40 % ;

grade III : l'un quelconque ou la combinaison d'au moins deux parmi : le niveau d'expression de Z16 présentant une perte d'empreinte est de 20 à 25 %, le niveau d'expression de Z16 présentant une variation du nombre de copies est de 5 à 8 %, et le niveau d'expression total de Z16 est de 40 à 50 % ;

grade IV : l'un quelconque ou la combinaison d'au moins deux parmi : le niveau d'expression de Z16 présentant une perte d'empreinte est supérieur à 25 %, le niveau d'expression de Z16 présentant une variation du nombre de copies est supérieur à 8 %, et le niveau d'expression total de Z16 est supérieur à 50 % ;

de préférence, pour une tumeur du système urinaire, le profil d'expression de Z1 et Z16 est classé en 5 grades :

grade 0 : l'un quelconque ou la combinaison d'au moins deux parmi : le niveau d'expression de Z1 et Z16 présentant une perte d'empreinte est inférieur à 17 %, le niveau d'expression de Z1 et Z16 présentant une variation du nombre de copies est inférieur à 2 % ;

grade I : l'un quelconque ou la combinaison d'au moins deux parmi : le niveau d'expression de Z1 et Z16 présentant une perte d'empreinte est de 17 à 20 %, le niveau d'expression de Z1 et Z16 présentant une variation du nombre de copies est de 2 à 3 % ;

grade II : l'un quelconque ou la combinaison d'au moins deux parmi : le niveau d'expression de Z1 et Z16 présentant une perte d'empreinte est de 20 à 25 %, le niveau d'expression de Z1 et Z16 présentant une variation du nombre de copies est de 3 à 7 % ;

grade III : l'un quelconque ou la combinaison d'au moins deux parmi : le niveau d'expression de Z1 et Z16 présentant une perte d'empreinte est de 25 à 30 %, le niveau d'expression de Z1 et Z16 présentant une variation du nombre de copies est de 7 à 12 % ;

grade IV : l'un quelconque ou la combinaison d'au moins deux parmi : le niveau d'expression de Z1 et Z16 présentant une perte d'empreinte est supérieur à 30 %, le niveau d'expression de Z1 et Z16 présentant une variation du nombre de copies est supérieur à 12 %.

5. Méthode selon l'une quelconque des revendications 1 à 4, comprenant en outre :

(1) l'obtention d'un échantillon de test ;
(2) la conception d'une sonde spécifique de la séquence des gènes soumis à empreinte ;
(3) la mise en œuvre d'une hybridation *in situ* utilisant la sonde de l'étape (2) sur l'échantillon de test ; et
(4) l'analyse d'images au microscope et la détermination du statut d'expression des gènes soumis à empreinte ;

dans laquelle l'analyse est effectuée par génération d'un profil d'expression de gènes soumis à empreinte par calcul des niveaux d'expression de gènes soumis à empreinte présentant une perte d'empreinte, des niveaux d'expression de gènes soumis à empreinte présentant une variation du nombre de copies, et des niveaux d'expression totaux des gènes soumis à empreinte, et classification des profils d'expression des gènes soumis à empreinte pour que soient déterminées la bénignité et la malignité d'une tumeur.

6. Méthode selon l'une quelconque des revendications 1 à 5, dans laquelle l'échantillon de test de l'étape (1) consiste en cellules et/ou tissus humains ;

de préférence l'échantillon de test comprend l'un quelconque ou la combinaison d'au moins deux parmi des cellules issues d'une cytoponction, des cellules issues d'une biopsie, des cellules exfoliées, un échantillon de sang, ou un échantillon de brossage cytologique ;

de préférence les cellules issues d'une cytoponction comprennent les échantillons de cytoponction à l'aiguille fine ou à l'aiguille à biopsie, parmi lesquels l'un quelconque ou la combinaison d'au moins deux parmi les échantillons de cytoponction à l'aiguille fine et à l'aiguille à biopsie provenant de thyroïde, de glande mammaire, de pancréas, de poumon, de foie, de prostate, d'ovaire, de ganglion lymphatique et de parotide sont préférables ;

de préférence, les cellules issues d'une biopsie comprennent les cellules de biopsie provenant de l'une quelconque ou de la combinaison d'au moins deux parmi une gastroscopie, une coloscopie, une cystoscopie, une hystéroscopie et une nasopharyngolaryngoscopie ;

de préférence, les cellules exfoliées comprennent les cellules exfoliées provenant de l'un quelconque ou de la combinaison d'au moins deux parmi une urine, des expectorations, des selles, un liquide pleural et un liquide d'ascite ; et

de préférence, les échantillons de brossage cytologique comprennent les échantillons de brossage provenant de l'un quelconque ou de la combinaison d'au moins deux parmi une bronche, un œsophage, une cavité buccale et un col de l'utérus.

7. Méthode selon l'une quelconque des revendications 1 à 6, dans laquelle l'hybridation *in situ* est effectuée au moyen de la méthode d'hybridation in situ RNAscope ; et

de préférence, l'hybridation *in situ* RNAscope est effectuée par utilisation d'une trousse de dosage colorimétrique monoplex ou multiplex, ou d'une trousse de dosage par fluorescence monoplex ou multiplex, parmi lesquelles la trousse de dosage colorimétrique rouge/marron monoplex ou la trousse de dosage par fluorescence multiplex est préférable.

8. Utilisation de la méthode de l'une quelconque des revendications 1 à 7 dans la détection d'un cancer.

Figure 1

Figure 2

Grade 0                    Grade I

Figure 3 (a)                    Figure 3 (b)

Grade II — Grade III — Grade IV

Figure 3 (c)  Figure 3 (d)  Figure 3 (e)

Z1-LOI  Z1-CNV  Z1-TE

Figure 4 (a)

Z16-LOI  Z16-CNV  Z16-TE

Figure 4 (b)

LOI  CNV

Figure 5 (a)  Figure 5 (b)

Figure 5 (c)

Figure 5 (d)

Figure 5 (e)

Figure 5 (f)

Figure 6 (a)

Figure 6 (b)

Figure 6 (c)

Figure 6 (d)

Figure 6 (e)

Figure 6 (f)

Figure 6 (g)

Figure 6 (h)

Figure 6 (i)

Figure 7 (a)

Figure 7 (b)

Figure 8 (a)                                        Figure 8 (b)

Figure 8 (c)

LOI

Figure 8 (d)

CNV

Figure 8 (e)

TE

Figure 8 (f)

Z1

Figure 9 (a)

Z16

Figure 9 (b)

Z8

Figure 9 (c)

Z10

Figure 9 (d)

Figure 9 (e)

Figure 9 (f)

Figure 9 (g)

Figure 9 (h)

Figure 9 (i)

Figure 9 (j)

Figure 9 (k)

Figure 10 (a)

Figure 10 (b)

Figure 11 (a)                                        Figure 11 (b)

Figure 11 (c)

**LOI**

Figure 11 (d)

**CNV**

Figure 11 (e)

**TE**

Figure 11 (f)

**LOI**

Figure 11 (g)

**CNV**

Figure 11 (h)

**TE**

Figure 11 (i)

46

## Z1

Figure 12 (a)

## Z16

Figure 12 (b)

## Z3

Figure 12 (c)

## Z10

Figure 12 (d)

## Z11

Figure 12 (e)

## Z4

Figure 12 (f)

## Z5

Figure 12 (g)

## Z6

Figure 12 (h)

Figure 12 (i)

Figure 12 (j)

Figure 12 (k)

Figure 12 (l)

Figure 12 (m)

Figure 12 (n)

Figure 12 (o)

Figure 13 (a)

Figure 13 (b)

Figure 14 (a)

Figure 14 (b)

Figure 14 (c)

Figure 14 (d)

Figure 14 (e)

Figure 14 (f)

Figure 15 (a)

Figure 15 (b)

Figure 15 (c)

Figure 15 (d)

Figure 15 (e)

Figure 15 (f)

Figure 15 (g)

Figure 15 (h)

Figure 16 (a)

Figure 16 (b)

Figure 17 (a)

Figure 17 (b)

Figure 17 (c)

Figure 17 (d)

Figure 18 (a)

Figure 18 (b)

Figure 18 (c)

Figure 18 (d)

Figure 18 (e)

Figure 18 (f)

Figure 18 (g)

Figure 18 (h)

Figure 18 (i)

Figure 18 (j)

Figure 18 (k)

Benign
Potential
Malignant

Figure 19 (a)    Figure 19 (b)    Figure 19 (c)

Benign
Potential
Malignant

Figure 20 (a)    Figure 20 (b)    Figure 20 (c)

Benign
Malignant

Figure 21 (a)    Figure 21 (c)

Benign
Malignant

Figure 22 (a)    Figure 22 (b)

Benign

Malignant

Figure 23 (a)

Figure 23 (b)

Benign

Malignant

Figure 24 (a)

Figure 24 (b)

Benign

Malignant

Figure 25 (a)

Figure 25 (b)

Benign

Malignant

Figure 26 (a)

Figure 26 (b)

Benign

Malignant

Figure 27 (a)

Figure 27 (b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KIM et al.** *Nucleic Acids Research*, 2015, vol. 43 (22)